# EUROPEAN PATENT APPLICATION

(11) **EP 2 423 192 A1**
(43) Date of publication of application: **29.02.2012**
(21) Application number: 10767184.4
(22) Date of filing: 19.04.2010
(51) Int. Cl.: C07D 213/16, C07D 471/04, C09K 11/06, H01L 51/50, C07F 1/08, C07F 1/10

(54) **METAL COMPLEX HAVING AROMATIC LIGAND THAT CONTAINS NITROGEN ATOM**

(30) Priority: 23.04.2009 JP 2009104910
(71) Applicant: Sumitomo Chemical Company, Limited, Tokyo 104-8260 (JP)
(72) Inventor: KURAMOCHI, Yusuke, Tsukuba-shi Ibaraki 305-0821 (JP); KOBAYASHI, Norifumi, Tsukuba-shi Ibaraki 305-0003 (JP); HIGASHIMURA, Hideyuki, Tsukuba-shi Ibaraki 305-0045 (JP)
(74) Representative: Duckworth, Timothy John
(86) International application number: PCT/JP2010/057308
(87) International publication number: WO 2010/123131

(57) **Abstract**

A metal complex containing a nitrogen-containing aromatic ring ligand which has a dendritic molecular chain, and a copper(I) ion or a silver(I) ion. For example, the metal complex which is represented by compositional formula (8) : 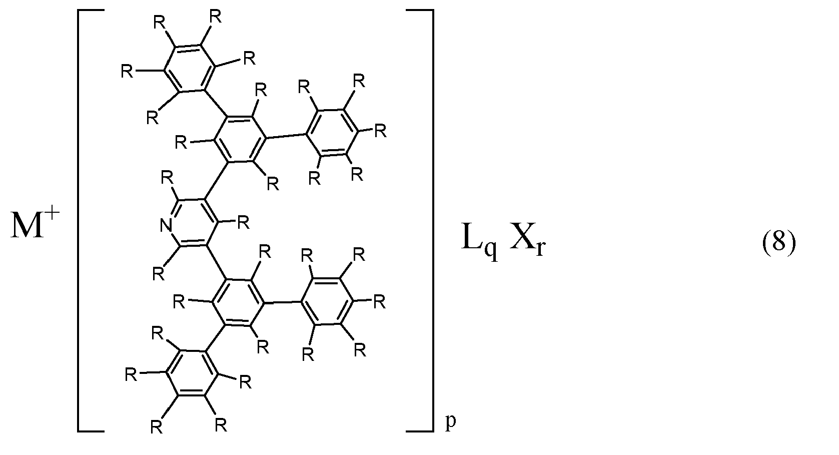 (wherein M⁺ is a copper(I) ion or a silver(I) ion, L is a ligand, and X is a counter ion; p is a positive number, and q and r are each independently numerical numbers of 0 or more; R represents a hydrogen atom, a halogen atom, a cyano group, a nitro group, a carboxyl group, an amino group, an acylamino group, a silyl group, a hydroxyl group, an acyl group, a hydrocarbyl group, a hydrocarbyloxy group, a hydrocarbylthio group, or a heterocyclic group, wherein the amino group, the silyl group, the hydrocarbyl group, the hydrocarbyloxy group and the hydrocarbylthio group optionally have a substituent) has excellent heat resistance.

## Description

### TECHNICAL FIELD

The present invention relates to a metal complex having an aromatic ring ligand containing a nitrogen atom.

### BACKGROUND ART

For a light-emitting material used in an organic electroluminescent device, a light-emitting material utilizing light-emission from a triplet excited state has been developed. As the light-emitting material utilizing light-emission from the triplet excited state, a metal complex obtained by binding pyridine with copper(I) halide has been proposed (K. R. Kyle et al., Journal of the American Chemical Society 113, 2954-2965 (1991)). However, the metal complex does not have sufficient heat resistance.

### DISCLOSURE OF INVENTION

The present invention provides a metal complex having excellent heat resistance.

The metal complex of the present invention has an aromatic ring ligand containing a nitrogen atom which has a dendritic molecular chain, and a copper(I) ion or a silver(I) ion.

The present invention also provides use of the metal complex for a light-emitting device.

Further, the present invention provides an aromatic ring ligand containing a nitrogen atom which has a dendritic molecular chain, which is used in the metal complex.

In the metal complex of the present invention, an aromatic ring ligand containing a nitrogen atom is usually bound to a copper(I) ion or a silver(I) ion through a coordinate bond. However, the bond may be an ionic bond, a hydrogen bond, π-π interaction, or van der Waals force.

The dendritic molecular chain (branched molecular structure) means a structure having symmetric or asymmetric dendritic branches, and contains a dendron structure and a hyper-branched structure. Herein, the dendron structure means a regular branched structure, and the hyper-branched structure means a non-regular branched structure.

The dendritic molecular chain preferably has a conjugated system from the viewpoint of transport of charge. It is preferable that from 30 to 100% by number of covalent bond among all covalent bonds in the dendritic molecular chain be conjugated. The conjugated covalent bond is more preferably from 50 to 100% by number, and further more preferably from 65 to 100% by number.

The dendritic molecular chain is composed of a branched portion and a terminal portion. From the viewpoint of chemical stability, it is preferable that the branched portion be a group having an aromatic ring and a terminal portion having an aromatic ring is bonded to the branched portion. It is more preferable that the branched portion be a trivalent group having an aromatic ring and the terminal portion having an aromatic ring is bonded to the branched portion.

Examples of the aromatic rings of the branched portion and the terminal portion bonded thereto include rings represented by the following formulae (A-1) to (A-32) which optionally have a substituent. Examples of the aromatic ring preferably include rings represented by formulae (A-1) to (A-7), and more preferably a ring represented by formula (A-1) .

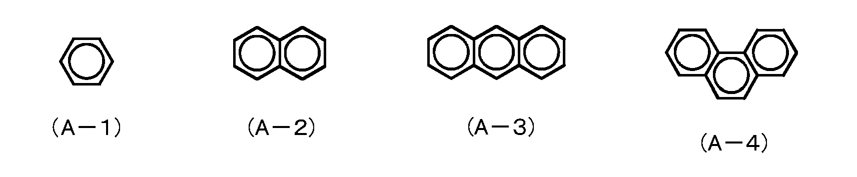

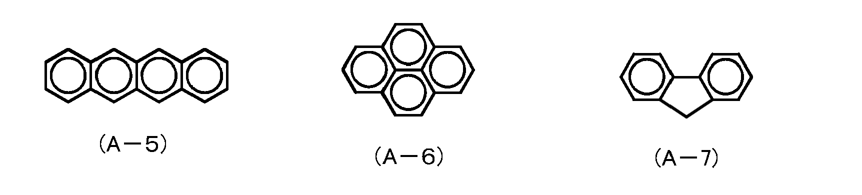

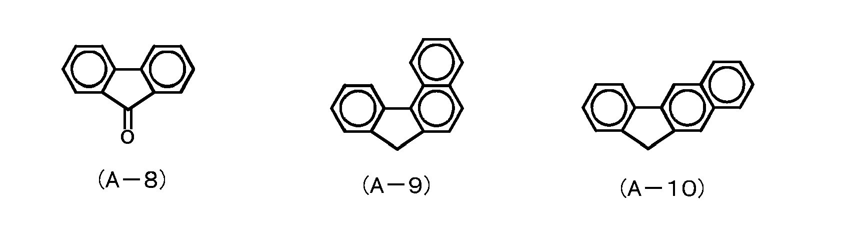

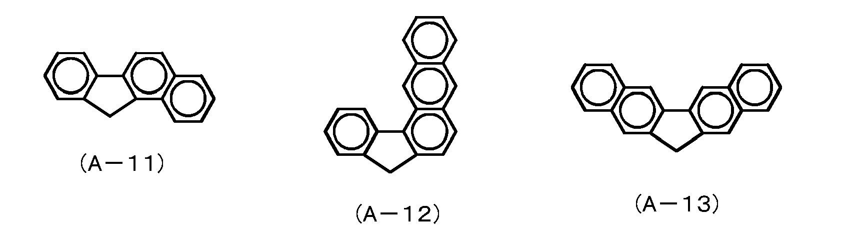

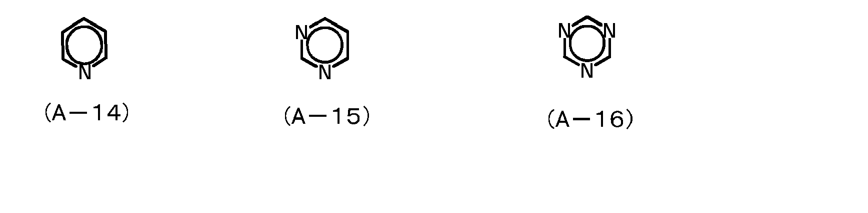

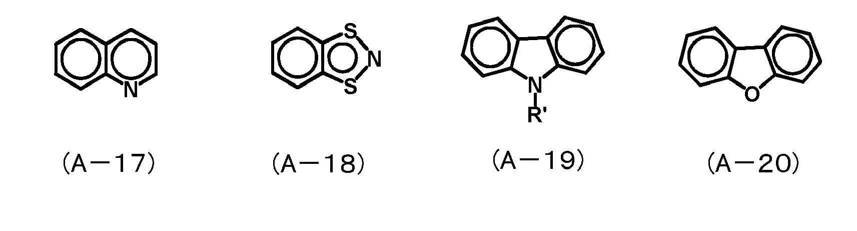

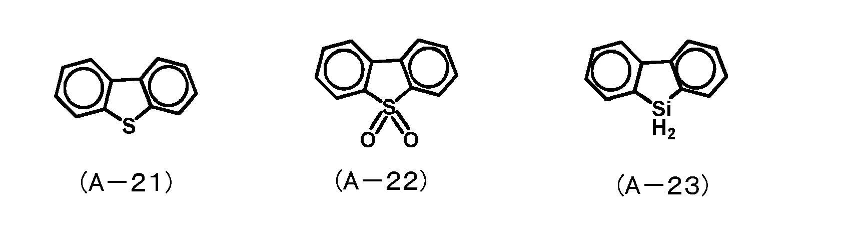

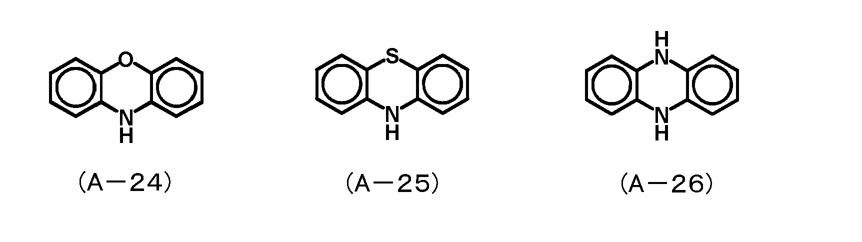

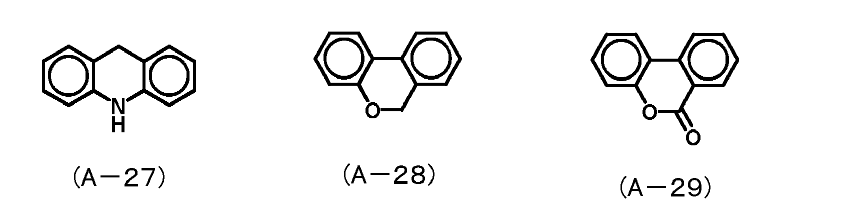

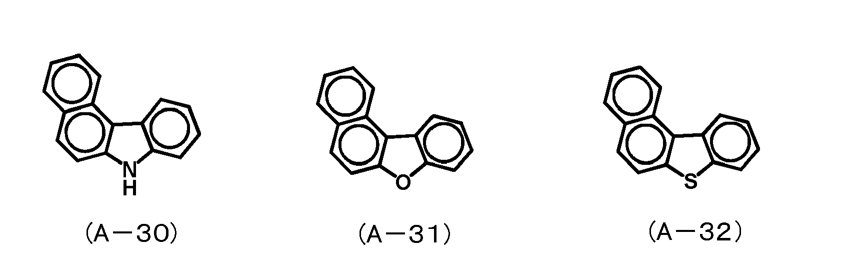

When the branched portion is a trivalent group having an aromatic ring, the number of substituent on the aromatic ring is preferably from 0 to 7, more preferably from 0 to 5, further preferably from 0 to 2, and particularly preferably 0.

When a group having an aromatic ring is bonded to the branched portion, the number of substituent on the aromatic ring is preferably from 0 to 8, more preferably from 0 to 6, further preferably from 0 to 3, and particularly preferably 1.

Unless otherwise particularly specified herein, when the substituent is bonded to a carbon atom, the substituent represents a halogen atom, a cyano group (-CN), a nitro group (-NO₂), a carboxyl group (-COOH), a -COO⁻ group, an amino group (-NH₂), a phosphono group (-P(=O)(OH)₂), a sulfo group (-SO₃H), an acylamino group, an imido group, a silyl group (-SiH₃), a hydroxyl group (-OH), an acyl group, an ammonio group (-NH₃⁺), a hydrocarbyl group, a hydrocarbyloxy group, a hydrocarbylthio group, or a heterocyclic group, preferably a carboxyl group, a -COO⁻ group, a phosphono group, a sulfo group, a hydroxyl group, an ammonio group, a hydrocarbyl group, a hydrocarbyloxy group, a hydrocarbylthio group, or a heterocyclic group, more preferably a hydrocarbyl group, a hydrocarbyloxy group, or a hydrocarbylthio group, and particularly preferably a hydrocarbyl group. When a substituent is plurally present, they may be the same as or different from each other.

Further, when the substituent is bonded to a nitrogen atom or a silicon atom, the substituent represents a hydrocarbyl group.

In the hydrocarbyl group, the hydrocarbyloxy group, and the hydrocarbylthio group, one or more hydrogen atoms contained in these groups may be further substituted with a halogen atom, a cyano group, a nitro group, a carboxyl group, a -COO⁻ group, an amino group, a phosphono group, a sulfo group, an acylamino group, an imido group, a silyl group, a hydroxyl group, an acyl group, an ammonio group, a hydrocarbyl group, a hydrocarbyloxy group, a hydrocarbylthio group, or a heterocyclic group.

In the amino group, the phosphono group, the sulfo group, the silyl group, and the ammonio group, one or more hydrogen atoms contained in these groups may be further substituted with a hydrocarbyl group.

When the substituent is a group that contains a carbon atom and does not contain an aromatic ring, the number of the carbon atom is from 1 to 50, preferably from 1 to 30, more preferably from 1 to 20, further preferably from 1 to 10, and particularly preferably from 1 to 4. When the substituent is a group containing an aromatic ring, the number of the carbon atom is from 2 to 50, preferably from 3 to 30, more preferably from 4 to 20, further preferably from 4 to 10, and particularly preferably from 4 to 6.

The halogen atom as a substituent is a fluorine atom, a chlorine atom, a bromine atom, or an iodine atom, preferably a fluorine atom, a chlorine atom, or a bromine atom, and more preferably a fluorine atom.

The -COO⁻ group as a substituent optionally has a counter ion. The counter ions include a lithium ion, a sodium ion, a potassium ion, a rubidium ion, a cesium ion, an ammonium ion, and the like. The counter ion is preferably a sodium ion, a potassium ion, or an ammonium ion, and more preferably a sodium ion or a potassium ion.

The amino group as a substituent is optionally substituted with a hydrocarbyl group as described above. Specific examples of the amino group include a phenylamino group, a diphenylamino group, a methylamino group, a dimethylamino group, an ethylamino group, a diethylamino group, a propylamino group, a dipropylamino group, a butylamino group, and a dibutylamino group. The amino group is preferably a diphenylamino group, a dimethylamino group, a diethylamino group, a dipropylamino group, or a dibutylamino group, and further preferably a diphenylamino group.

The acylamino groups as a substituent include a formamide group, an acetamido group, a propioamido group, a butyroamido group, a benzamido group, a trifluoroacetamido group, a pentafluorobenzamido group, a diformamido group, a diacetamido group, a dipropioamido group, a dibutyroamido group, a dibenzamido group, a ditrifluoroacetamido group, a dipentafluorobenzamido group, and the like. The acylamino group is preferably a formamide group, an acetamido group, a propioamido group, a butyroamido group, or a benzamido group.

The imido groups as a substituent include an N-succinimido group, an N-phthalimido group, a benzophenoneimido group, and the like. The imido group is preferably an N-phthalimido group.

The silyl group as a substituent is optionally substituted with a hydrocarbyl group as described above. Specifically, the silyl groups include a silyl group substituted with 1 to 3 groups selected from the group consisting of an alkyl group, an aryl group, and an arylalkyl group. The silyl group is preferably a trimethylsilyl group, a triethylsilyl group, a tripropylsilyl group, a triisopropylsilyl group, a dimethyl(isopropyl)silyl group, a diethyl(isopropyl)silyl group, a tert-butyl(dimethyl)silyl group, a pentyl(dimethyl)silyl group, a hexyl(dimethyl)silyl group, a heptyl(dimethyl)silyl group, an octyl(dimethyl)silyl group, a 2-ethylhexyl-dimethylsilyl group, a nonyl(dimethyl)silyl group, a decyl(dimethyl)silyl group, a 3,7-dimethyloctyl-dimethylsilyl group, a lauryl(dimethyl)silyl group, a triphenylsilyl group, a trip-xylylsilyl group, a tribenzylsilyl group, a diphenyl(methyl)silyl group, a tert-butyl(diphenyl)silyl group, or a dimethyl(phenyl)silyl group, more preferably a trimethylsilyl group, a triethylsilyl group, or tripropylsilyl group, and further preferably a trimethylsilyl group.

The acyl groups as a substituent include an acetyl group, a propionyl group, a butyryl group, an isobutyryl group, a pivaloyl group, a benzoyl group, a trifluoroacetyl group, a pentafluorobenzoyl group, and the like. The acyl group is preferably an acetyl group, a propionyl group, or a benzoyl group.

The ammonio group as a substituent is optionally substituted with a hydrocarbyl group as described above. The specific examples of the ammonio group include an ammonio group, a methylammonio group, a dimethylammonio group, a trimethylammonio group, an ethylammonio group, a diethylammonio group, a triethylammonio group, a propylammonio group, a dipropylammonio group, a tripropylammonio group, a butylammonio group, a dibutylammonio group, a tributylammonio group, and the like. The ammonio group is preferably a trimethylammonio group, a triethylammonio group, a tripropylammonio group, or a tributylammonio group.

The ammonio group as a substituent optionally has a counter ion. The counter ions include a fluoride ion, a chloride ion, a bromide ion, an iodide ion, a hydroxide ion, a sulfate ion, a nitrate ion, a carbonate ion, an acetate ion, a perchlorate ion, a tetrafluoroborate ion, a hexafluorophosphate ion, a hexafluoroantimony ion, a hexafluoroarsenate ion, a methanesulfonate ion, a trifluoromethanesulfonate ion, a trifluoroacetate ion, a benzenesulfonate ion, a p-toluenesulfonate ion, a dodecylbenzenesulfonate ion, a tetraphenylborate ion, a tetrakis(pentafluorophenyl)borate ion, and the like. The counter ion is preferably a fluoride ion, a chloride ion, a bromide ion, an iodide ion, a hydroxide ion, a sulfate ion, a nitrate ion, a perchlorate ion, a tetrafluoroborate ion, a hexafluorophosphate ion, a tetraphenylborate ion, or a tetrakis(pentafluorophenyl)borate ion, and further preferably a chloride ion, a bromide ion, or an iodide ion. In addition, the counter ion may be a polymer ion having these ions in a repeating unit.

The hydrocarbyl groups as a substituent include a methyl group, an ethyl group, a 1-propyl group, a 2-propyl group, a 1-butyl group, a 2-butyl group, a sec-butyl group, a tert-butyl group, a pentyl group, a hexyl group, an octyl group, a decyl group, a dodecyl group, a 2-ethylhexyl group, a 3,7-dimethyloctyl group, a cyclopropyl group, a cyclopentyl group, a cyclohexyl group, a 1-adamantyl group, a 2-adamantyl group, a norbornyl group, an ammonioethyl group, a benzyl group, an a,a-dimethylbenzyl group, a 1-phenethyl group, a 2-phenethyl group, a vinyl group, a propenyl group, a butenyl group, an oleyl group, an eicosapentaenyl group, a docosahexaenyl group, a 2,2-diphenylvinyl group, a 1,2,2-triphenylvinyl group, a 2-phenyl-2-propenyl group, a phenyl group, a 2-tolyl group, a 4-tolyl group, a 4-trifluoromethylphenyl group, a 4-methoxyphenyl group, a 4-cyanophenyl group, a 2-phenylphenyl group, a 3-phenylphenyl group, a 4-phenylphenyl group, a biphenylphenyl group, a 3,5-diphenylphenyl group, a 3,4-diphenylphenyl group, a pentaphenylphenyl group, a 4-(2,2-diphenylvinyl)phenyl group, a 4-(1,2,2-triphenylvinyl)phenyl group, a fluorenyl group, a 1-naphthyl group, a 2-naphthyl group, a 9-anthryl group, a 2-anthryl group, a 9-phenanthryl group, a 1-pyrenyl group, a chrysenyl group, a naphthacenyl group, a coronenyl group, and the like. The hydrocarbyl group is preferably a methyl group, an ethyl group, a 1-propyl group, a 2-propyl group, a 1-butyl group, a 2-butyl group, a sec-butyl group, a tert-butyl group, a pentyl group, a hexyl group, an octyl group, a decyl group, a dodecyl group, a 2-ethylhexyl group, a 3,7-dimethyloctyl group, a benzyl group, an a,a-dimethylbenzyl group, a 1-phenethyl group, a 2-phenethyl group, a vinyl group, a propenyl group, a butenyl group, an oleyl group, an eicosapentaenyl group, a docosahexaenyl group, a 2,2-diphenylvinyl group, a 1,2,2-triphenylvinyl group, a 2-phenyl-2-propenyl group, a phenyl group, a 2-tolyl group, a 4-tolyl group, a 4-trifluoromethylphenyl group, a 4-methoxyphenyl group, a 4-cyanophenyl group, a 2-phenylphenyl group, a 3-phenylphenyl group, a 4-phenylphenyl group, a biphenylphenyl group, a 3,5-diphenylphenyl group, a 3,4-diphenylphenyl group, a pentaphenylphenyl group, a 4-(2,2-diphenylvinyl)phenyl group, a 4-(1,2,2-triphenylvinyl)phenyl group, a fluorenyl group, a 1-naphthyl group, a 2-naphthyl group, a 9-anthryl group, a 2-anthryl group, or a 9-phenanthryl group. The hydrocarbyl group is more preferably a methyl group, an ethyl group, a 1-propyl group, a 2-propyl group, a 1-butyl group, a 2-butyl group, a sec-butyl group, a tert-butyl group, a pentyl group, a hexyl group, an octyl group, a decyl group, a dodecyl group, a 2-ethylhexyl group, a 3,7-dimethyloctyl group, a benzyl group, a vinyl group, a propenyl group, a butenyl group, a phenyl group, a 2-phenylphenyl group, a 3-phenylphenyl group, a 4-phenylphenyl group, or a biphenylphenyl group. The hydrocarbyl group is further preferably a methyl group, an ethyl group, a 1-propyl group, a 2-propyl group, a 1-butyl group, a 2-butyl group, a tert-butyl group, a pentyl group, a hexyl group, a 2-ethylhexyl group, a vinyl group, a propenyl group, a butenyl group, or a phenyl group. The hydrocarbyl group is particularly preferably a methyl group, an ethyl group, a tert-butyl group, or a phenyl group. The hydrocarbyl group is most preferably a methyl group or a tert-butyl group.

The hydrocarbyloxy groups as a substituent include a methoxy group, an ethoxy group, a 1-propyloxy group, a 2-propyloxy group, a 1-butyloxy group, a 2-butyloxy group, a sec-butyloxy group, a tert-butyloxy group, a pentyloxy group, a hexyloxy group, an octyloxy group, a decyloxy group, a dodecyloxy group, a 2-ethylhexyloxy group, a 3,7-dimethyloctyloxy group, a cyclopropyloxy group, a cyclopentyloxy group, a cyclohexyloxy group, a 1-adamantyloxy group, a 2-adamantyloxy group, a norbornyloxy group, an ammonioethoxy group, a trifluoromethoxy group, a benzyloxy group, an α,α-dimethylbenzyloxy group, a 2-phenethyloxy group, a 1-phenethyloxy group, a phenoxy group, a methoxyphenoxy group, an octylphenoxy group, a 1-naphthyloxy group, a 2-naphthyloxy group, a pentafluorophenyloxy group, and the like. The hydrocarbyloxy group is preferably a methoxy group, an ethoxy group, a 1-propyloxy group, a 2-propyloxy group, a 1-butyloxy group, a 2-butyloxy group, a sec-butyloxy group, a tert-butyloxy group, a pentyloxy group, a hexyloxy group, an octyloxy group, a decyloxy group, a dodecyloxy group, a 2-ethylhexyloxy group, or a 3,7-dimethyloctyloxy group, and further preferably a methoxy group or an ethoxy group.

The hydrocarbylthio groups as a substituent include a methylthio group, an ethylthio group, a 1-propylthio group, a 2-propylthio group, a 1-butylthio group, a 2-butylthio group, a sec-butylthio group, a tert-butylthio group, a pentylthio group, a hexylthio group, an octylthio group, a decylthio group, a dodecylthio group, a 2-ethylhexylthio group, a 3,7-dimethyloctylthio group, a cyclopropylthio group, a cyclopentylthio group, a cyclohexylthio group, a 1-adamantylthio group, a 2-adamantylthio group, a norbornylthio group, an ammonioethylthio group, a trifluoromethylthio group, a benzylthio group, an α,α-dimethylbenzylthio group, a 2-phenethylthio group, a 1-phenethylthio group, a phenylthio group, a methoxyphenylthio group, an octylphenylthio group, a 1-naphthylthio group, a 2-naphthylthio group, or a pentafluorophenylthio group. The hydrocarbylthio group is preferably a methylthio group, an ethylthio group, a 1-propylthio group, a 2-propylthio group, a 1-butylthio group, a 2-butylthio group, a sec-butylthio group, a pentylthio group, a hexylthio group, an octylthio group, a decylthio group, a dodecylthio group, a 2-ethylhexylthio group, or a 3,7-dimethyloctylthio group, and further preferably a methylthio group or an ethylthio group.

The heterocyclic group as a substituent means an atomic group formed by removing one hydrogen atom from a heterocyclic compound. The heterocyclic groups include a furyl group, a thienyl group, a pyrrolyl group, an imidazolyl group, an oxazolyl group, a thiazolyl group, and a pyridyl group which optionally have a substituent. The heterocyclic groups are preferably a thienyl group, an imidazolyl group, an oxazolyl group, a thiazolyl group and a pyridyl group which optionally have a substituent, and more preferably an imidazolyl group and a pyridyl group which optionally have a substituent.

The branched portion of the dendritic molecular chain is preferably a trivalent group represented by formula (1): 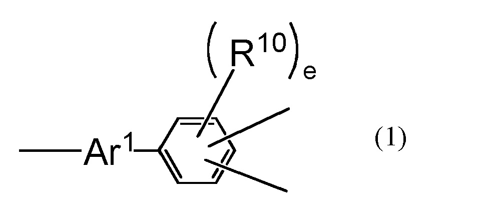 wherein Ar¹ is a direct bond, an oxygen atom, an imino group (-NH-), a -Q¹-CONH- group, a diimido group, a hydrocarbondiyl group, a -Q¹-O- group, a -Q¹-S- group, or a divalent heterocyclic group, and Q¹ is a hydrocarbondiyl group, wherein the imino group and the hydrocarbondiyl group optionally have a substituent; R¹⁰ is a halogen atom, a cyano group, a nitro group, a carboxyl group, an amino group, an acylamino group, an imido group, a silyl group, a hydroxyl group, an acyl group, a hydrocarbyl group, a hydrocarbyloxy group, a hydrocarbylthio group, or a divalent heterocyclic group, wherein the amino group, hydrocarbyl group, the hydrocarbyloxy group, and the hydrocarbylthio group optionally have a substituent; e is an integer of integers from 0 to 3; and when e is 2 or more, R¹⁰ which is plurally present may be the same as or different from each other.

The terminal portion bonded to the branched portion is preferably a group represented by formula (2): 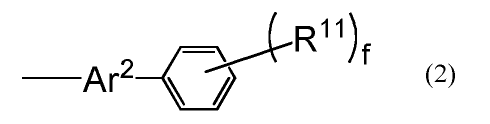 wherein Ar² is a direct bond, an oxygen atom, an imino group, a -Q²-CONH- group, a diimido group, a hydrocarbondiyl group, a -Q²-O- group, a -Q²-S- group, or a divalent heterocyclic group, and Q² is a hydrocarbondiyl group, wherein the imino group and the hydrocarbondiyl group optionally have a substituent; R¹¹ is a halogen atom, a cyano group, a nitro group, a carboxyl group, a -COO group, an amino group, a phosphono group, a sulfo group, an acylamino group, an imido group, a silyl group, a hydroxyl group, an acyl group, an ammonio group , a hydrocarbyl group, a hydrocarbyloxy group, a hydrocarbylthio group, or a divalent heterocyclic group; the amino group, the phosphono group, the sulfo group, the silyl group, the ammonio group, the hydrocarbyl group, the hydrocarbyloxy group and the hydrocarbylthio group optionally have a substituent; f is an integer of from 0 to 5; and when f is 2 or more, R¹¹ which is plurally present may be the same as or different from each other.

It is more preferable that the branched portion of the dendritic molecular chain be the trivalent group represented by formula (1) and a monovalent group represented by formula (2) as the terminal portion be bonded to the branched portion.

In formulae (1) and (2), the amino groups represented by Ar¹ and Ar² which optionally have a substituent include a phenylamino group, a methylamino group, an ethylamino group, a propylamino group, a butylamino group, and a dibutylamino group. The amino group is preferably a phenylamino group.

In formulae (1) and (2), Q¹ and Q² in the -Q¹-CONH-group and the -Q²-CONH- group represented by Ar¹ and Ar² include a direct bond, methylene, trimethylene, tetramethylene, a -C₆H₄CH₂- group, fluoromethylene, a - C₆FH₃CH₂- group, or the like. Q¹ and Q² are preferably a direct bond, methylene, trimethylene, or a -C₆H₄CH₂- group, and more preferably a direct bond.

In formulae (1) and (2), the diimido group represented by Ar¹ and Ar² include a pyromellitic diimido group, a naphthalene-1,4,5,8-tetracarboxylic diimido group, and the like.

In formulae (1) and (2), the hydrocarbondiyl groups represented by Ar¹ and Ar² which optionally have a substituent include a methylene group, an ethylene group, a trimethylene group, a tetramethylene group, a pentamethylene group, a hexamethylene group, an octamethylene group, a decamethylene group, a dodecamethylene group, an ethylhexylene group, a dimethyloctylene group, a cyclopropylene group, a cyclopentylene group, a cyclohexylene group, an adamantanediyl group, a norbornanediyl group, an ammonioethylene group, a -C₆H₄CH₂- group, a -CH₃C₆H₃CH₂-group, a -C₆H₄CH₂CH₂- group, a vinylene group, a propenylene group, a butenylene group, a diphenylvinylene group, a phenylvinylene group, a phenylpropenylene group, a phenylene group, a methylphenylene group, a trifluoromethylphenylene group, a methoxyphenylene group, a cyanophenylene group, a biphenyldiyl group, a terphenyldiyl group, a tetraphenylphenylene group, a (2,2-diphenylvinyl)phenylene group, a (1,2,2-triphenylvinyl)phenylene group, a fluorenediyl group, a naphthylene group, an anthrylene group, a phenanthrylene group, a pyrenediyl group, and the like. Preferable examples thereof include a methylene group, an ethylene group, a -C₆H₄CH₂- group, a -C₆H₄CH₂CH₂- group, a vinylene group, a propenylene group, a butenylene group, a phenylvinylene group, a phenylene group, a biphenyldiyl group, a terphenyldiyl group, a tetraphenylphenylene group, a fluorenediyl group, a naphthylene group, an anthrylene group, a phenanthrylene group, and a pyrenediyl group. The hydrocarbondiyl group is more preferably a methylene group, a -C₆H₄CH₂- group, a vinylene group, a propenylene group, a phenylvinylene group, a phenylene group, a fluorenediyl group, or a naphthylene group, and further preferably a vinylene group, a phenylvinylene group, or a phenylene group.

In formulae (1) and (2), Q¹ and Q² in the -Q¹-O- group and the -Q²-O- group represented by Ar¹ and Ar² include the above-described hydrocarbondiyl group. Q¹ and Q² are preferably a methylene group, an ethylene group, a -C₆H₄CH₂-group, a -C₆H₄CH₂CH₂- group, a phenylene group, or a naphthylene group, and more preferably a methylene group or an ethylene group.

In formulae (1) and (2), Q¹ and Q² in the -Q¹-O- group and the -Q²-S- group represented by Ar¹ and Ar² include the above-described hydrocarbondiyl group. Q¹ and Q² are preferably a methylene group, an ethylene group, a -C₆H₄CH₂-group, a -C₆H₄CH₂CH₂- group, a phenylene group, or a naphthylene group, and more preferably a methylene group or an ethylene group.

In formulae (1) and (2), the divalent heterocyclic groups represented by Ar¹ and Ar² mean an atomic group by removing two hydrogen atoms from a heterocyclic compound. The divalent heterocyclic groups include a furandiyl group, a thiophenediyl group, a pyrrolediyl group, a pyrimidinediyl group, a pyrazinediyl group, an imidazolediyl group, an oxazolediyl group, a thiazolediyl group, and a pyridinediyl group which optionally have a substituent, and the like. The divalent heterocyclic groups are preferably a furandiyl group, a thiophenediyl group, an oxazolediyl group, and a thiazolediyl group which optionally have a substituent.

In formulae (1) and (2), each of Ar¹ and Ar² is preferably a direct bond, an oxygen atom, an imino group, a -Q²-CONH- group, a hydrocarbondiyl group, or a -Q²-O- group, wherein Q² indicates a hydrocarbondiyl group. Each of Ar¹ and Ar² is more preferably a direct bond, a hydrocarbondiyl group, or a -Q²-O- group.

In formula (1), specific examples of the halogen atom, and the cyano group, the nitro group, the carboxyl group, the amino group, the acylamino group, the imido group, the silyl group, the hydroxyl group, the acyl group, the hydrocarbyl group, the hydrocarbyloxy group, the hydrocarbylthio group and the heterocyclic group which are represented by R¹⁰ are as described above. R¹⁰ is preferably a hydrocarbyl group or a hydrocarbyloxy group, and more preferably a hydrocarbyl group.

In formula (1), e is preferably an integer of from 0 to 2, more preferably 0 or 1, and further preferably 0.

In formula (2), specific examples of the halogen atom, and the cyano group, the nitro group, the carboxyl group, the -COO⁻ group, the amino group, the phosphono group, the sulfo group, the acylamino group, the imido group, the silyl group, the hydroxyl group, the acyl group, the ammonio group, the hydrocarbyl group, the hydrocarbyloxy group, the hydrocarbylthio group and the heterocyclic group which are represented by R¹¹ are as described above, and preferable examples of R¹¹ include a carboxyl group, a -COO⁻ group, a phosphono group group, a sulfo group, a hydroxyl group, an ammonio group, a hydrocarbyl group, a hydrocarbyloxy group, a hydrocarbylthio group, and a heterocyclic group. R¹⁰ is more preferably a hydrocarbyl group, a hydrocarbyloxy group, or a hydrocarbylthio group, and further preferably a hydrocarbyl group.

In formula (2), f is preferably an integer of from 0 to 3, more preferably an integer of from 0 to 2, and further preferably 1.

In the dendritic molecular chain, each of Ar¹ and Ar² is preferably a direct bond from the viewpoint of transport of charge.

The trivalent groups represented by formula (1) include groups represented by the following formulae (a-1) to (a-16).

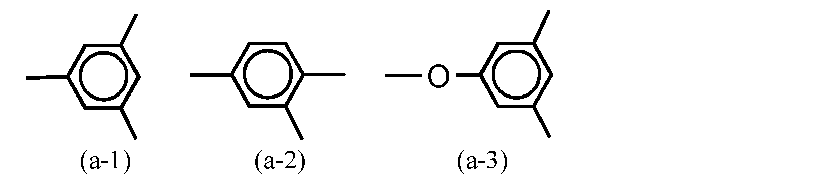

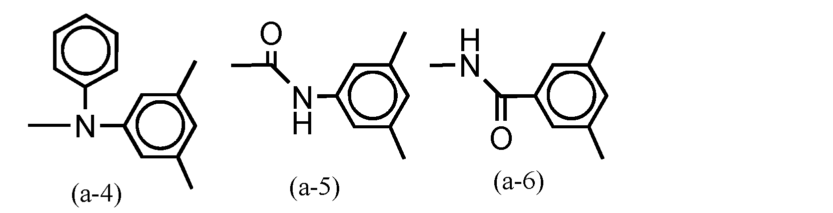

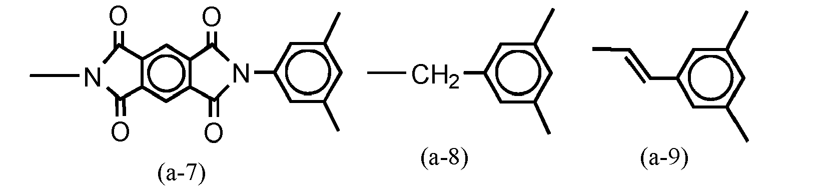

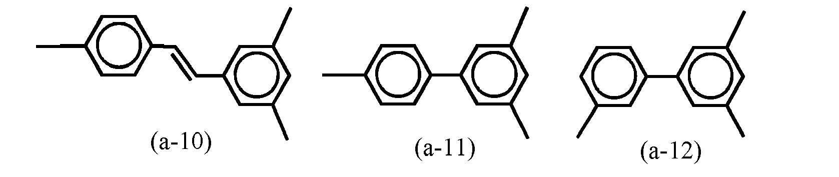

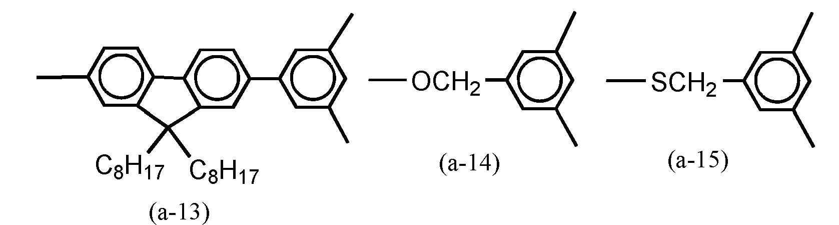

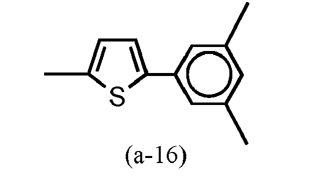

The monovalent groups represented by formula (2) include groups represented by the following formulae (b-1) to (b-32).

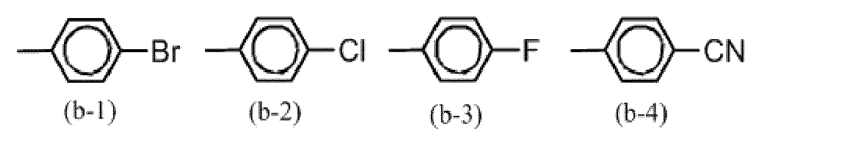

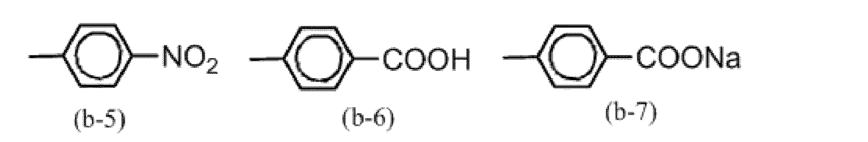

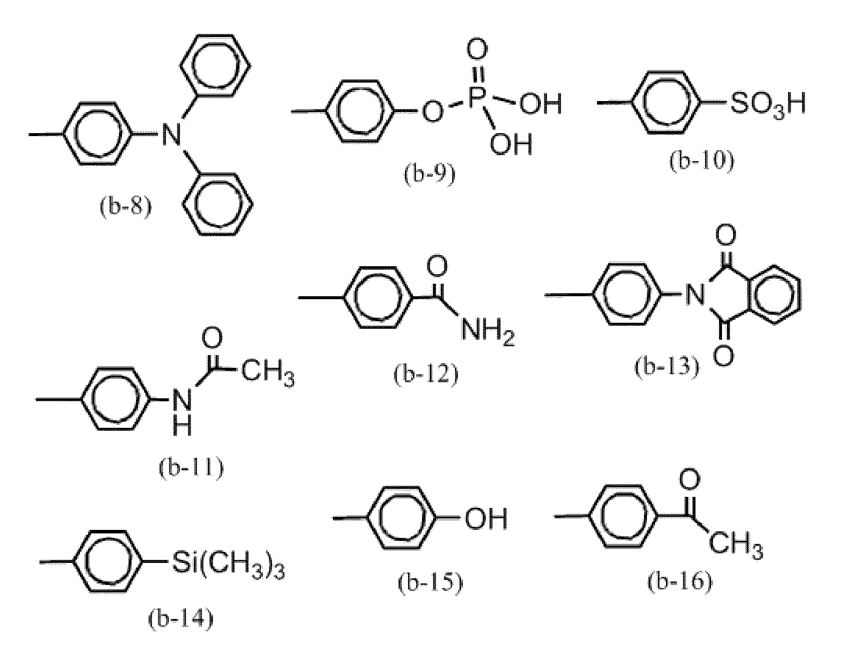

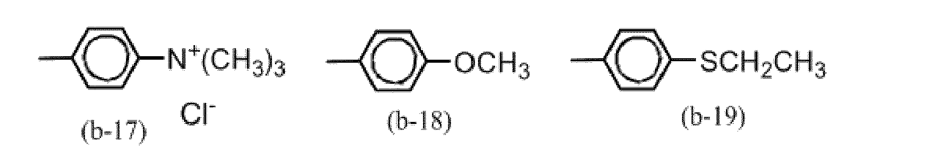

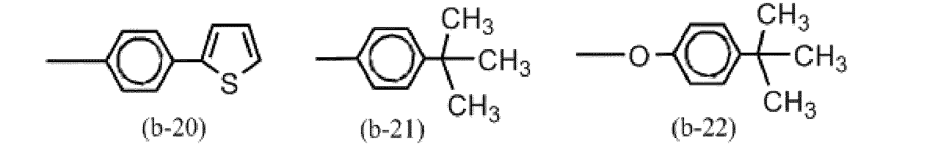

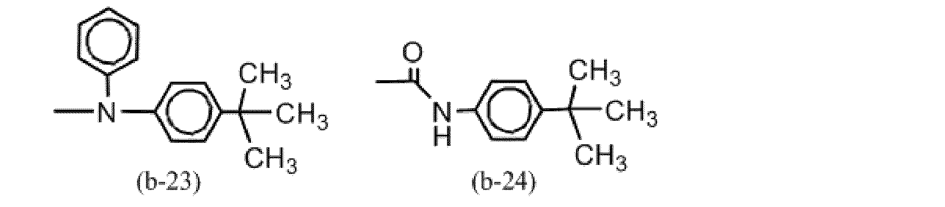

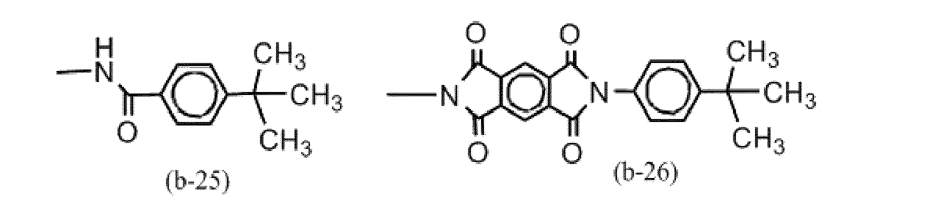

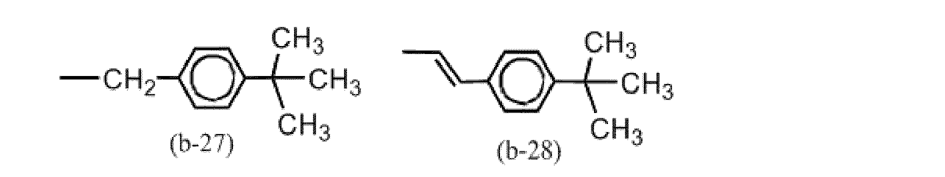

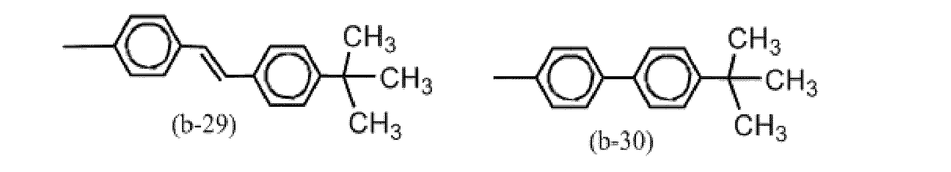

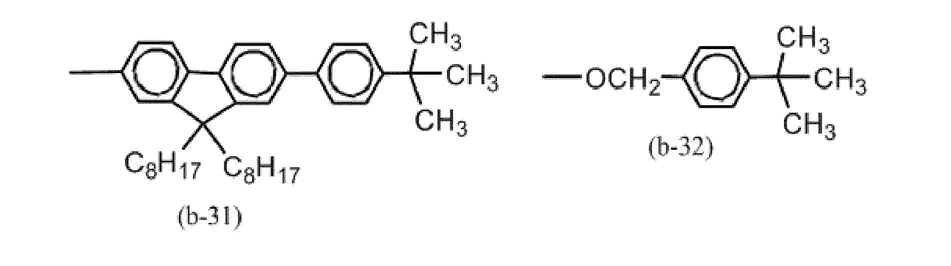

In the aromatic ring ligand containing a nitrogen atom which has a dendritic molecular chain, the aromatic ring ligand containing a nitrogen atom means an aromatic compound containing a nitrogen atom inside or outside the ring, which can be converted into a ligand.

The aromatic ring ligand containing a nitrogen atom is preferably one represented by formula (3): 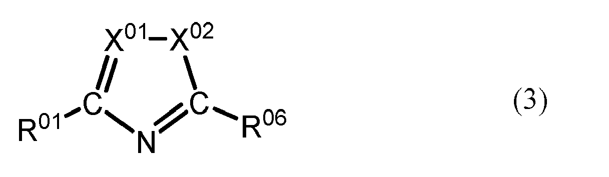 wherein X⁰¹ is a nitrogen atom or a -C(R⁰²)= group, and X⁰² is -X⁰³=X⁰⁴-, a -N(R⁰³)- group, N⁻, an oxygen atom, or a sulfur atom; X⁰³ is a nitrogen atom or a -C(R⁰⁴)= group, and X⁰⁴ is a nitrogen atom or a -C(R⁰⁵)= group; provided that, when X⁰² is -X⁰³=X⁰⁴-, at least one of X⁰¹, X⁰³, and X⁰⁴ is not a nitrogen atom; when X⁰¹ is a nitrogen atom, X⁰² is - X⁰³=X⁰⁴- or a -N(R⁰³)- group, and when X⁰² is N⁻, an oxygen atom, or a sulfur atom, X⁰¹ is a -C(R⁰²)= group; R⁰¹, R⁰², R⁰⁴, R⁰⁵, and R⁰⁶ each independently represent a hydrogen atom, a halogen atom, a cyano group, a nitro group, a carboxyl group, a -COO⁻ group, an acylamino group, an imido group, a silyl group, a hydroxyl group, an acyl group, a hydrocarbyl group, a hydrocarbyloxy group, a hydrocarbylthio group, or a heterocyclic group, and R⁰³ represents a hydrogen atom, a hydrocarbyl group, or a heterocyclic group, wherein the silyl group, the hydrocarbyl group, the hydrocarbyloxy group and the hydrocarbylthio group optionally have a substituent; at least one of R⁰¹, R⁰², R⁰³, R⁰⁴, R⁰⁵, and R⁰⁶ has the dendritic molecular chain; and R⁰¹ and R⁰² R⁰² and R⁰³, R⁰² and R⁰⁴, R⁰³ and R⁰⁶, R⁰⁴ and R⁰⁵, or R⁰⁵ and R⁰⁶ may be each taken together to form a ring.

In formula (3), specific examples of respective groups for R⁰¹, R⁰², R⁰⁴, R⁰⁵, and R⁰⁶ are as described above, and they are preferably a hydrogen atom, a hydrocarbyl group, a hydrocarbyloxy group, or a hydrocarbylthio group, more preferably a hydrogen atom or a hydrocarbyl group, and further preferably a hydrogen atom.

In formula (3), specific examples of R⁰³ are as described above, and it is preferably a hydrocarbyl group.

Examples of the nitrogen-containing aromatic ring ligand which has a dendritic molecular chain include a ligand in which two groups each formed by removing one hydrogen atom from R⁰⁶ in formula (3) are coupled. The respective coupled groups formed by removing one hydrogen atom from R⁰⁶ in formula (3) may be the same as or different from each other. When each X⁰² in the coupled groups is a -N(R⁰³)- group, both R⁰³ may be taken together to form a ring. When each X⁰² is a -X⁰³=X⁰⁴- group, both X⁰⁴ may be taken together to form a ring. When one X⁰² is an N(R⁰³)- group and the other X⁰² is a -X⁰³=X⁰⁴- group, R⁰³ and X⁰⁴ may be taken together to form a ring.

Formula (3) is more preferably one represented by formula (4): 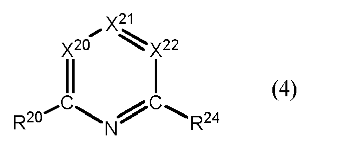 wherein X²⁰ is a nitrogen atom or a -C(R²¹)= group, X²¹ is a nitrogen atom or a -C(R²²)= group, and X²² is a nitrogen atom or a -C(R²³)= group; provided that at least one of X²⁰, X²¹, and X²² is not a nitrogen atom; at least one of R²⁰, R²¹, R²², R²³, and R²⁴ has a dendritic molecular chain, and R²⁰, R²¹, R²², R²³, and R²⁴ each independently represent a hydrogen atom, a halogen atom, a cyano group, a nitro group, a carboxyl group, a -COO⁻ group, an acylamino group, an imido group, a silyl group, a hydroxyl group, an acyl group, a hydrocarbyl group, a hydrocarbyloxy group, a hydrocarbylthio group, or a heterocyclic group, wherein the silyl group, the hydrocarbyl group, the hydrocarbyloxy group and the hydrocarbylthio group optionally have a substituent; and R²⁰ and R²¹, R²¹ and R²², R²² and R²³, and R²³ and R²⁴ may be each taken together to form a ring, or formula (5): 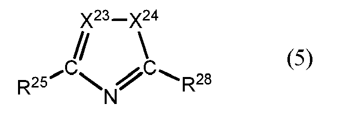 wherein X²³ is a nitrogen atom or a -C(R²⁶)= group, and X²⁴ is a -N(R²⁷)= group, N⁻, an oxygen atom, or a sulfur atom; provided that, when X²³ is a nitrogen atom, X²⁴ is a - N(R²⁷)- group, when X²⁴ is N⁻, an oxygen atom, or a sulfur atom, X²³ is a -C(R²⁶)= group; at least one of R²⁵, R²⁶, R²⁷, and R²⁸ has a dendritic molecular chain, and R²⁵, R²⁶, and R²⁸ each independently represent a hydrogen atom, a halogen atom, a cyano group, a nitro group, a carboxyl group, a - COO⁻ group, an acylamino group, an imido group, a silyl group, a hydroxyl group, an acyl group, a hydrocarbyl group, a hydrocarbyloxy group, a hydrocarbylthio group, or a heterocyclic group, wherein the silyl group, the hydrocarbyl group, the hydrocarbyloxy group and the hydrocarbylthio group optionally have a substituent; R²⁷ is a hydrogen atom, a hydrocarbyl group, or a heterocyclic group, wherein the hydrocarbyl group optionally has a substituent; and R²⁵ and R²⁶, R²⁶ and R²⁷, and R²⁷ and R²⁸ may be each taken together to form a ring.

In formula (4), specific examples of respective groups for R²⁰, R²¹, R²², R²³, and R²⁴ are as described above, and they are preferably a hydrogen atom, a hydrocarbyl group, a hydrocarbyloxy group, or a hydrocarbylthio group, more preferably a hydrogen atom or a hydrocarbylthio group, and further preferably a hydrogen atom.

In formula (4), X²⁰ is preferably a -C(R²¹)= group, X²¹ is preferably a -C(R²²)= group, and X²² is preferably a - C(R²³)= group.

In formula (5), specific examples of respective groups for R²⁵, R²⁶, and R²⁸ are as described above, and they are preferably a hydrogen atom, a hydrocarbyl group, a hydrocarbyloxy group, or a hydrocarbylthio group, more preferably a hydrogen atom or a hydrocarbylthio group, and further preferably a hydrogen atom.

In formula (5), specific examples of R²⁷ are as described above, and it is preferably a hydrocarbyl group.

In formula (5), X²³ is preferably a -C(R²⁶)= group, X²⁴ is preferably a -N(R²⁷)- group, a sulfur atom, or an oxygen atom, and more preferably a -N(R²⁷)- group.

Further, the nitrogen-containing aromatic ring ligand which has a dendritic molecular chain is a ligand obtained by coupling two groups selected from a monovalent group formed by removing one hydrogen atom from R²⁴ in formula (4), and a monovalent group formed by removing one hydrogen atom from R²⁸ in formula (5). The respective monovalent groups formed by removing one hydrogen atom from R²⁴ in formula (4) or the respective monovalent groups formed by removing one hydrogen atom from R²⁸ in formula (5) may be the same as or different from each other. When each of the coupled groups is the group formed by removing one hydrogen atom from R²⁴ in the formula (4), both R²³ may be taken together to form a ring. When each of the coupled groups is the group formed by removing one hydrogen atom from R²⁸ in formula (5), both R²⁷ may be taken together to form a ring. When one of the coupled groups is the group formed by removing one hydrogen atom from R²⁴ in formula (4) and the other is the group formed by removing one hydrogen atom from R²⁸ in formula (5), R²³ and R²⁷ may be taken together to form a ring.

Examples of the nitrogen-containing aromatic ring, in which the monovalent and divalent groups are coupled, include rings having a skeleton structure represented by the following formulae (C-1) to (C-11):

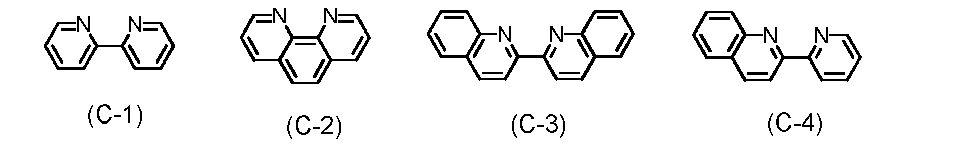

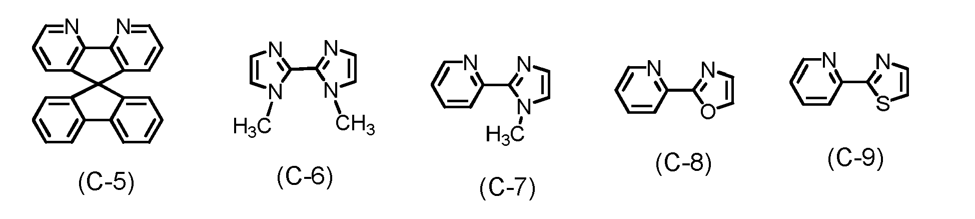

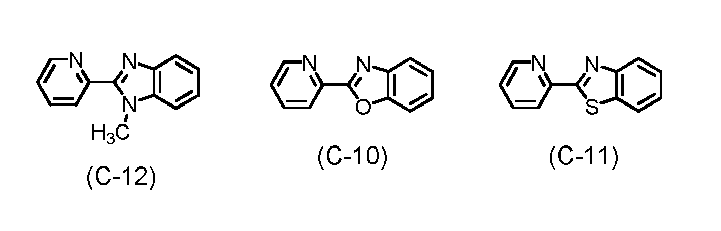

When the number of branches is two, typical examples of the nitrogen-containing aromatic ring ligand which has a dendritic molecular chain are as follows: 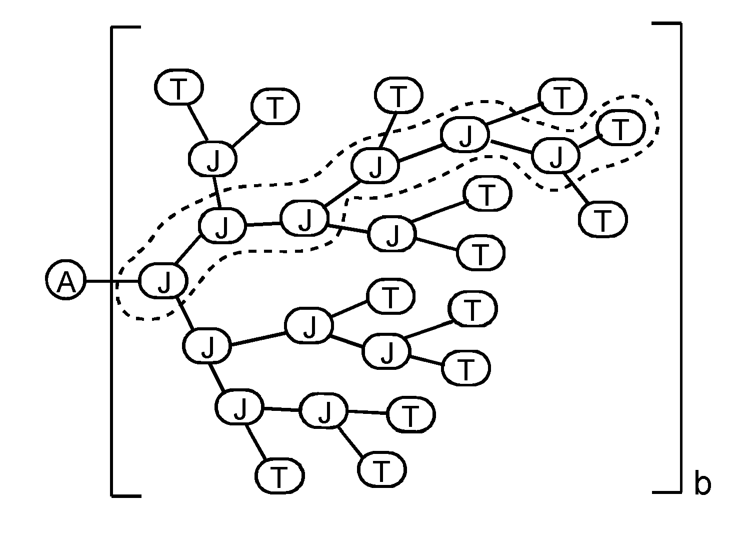 wherein A represents a nitrogen-containing aromatic ring, J represents a branched portion, T represents a terminal portion bonded to the branched portion, and b represents an integer of one or more; hereinafter, a molecular chain which has the most branched portions, as shown inside a dashed line, is referred to as "maximum branched molecular chain."

The number of the branched portion in the maximum branched molecular chain is preferably 2 or more. From the viewpoints of solubility to an organic solvent, the number is usually 50 or less, preferably 20 or less, more preferably 10 or less, further preferably 6 or less, and particularly preferably 4 or less.

Accordingly, the number of the branched portion in the maximum branched molecular chain is usually from 1 to 50, preferably from 1 to 20, more preferably from 2 to 10, further preferably from 2 to 6, and particularly preferably from 2 to 4.

Examples of the nitrogen-containing aromatic ring ligand which has a dendritic molecular chain include structures represented by the following formulae (B-1) to (B-15).

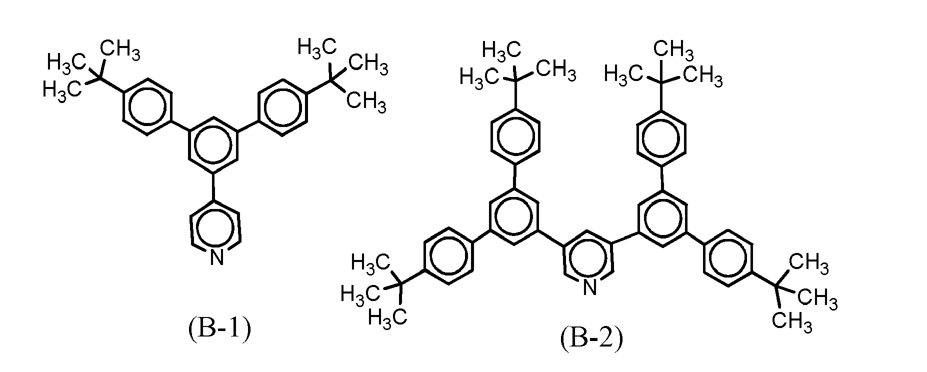

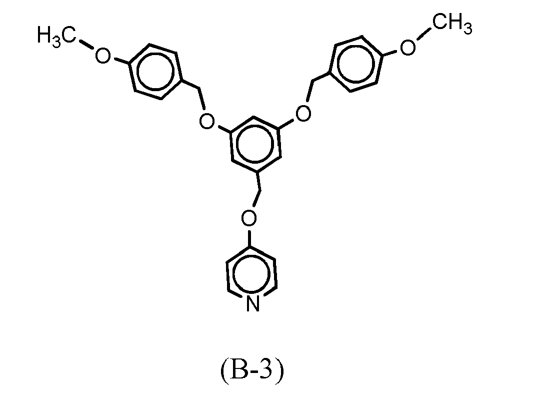

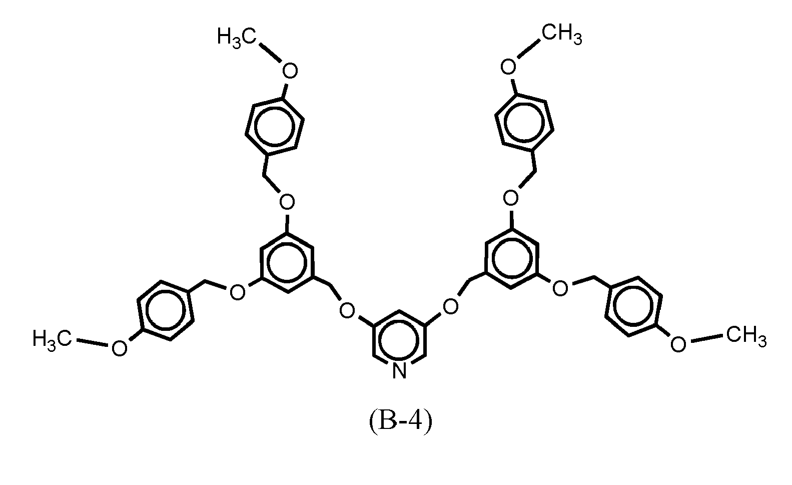

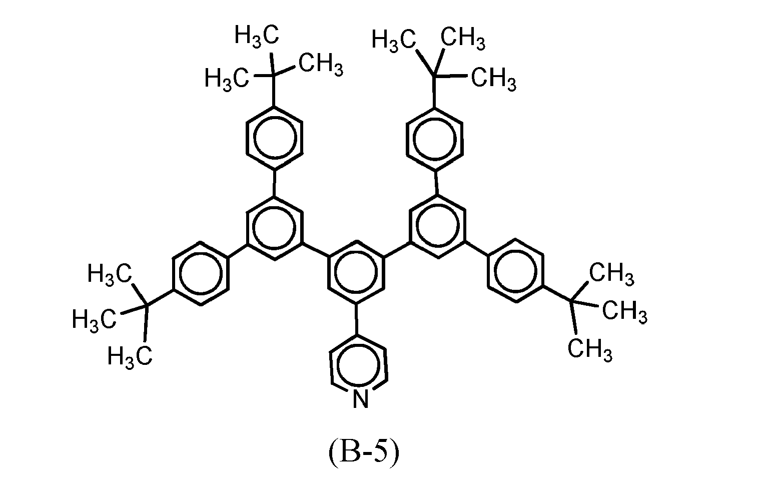

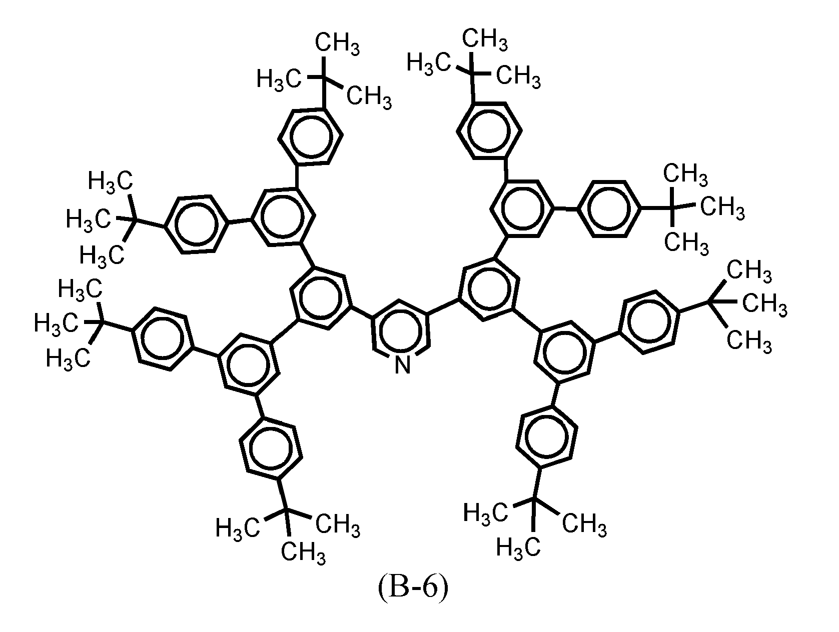

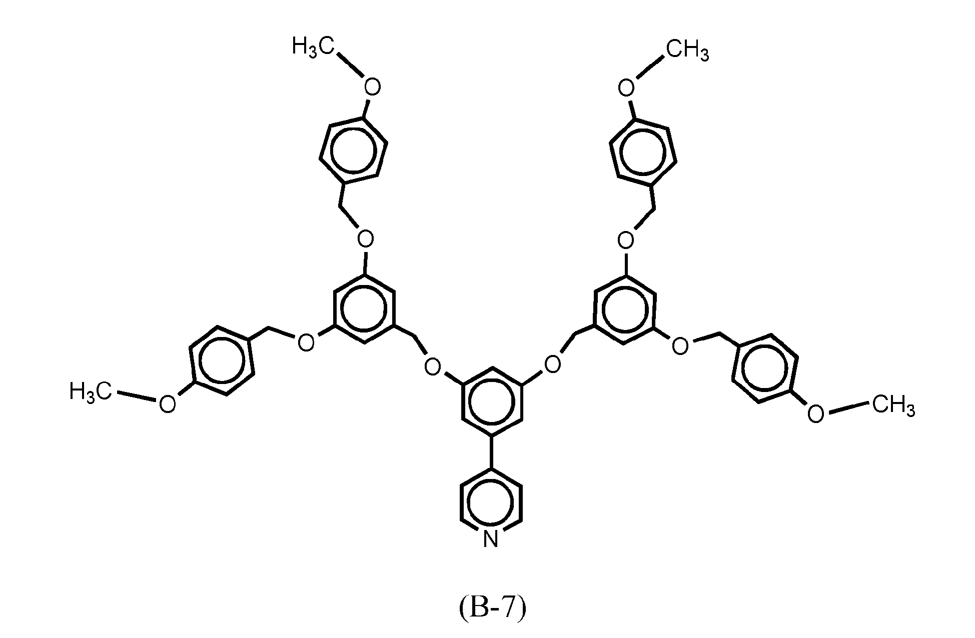

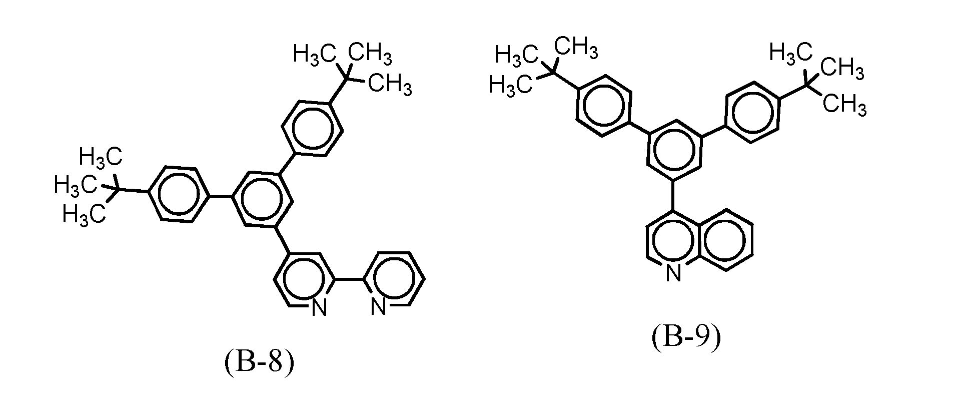

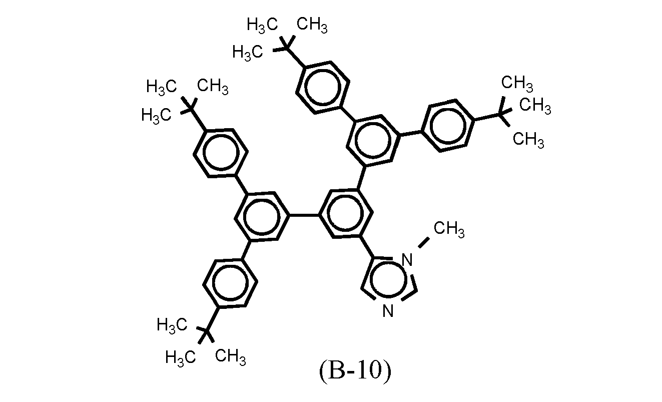

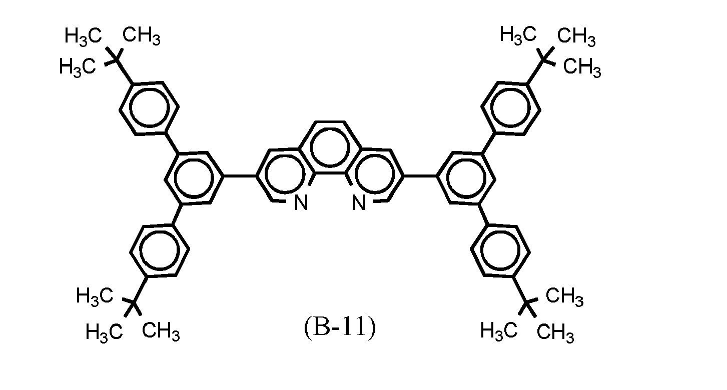

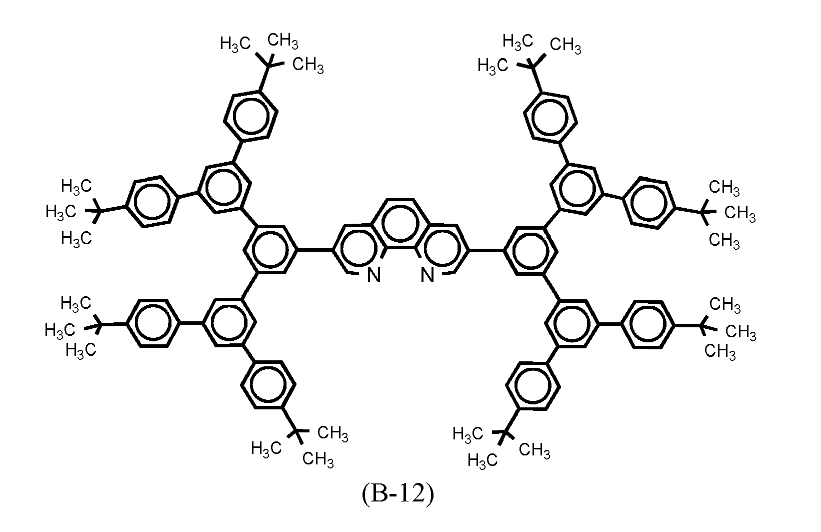

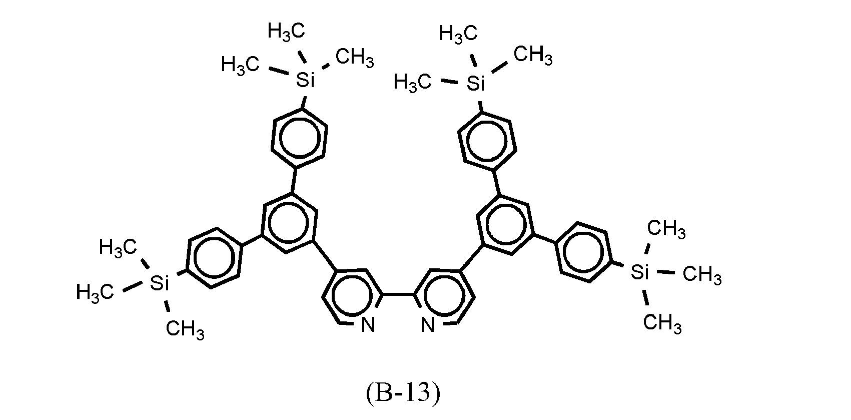

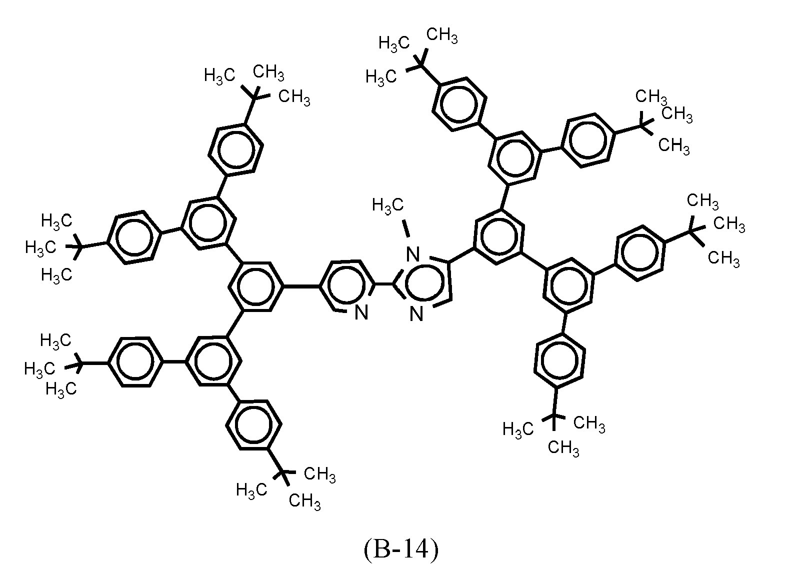

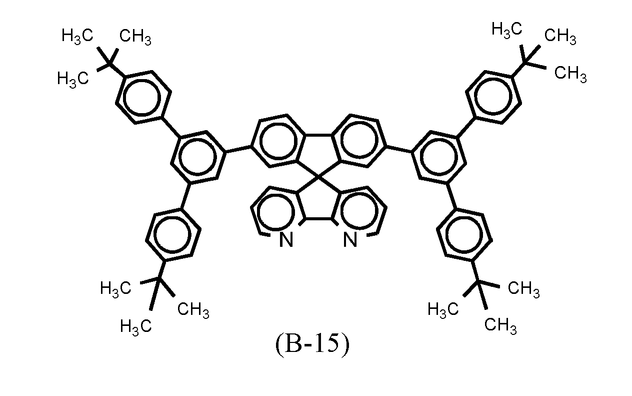

From the viewpoints of resource amount and cost efficiency, among the silver(I) ion and the copper(I) ion in the metal complex of the present invention, the copper(I) ion is more preferable.

The metal complex of the present invention optionally has a ligand other than the nitrogen-containing aromatic ring ligand and/or a counter ion, in addition to the nitrogen-containing aromatic ring ligand, and a copper(I) ion or a silver(I) ion.

Examples of the ligand other than the nitrogen-containing aromatic ring ligand include a heterocyclic compound, a phosphine compound, an amine compound, and a ligand having the following structure. The ligands represented by L1 to L81 optionally have a substituent.

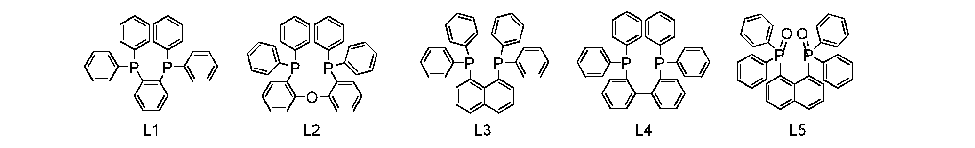

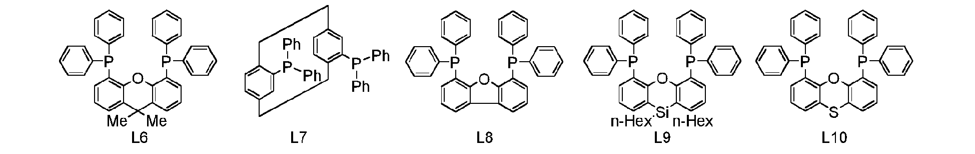

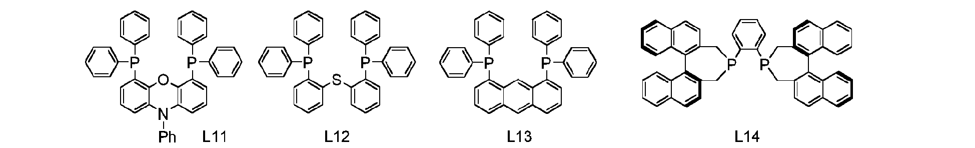

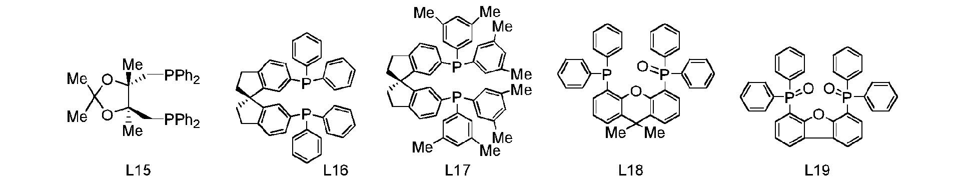

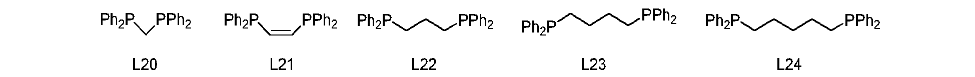

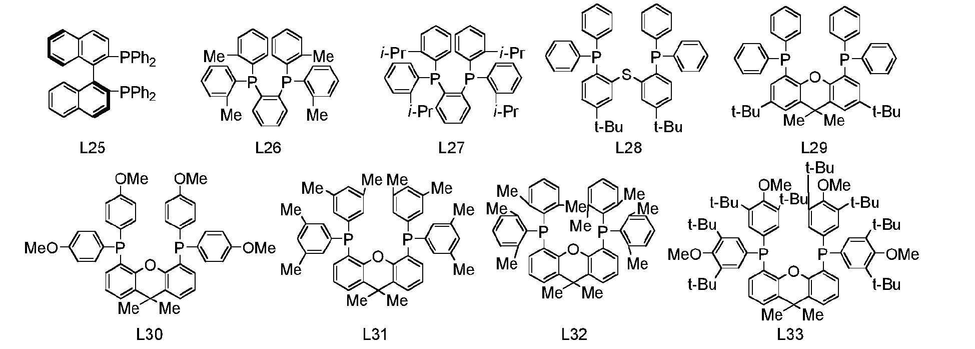

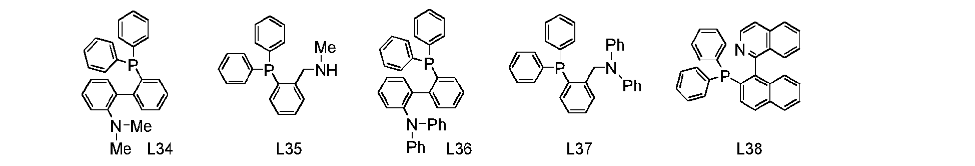

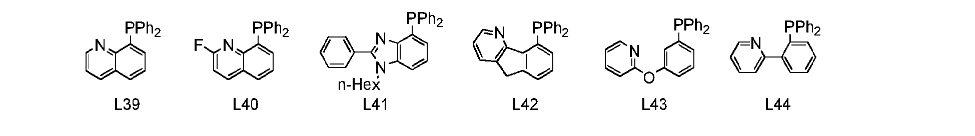

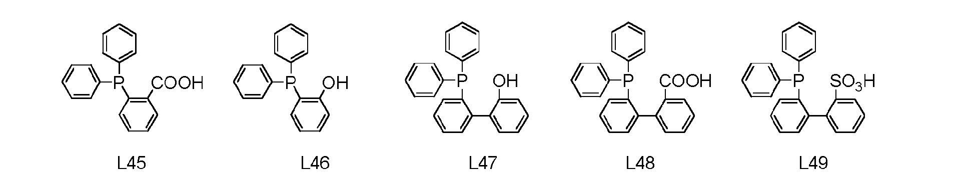

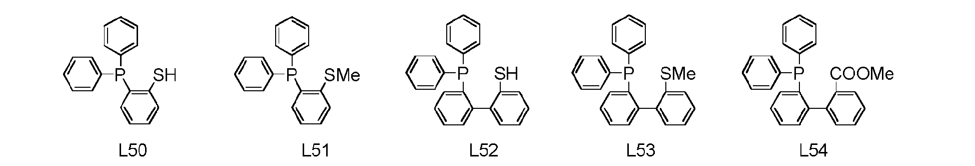

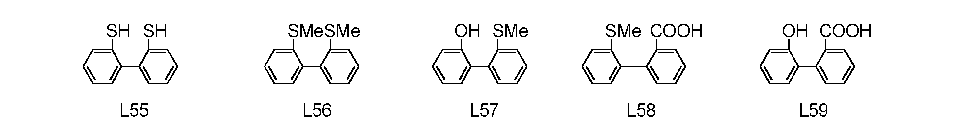

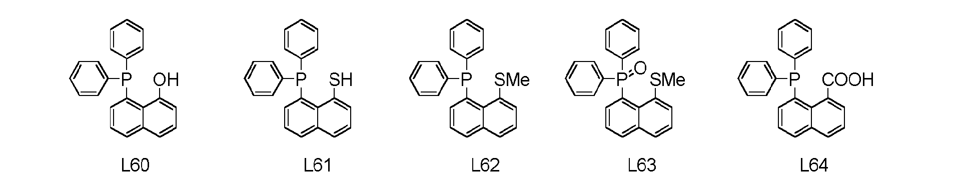

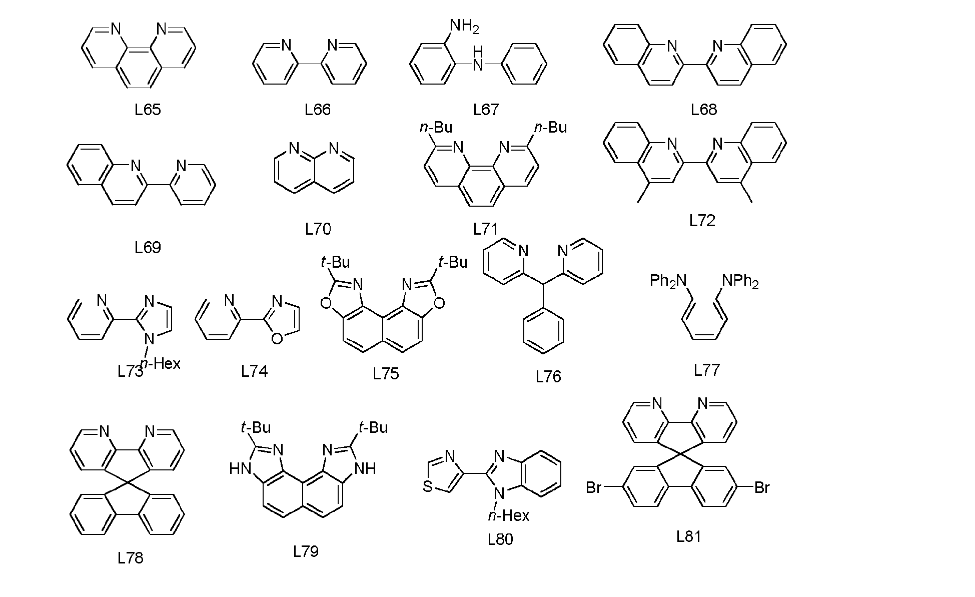

The heterocyclic compounds include pyridine, quinoline, pyrazole, imidazole, oxazole, thiazole, benzoimidazole, benzoxazole, benzothiazole, triazine, pyrimidine, pyrazine, piperidine, piperazine, morpholine, thiophene, furan, triazole, triazine, and acridine which optionally have a substituent. The heterocyclic compound are preferably pyridine, quinoline, imidazole, benzoimidazole, and triazine which optionally have a substituent.

The phosphine compounds include triphenylphosphine, propyldiphenylphosphine, tert-butyldiphenylphosphine, 1-butyldiphenylphosphine, 1-hexyldiphenylphosphine, cyclohexyldiphenylphosphine, dicyclohexylphenylphosphine, tricyclohexylphosphine, trimethylphosphine, tri(2-furyl)phosphine, tri(3-furyl)phosphine, tri(2-pyridyl)phosphine, tri(3-pyridyl)phosphine, tri(4-pyridyl)phosphine, 2-furyldiphenylphosphine, 3-furyldiphenylphosphine, 2-pyridyldiphenylphosphine, 3-pyridyldiphenylphosphine, 4-pyridyldiphenylphosphine, and the like. The phosphine compound is preferably triphenylphosphine, tricyclohexylphosphine, trimethylphosphine, tri(2-furyl)phosphine, tri(3-furyl)phosphine, tri(2-pyridyl)phosphine, tri(3-pyridyl)phosphine, tri(4-pyridyl)phosphine, 2-furyldiphenylphosphine, 3-furyldiphenylphosphine, 2-pyridyldiphenylphosphine, 3-pyridyldiphenylphosphine, or 4-pyridyldiphenylphosphine. The phosphine compound is more preferably triphenylphosphine. The phenyl group, the cyclohexyl group, the furyl group and the pyridyl group in the phosphine compound shown herein optionally have a substituent.

The amine compounds include triphenylamine, diphenylamine, propyldiphenylamine, tert-butyldiphenylamine, n-butyldiphenylamine, n-hexyldiphenylamine, cyclohexyldiphenylamine, dicyclohexylphenylamine, tricyclohexylamine, trimethylamine, dimethylamine, and the like. The amine compound is preferably triphenylamine, diphenylamine, tricyclohexylamine, trimethylamine, or dimethylamine. The phenyl group and the cyclohexyl group in the amine compound shown herein optionally have a substituent.

The ligands other than a ligand of a nitrogen-containing aromatic ring are preferably pyridine, quinoline, imidazole, benzoimidazole, triazine, triphenylphosphine, L1 to L33, and L65 to L71, and more preferably pyridine, imidazole, benzoimidazole, triphenylphosphine, L1, L2, L4, L6, L65, and L66.

The counter ions include a fluoride ion, a chloride ion, a bromide ion, an iodide ion, a sulfate ion, a nitrate ion, a carbonate ion, an acetate ion, a perchlorate ion, a tetrafluoroborate ion, a hexafluorophosphate ion, a hexafluoroantimony ion, a hexafluoroarsenate ion, a methanesulfonate ion, a trifluoromethanesulfonate ion, a trifluoroacetate ion, a benzenesulfonate ion, a p-toluenesulfonate ion, a dodecylbenzenesulfonate ion, a tetraphenylborate ion, and a tetrakis(pentafluorophenyl)borate ion. The counter ion is preferably a fluoride ion, a chloride ion, a bromide ion, an iodide ion, a sulfate ion, a nitrate ion, a perchlorate ion, a tetrafluoroborate ion, a hexafluorophosphate ion, a tetraphenylborate ion, or a tetrakis(pentafluorophenyl)borate ion. The counter ion is further preferably a chloride ion, a bromide ion, or an iodide ion. Herein, the counter ion represented by X may be a polymer compound having the structure of these ions in a repeating unit.

From the viewpoint of transport of charge, the metal complex of the present invention is further preferably represented by compositional formula (6): 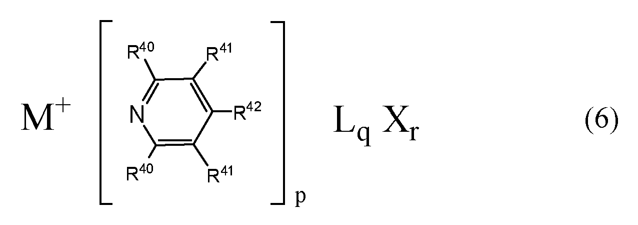 wherein M⁺ is a copper(I) ion or a silver(I) ion, L is a ligand, and X is a counter ion; p is a positive number, and q and r are each independently numerical numbers of 0 or more; R⁴⁰, R⁴¹, and R⁴² each independently represent a hydrogen atom, a halogen atom, a cyano group, a nitro group, a carboxyl group, an amino group, an acylamino group, a silyl group, a hydroxyl group, an acyl group, a hydrocarbyl group, a hydrocarbyloxy group, a hydrocarbylthio group, or a heterocyclic group, wherein the amino group, the silyl group, the hydrocarbyl group, the hydrocarbyloxy group, and the hydrocarbylthio group optionally have a substituent; at least one hydrogen atom in R⁴⁰, R⁴¹, and R⁴¹ is substituted with a group represented by formula (7): 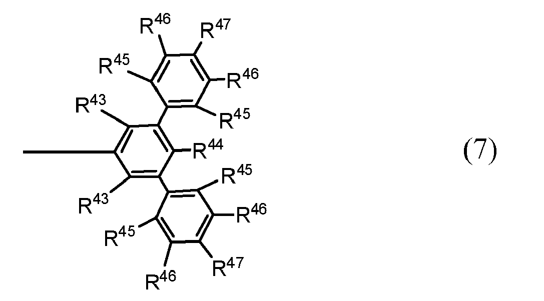 ; the respective R⁴⁰ or the respective R⁴¹ may be the same as or different from each other, and R⁴⁰ and R⁴¹, and R⁴¹ and R⁴² may be each taken together to form a ring; in formula (7), R⁴³, R⁴⁴, R⁴⁵, R⁴⁶, or R⁴⁷ is a hydrogen atom, a halogen atom, a cyano group, a nitro group, a carboxyl group, an amino group, an acylamino group, a silyl group, a hydroxyl group, an acyl group, a hydrocarbyl group, a hydrocarbyloxy group, or a hydrocarbylthio group, wherein the amino group, the silyl group, the hydrocarbyl group, the hydrocarbyloxy group and the hydrocarbylthio group optionally have a substituent; R⁴³, R⁴⁵, R⁴⁶, or R⁴⁷ which is plurally present may be the same as or different from each other; R⁴³ and R⁴⁵, R⁴⁴ and R⁴⁵, R⁴⁵ and R⁴⁶, or R⁴⁶ and R⁴⁷ may be each taken together to form a ring; and when L, X, and a structure represented by the following formula: 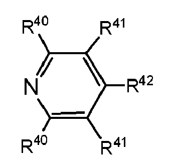 are plurally present, they may be the same as or different from each other.

L is a ligand other than the nitrogen-containing aromatic ring ligand, and specific examples and preferable examples thereof are as described above. X is a counter ion, and specific examples and preferable examples thereof are as described above.

Specific examples of the respective groups for R⁴⁰, R⁴¹, and R⁴² are as described above. The groups are preferably a hydrogen atom, a hydrocarbyl group, and a hydrocarbyloxy group, more preferably a hydrogen atom and a hydrocarbyl group, and further preferably a hydrogen atom.

Specific examples of the respective groups for R⁴³, R⁴⁴, and R⁴⁵ are as described above. The groups are preferably a hydrogen atom, a hydrocarbyl group, and a hydrocarbyloxy group, and more preferably a hydrogen atom.

Specific examples of the respective groups for R⁴⁶ and R⁴⁷ are as described above. The groups are preferably a hydrogen atom, a hydrocarbyl group, and a hydrocarbyloxy group, and more preferably a hydrocarbyl group.

At least one hydrogen atom in R⁴⁰, R⁴¹, and R⁴² is substituted with the group represented by formula (7). It is preferable that a hydrogen atom in R⁴¹ or R⁴² be substituted with the group represented by formula (7).

q and r preferably satisfy 0 < (q + r) ≤ 4, more preferably satisfy 0 < (q + r) ≤ 3, and further preferably satisfy 0 < (q + r) ≤ 2. p is preferably a numerical number of 0 < p ≤ 2.

From the viewpoint of transport of charge, the metal complex of the present invention is particularly preferably represented by compositional formula (8): 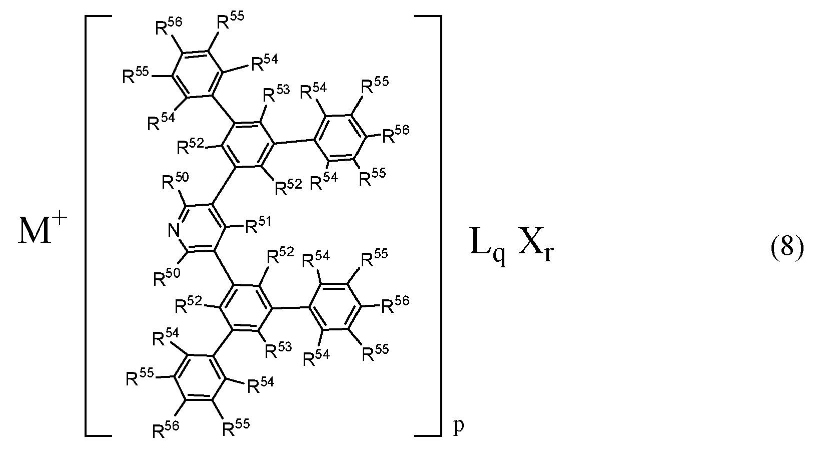 wherein M⁺ is a copper(I) ion or a silver(I) ion, L is a ligand, and X is a counter ion; p is a positive number, and q and r are each independently numerical numbers of 0 or more; R⁵⁰, R⁵¹, R⁵², R⁵³, R⁵⁴, R⁵⁵, or R⁵⁶ represents a hydrogen atom, a halogen atom, a cyano group, a nitro group, a carboxyl group, an amino group, an acylamino group, a silyl group, a hydroxyl group, an acyl group, a hydrocarbyl group, a hydrocarbyloxy group, or a hydrocarbylthio group, wherein the amino group, the silyl group, the hydrocarbyl group, the hydrocarbyloxy group, and the hydrocarbylthio group optionally have a substituent; R⁵⁰, R⁵², R⁵³, R⁵⁴, R⁵⁵, or R⁵⁶ which is plurally present may be the same as or different from each other; R⁵² and R⁵⁴, R⁵³ and R⁵⁴, R⁵⁴ and R⁵⁵, and R⁵⁵ and R⁵⁶ may be each taken together to form a ring; and when L, X, and a structure represented by the following formula: 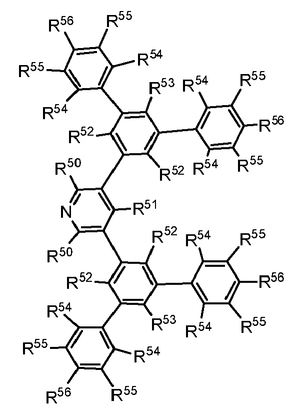 are plurally present, they may be the same as or different from each other.

Specific examples of the respective groups for R⁵⁰ and R⁵¹ are as described above. The groups are preferably a hydrogen atom, a hydrocarbyl group, and a hydrocarbyloxy group, more preferably a hydrogen atom and a hydrocarbyl group, and further preferably a hydrogen atom.

Specific examples of the respective groups for R⁵², R⁵³, and R⁵⁴ are as described above. The groups are preferably a hydrogen atom, a hydrocarbyl group, and a hydrocarbyloxy group, and more preferably a hydrogen atom.

Specific examples of the respective groups for R⁵⁵ and R⁵⁶ are as described above. The groups are preferably a hydrogen atom, a hydrocarbyl group, and a hydrocarbyloxy group, and more preferably a hydrocarbyl group.

From the viewpoint of transport of charge, the metal complex of the present invention is further preferably represented by compositional formula (9): 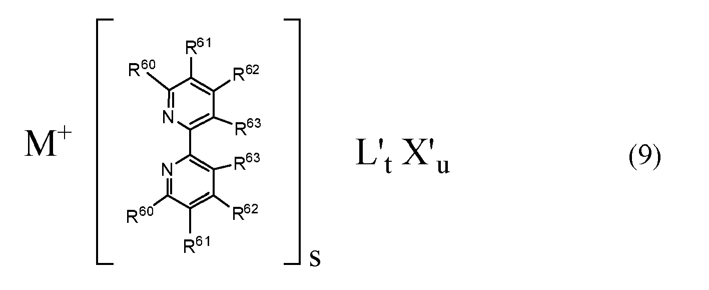 wherein M⁺ is a copper(I) ion or a silver(I) ion, L' is a ligand, and X' is a counter ion; s is a positive number, and t and u are each independently numerical numbers of 0 or more; R⁶⁰, R⁶¹, R⁶², and R⁶³ each independently represent a hydrogen atom, a halogen atom, a cyano group, a nitro group, a carboxyl group, an amino group, an acylamino group, a silyl group, a hydroxyl group, an acyl group, a hydrocarbyl group, a hydrocarbyloxy group, a hydrocarbylthio group, or a heterocyclic group, wherein the amino group, the silyl group, the hydrocarbyl group, the hydrocarbyloxy group and the hydrocarbylthio group optionally have a substituent; at least one hydrogen atom in R⁶⁰, R⁶¹, R⁶², and R⁶³ is substituted with a group represented by formula (7); the respective R⁶⁰, the respective R⁶¹, the respective R⁶², or the respective R⁶³ may be the same as or different from each other, and R⁶⁰ and R⁶¹, R⁶¹ and R⁶², R⁶² and R⁶³, and both R⁶³ may be each taken together to form a ring; and when L', X', and a structure represented by formula: 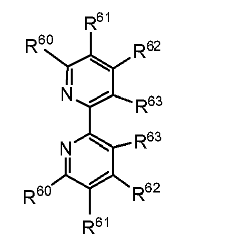 are plurally present, they may be the same as or different from each other,
or formula (10): 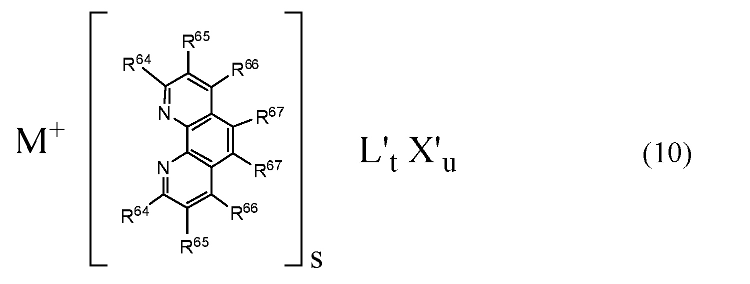 wherein M⁺ is a copper(I) ion or a silver(I) ion, L' is a ligand, and X' is a counter ion; s is a positive number, and t and u are each independently numerical numbers of 0 or more; R⁶⁴, R⁶⁵, R⁶⁶, and R⁶⁷ each independently represent a hydrogen atom, a halogen atom, a cyano group, a nitro group, a carboxyl group, an amino group, an acylamino group, a silyl group, a hydroxyl group, an acyl group, a hydrocarbyl group, a hydrocarbyloxy group, a hydrocarbylthio group, or a heterocyclic group, wherein the amino group, the silyl group, the hydrocarbyl group, the hydrocarbyloxy group and the hydrocarbylthio group optionally have a substituent; at least one hydrogen atom in R⁶⁴, R⁶⁵, R⁶⁶, and R⁶⁷ is substituted with the group represented by formula (7); the respective R⁶⁴, the respective R⁶⁵, the respective R⁶⁶, or the respective R⁶⁷ may be the same as or different from each other, and R⁶⁴ and R⁶⁵, R⁶⁵ and R⁶⁶, R⁶⁶ and R⁶⁷, and both R⁶⁷ may be each taken together to form a ring; when L', X', and a structure represented by formula: 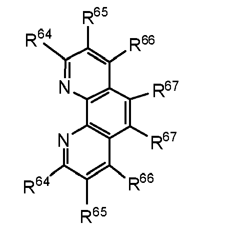 are plurally present, they may be the same as or different from each other,
or formula (11): 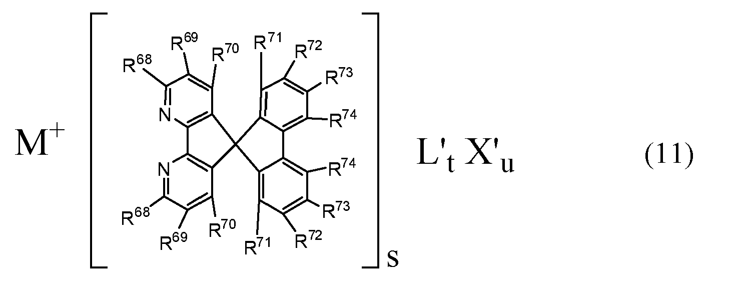 wherein M⁺ is a copper(I) ion or a silver(I) ion, L' is a ligand, and X' is a counter ion; s is a positive number, and t and u are each independently numerical numbers of 0 or more; R⁶⁸, R⁶⁹, R⁷⁰, R⁷¹, R⁷², R⁷³, and R⁷⁴ each independently represent a hydrogen atom, a halogen atom, a cyano group, a nitro group, a carboxyl group, an amino group, an acylamino group, a silyl group, a hydroxyl group, an acyl group, a hydrocarbyl group, a hydrocarbyloxy group, a hydrocarbylthio group, or a heterocyclic group, wherein the amino group, the silyl group, the hydrocarbyl group, the hydrocarbyloxy group, and the hydrocarbylthio group optionally have a substituent; at least one hydrogen atom in R⁷¹, R⁷², R⁷³, and R⁷⁴ is substituted with a group represented by formula (7); the respective R⁶⁸, the respective R⁶⁹, the respective R⁷⁰, the respective R⁷¹, the respective R⁷², the respective R⁷³, or the respective R⁷⁴ may be the same as or different from each other, and R⁶⁸ and R⁶⁹, R⁶⁹ and R⁷⁰, R⁷¹ and R⁷², R⁷² and R⁷³, R⁷³ and R⁷⁴, and both R⁷⁴ may be each taken together to form a ring; and when L', X', and a structure represented by formula: 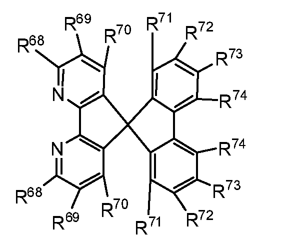 are plurally present, they may be the same as or different from each other.

L' is a ligand other than the nitrogen-containing aromatic ring ligand, and specific examples and preferable examples thereof are as described above. X' is a counter ion, and specific examples and preferable examples thereof are as described above.

t and u preferably satisfy 0 < (t + u) ≤ 3, more preferably 0 < (t + u) ≤ 2, and further preferably 0 < (t + u) ≤ 1. s is preferably a numerical number of 0 < s ≤ 2.

Specific examples of the respective groups for R⁶⁰, R⁶¹, R⁶², R⁶³, R⁶⁴, R⁶⁵, R⁶⁶, R⁶⁷, R⁶⁸, R⁶⁹, R⁷⁰, R⁷¹, R⁷², R⁷³, and R⁷⁴ are as described above. The groups are preferably a hydrogen atom, a hydrocarbyl group, and a hydrocarbyloxy group, more preferably a hydrogen atom and a hydrocarbylthio group, and further preferably a hydrogen atom.

At least one of R⁶⁰, R⁶¹, R⁶², and R⁶³ is preferably a group represented by formula (7). At least one of R⁶¹, R⁶², and R⁶³ is more preferably a group represented by formula (7). At least one of R⁶¹ and R⁶² is further preferably a group represented by formula (7).

At least one of R⁶⁴, R⁶⁵, R⁶⁶, and R⁶⁷ is preferably a group represented by formula (7). At least one of R⁶⁵, R⁶⁶, and R⁶⁷ is more preferably a group represented by formula (7) .

At least one of R⁷¹, R⁷², R⁷³, and R⁷⁴ is preferably a group represented by formula (7). At least one of R⁷¹, R⁷², and R⁷³ is preferably a group represented by formula (7). At least one of R⁷² and R⁷³ is further preferably a group represented by formula (7).

From the viewpoint of transport of charge, the metal complex of the present invention is particularly preferably represented by compositional formula (12): 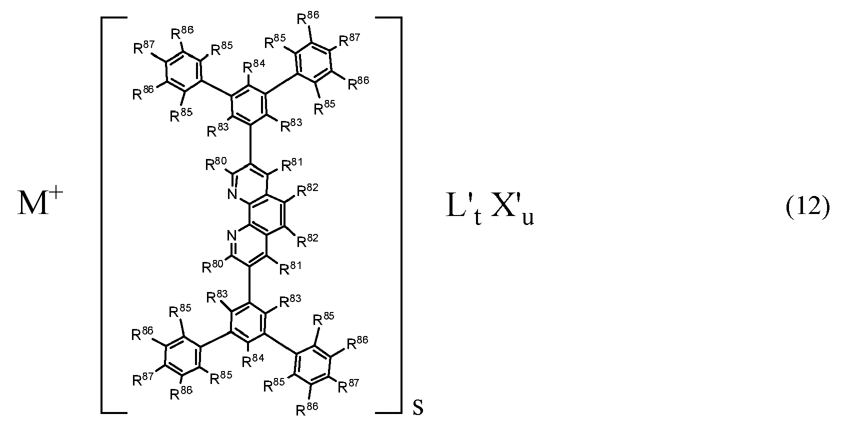 wherein M⁺ is a copper(I) ion or a silver(I) ion, L' is a ligand, and X' is a counter ion; s is a positive number, and t and u are each independently numerical numbers of 0 or more; R⁸⁰, R⁸¹, R⁸², R⁸³, R⁸⁴, R⁸⁵, R⁸⁶, and R⁸⁷ each independently represent a hydrogen atom, a halogen atom, a cyano group, a nitro group, a carboxyl group, an amino group, an acylamino group, an imido group, a silyl group, a hydroxyl group, an acyl group, a hydrocarbyl group, a hydrocarbyloxy group, or hydrocarbylthio group, wherein the amino group, the silyl group, the hydrocarbyl group, the hydrocarbyloxy group, and the hydrocarbylthio group optionally have a substituent; R⁸⁰, R⁸¹, R⁸², R⁸³, R⁸⁴, R⁸⁵, R⁸⁶, or R⁸⁷ which is plurally present may be the same as or different from each other; R⁸³ and R⁸⁵, R⁸⁴ and R⁸⁵, R⁸⁵ and R⁸⁶, and R⁸⁶ and R⁸⁷ may be each taken together to form a ring; and when L', X', and a structure represented by formula: 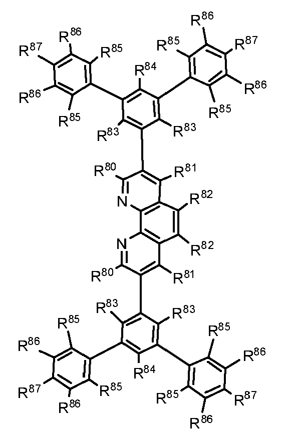 are plurally present, they may be the same as or different from each other.

Specific examples of the respective groups for R⁸⁰, R⁸¹, and R⁸² are as described above. The groups are preferably a hydrogen atom, a hydrocarbyl group, and a hydrocarbyloxy group, more preferably a hydrogen atom and a hydrocarbylthio group, and further preferably a hydrogen atom.

Specific examples of the respective groups for R⁸³, R⁸⁴, and R⁸⁵ are as described above. The groups are preferably a hydrogen atom, a hydrocarbyl group, and a hydrocarbyloxy group, and more preferably a hydrogen atom.

Specific examples of the respective groups for R⁸⁶ and R⁸⁷ are as described above. The groups are preferably a hydrogen atom, a hydrocarbyl group, and a hydrocarbyloxy group, and more preferably a hydrocarbyl group.

From the viewpoint of transport of charge, the metal complex of the present invention is particularly preferably represented by compositional formula (13): 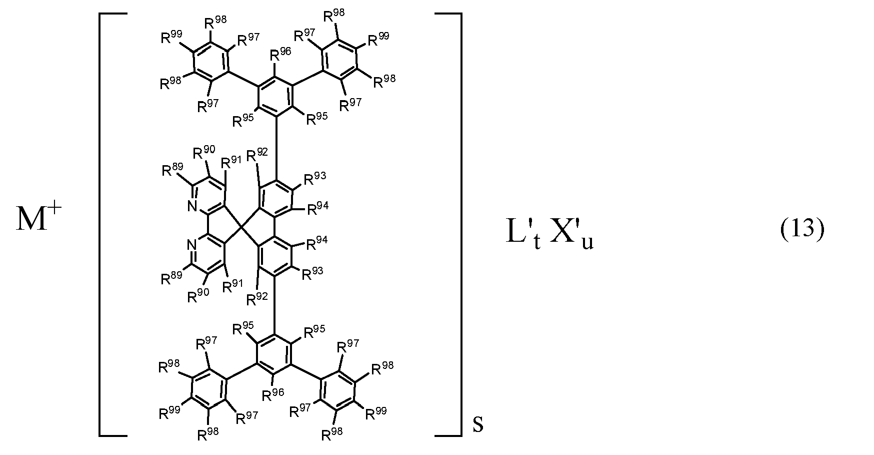 wherein M⁺ is a copper(I) ion or a silver(I) ion, L' is a ligand, and X' is a counter ion; s is a positive number, and t and u are each independently numerical numbers of 0 or more; R⁸⁹, R⁹⁰, R⁹¹, R⁹², R⁹³, R⁹⁴, R⁹⁵, R⁹⁶, R⁹⁷, R⁹⁸, or R⁹⁹ each independently represents a hydrogen atom, a halogen atom, a cyano group, a nitro group, a carboxyl group, an amino group, an acylamino group, an imido group, a silyl group, a hydroxyl group, an acyl group, a hydrocarbyl group, a hydrocarbyloxy group, or a hydrocarbylthio group, wherein the amino group, the silyl group, the hydrocarbyl group, the hydrocarbyloxy group, and the hydrocarbylthio group optionally have a substituent; R⁸⁹, R⁹⁰, R⁹¹, R⁹², R⁹³, R⁹⁴, R⁹⁵, R⁹⁶, R⁹⁷, R⁹⁸, or R⁹⁹ which is plurally present may be the same as or different from each other; R⁸⁹ and R⁹⁰, R⁹⁰ and R⁹¹, R⁹² and R⁹⁵, R⁹³ and R⁹⁴, both R⁹⁴, R⁹³ and R⁹⁵, R⁹⁵ and R⁹⁷, R⁹⁶ and R⁹⁷, R⁹⁷ and R⁹⁸, and R⁹⁸ and R⁹⁹ may be each taken together to form a ring; and when L', X', and a structure represented by formula: 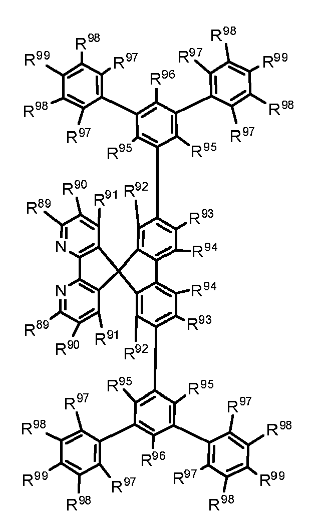 are plurally present, they may be the same as or different from each other.

Specific examples of the respective groups for R⁸⁹, R⁹⁰, and R⁹¹ are as described above. The groups are preferably a hydrogen atom, a hydrocarbyl group, and a hydrocarbyloxy group, more preferably a hydrogen atom and a hydrocarbylthio group, and further preferably a hydrogen atom.

Specific examples of the respective groups for R⁹², R⁹³, R⁹⁴, R⁹⁵, R⁹⁶, and R⁹⁷ are as described above. The groups are preferably a hydrogen atom, a hydrocarbyl group, and a hydrocarbyloxy group, and more preferably a hydrogen atom.

Specific examples of the respective groups for R⁹⁸ and R⁹⁹ are as described above. The groups are preferably a hydrogen atom, a hydrocarbyl group, and a hydrocarbyloxy group, and more preferably a hydrocarbyl group.

Further, the metal complex of the present invention is preferably a light-emitting metal complex. The "Light-emitting" means a property in which light is emitted by an external field (light, electric field, magnetic field, pressure, etc.).

The metal complex of the present invention includes the following metal complexes.

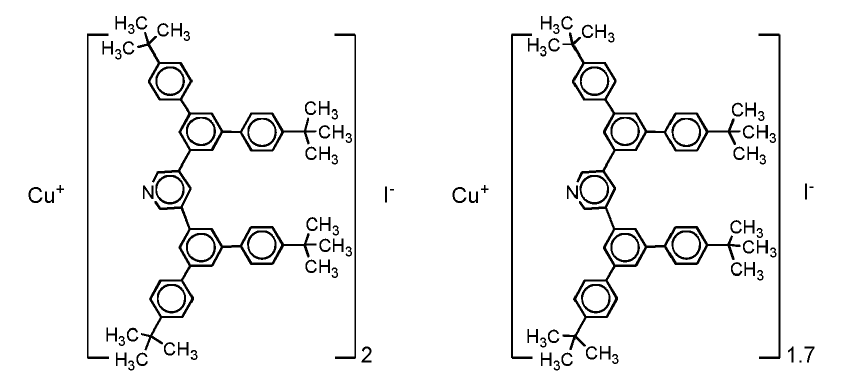

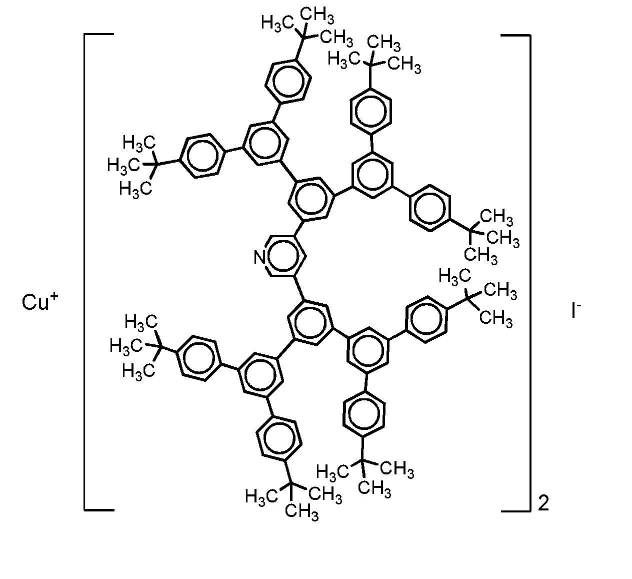

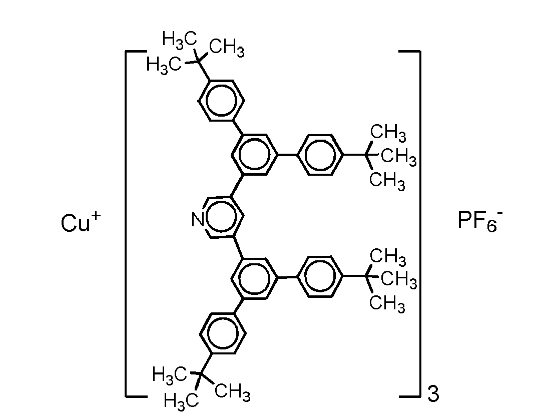

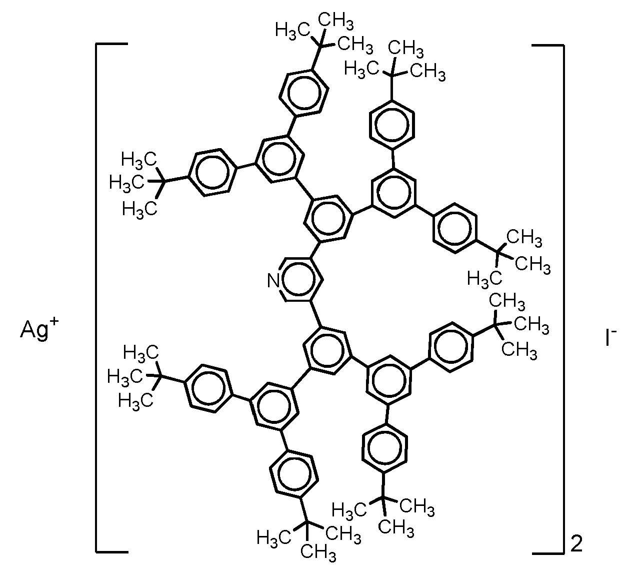

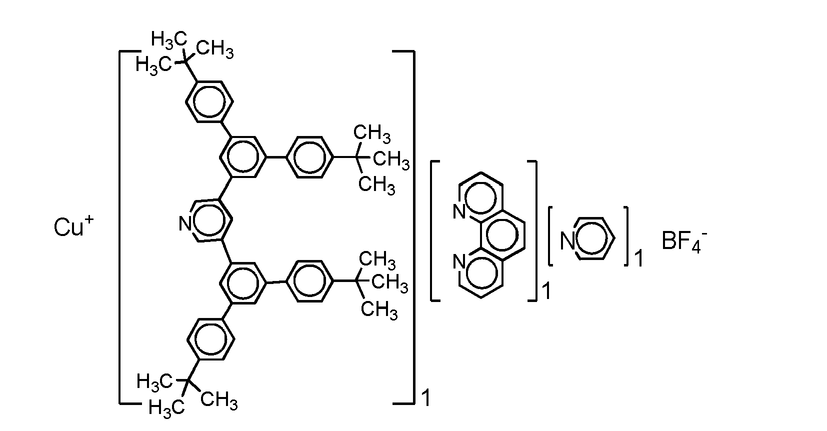

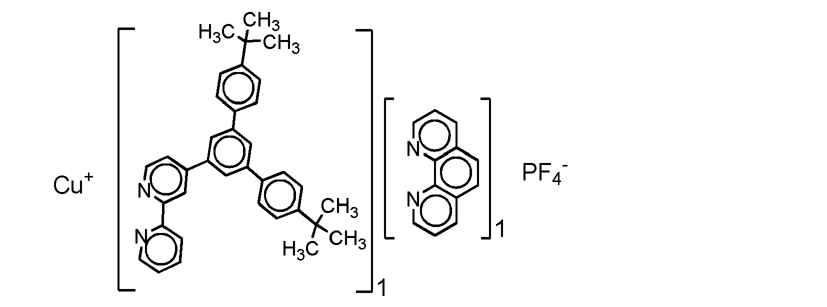

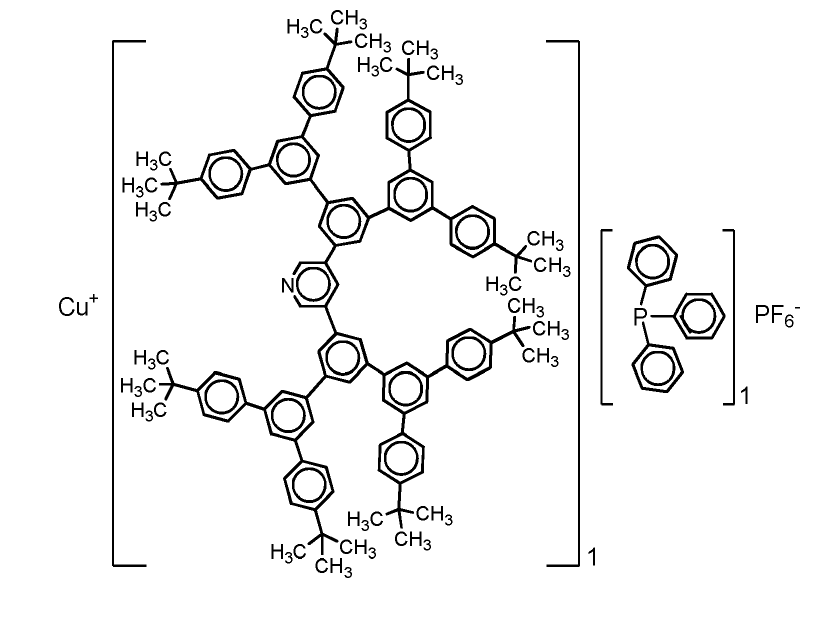

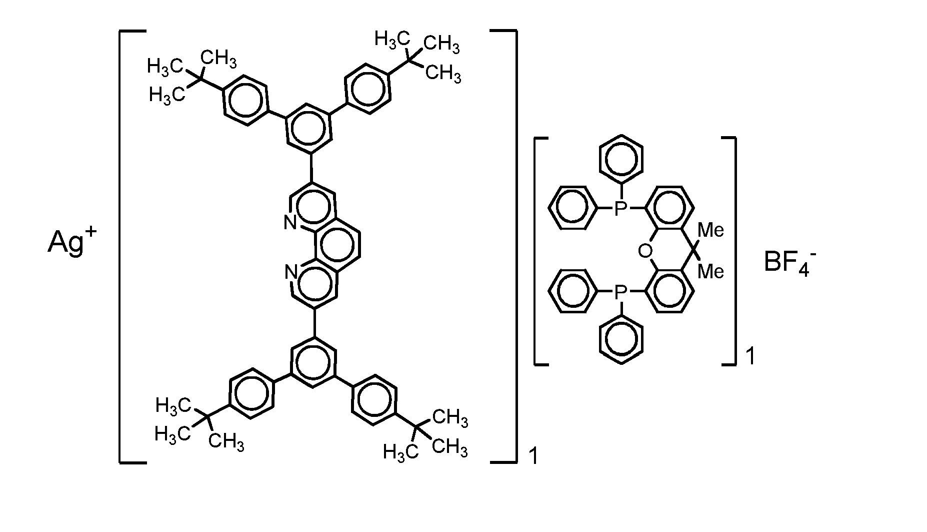

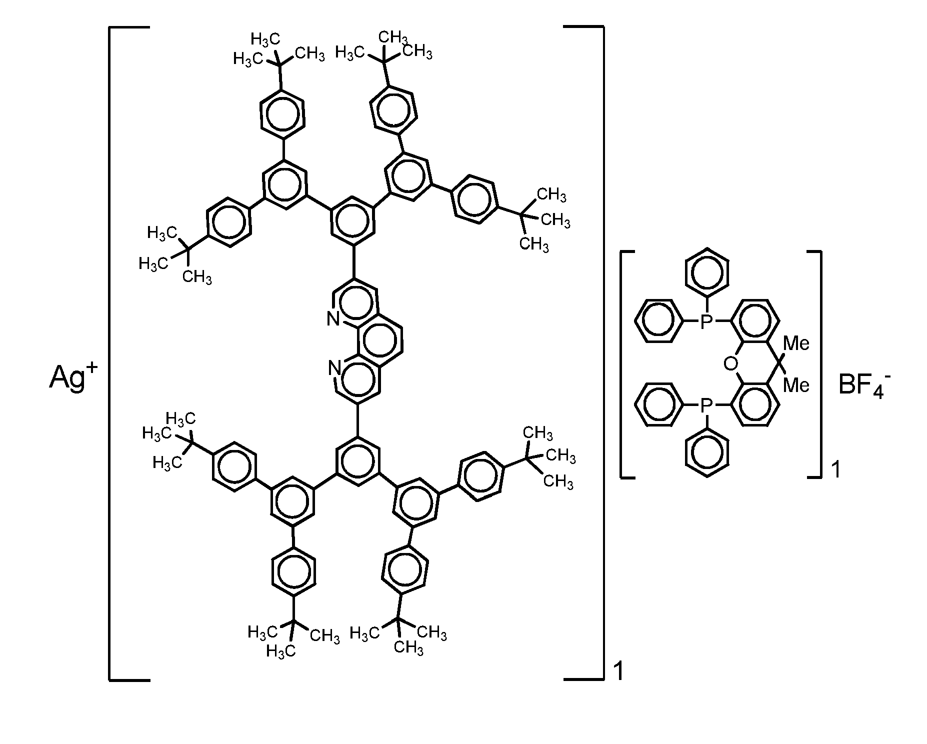

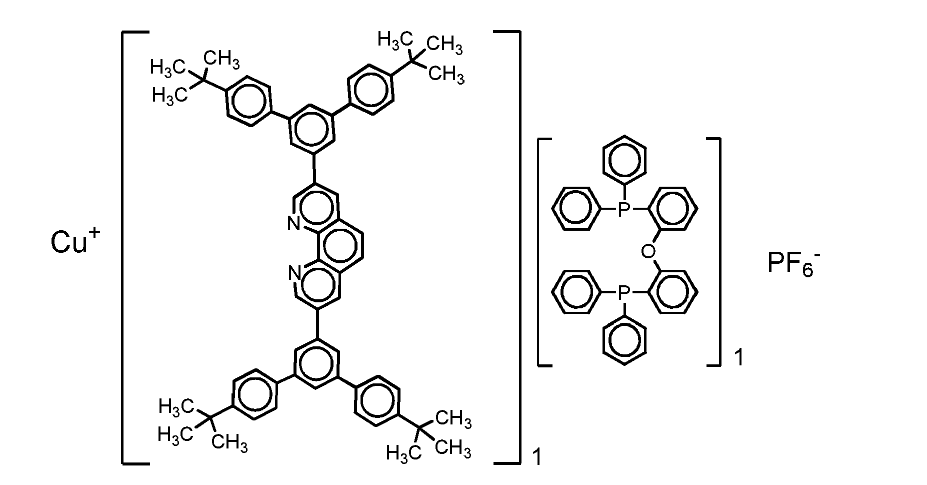

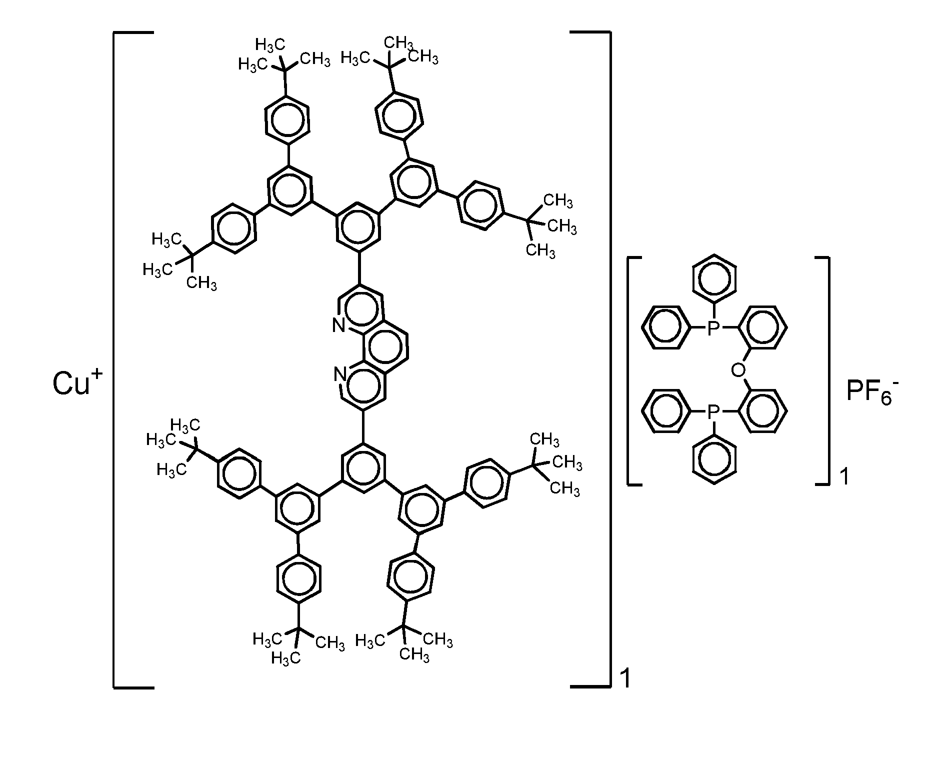

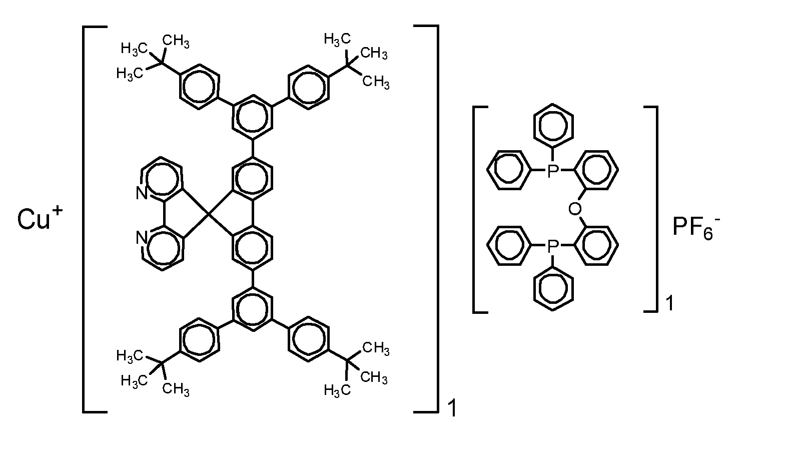

The metal complex of the present invention can be easily obtained by mixing a corresponding metal salt with a compound which is converted into a nitrogen-containing aromatic ring ligand in a solvent at room temperature, and then collecting the resulting precipitate or distilling off the solvent from the resulting solution.

In the mixing, in order to dissolve the corresponding metal salt and the nitrogen-containing aromatic ring ligand evenly in the solvent, or in the case of a solution having high viscosity, to stirring the solution easily, an aqueous solvent such as a buffer, and an organic solvent may be used. An organic solvent is preferable.

Examples of the organic solvent include chlorinated hydrocarbon solvents such as chloroform, methylene chloride, 1,2-dichloroethane, 1,1,2-trichloroethane, chlorobenzene, and o-dichlorobenzene, ether solvents such as tetrahydrofuran and dioxane, aromatic hydrocarbon solvents such as toluene and xylene, aliphatic hydrocarbon solvents such as cyclohexane, methyl cyclohexane, pentane, hexane, heptane, octane, nonane, and decane, ketone solvents such as acetone, methyl ethyl ketone, and cyclohexanone, ester solvents such as ethyl acetate, butyl acetate, and ethyl cellosolve acetate, polyhydric alcohol such as ethylene glycol, ethylene glycol monobutyl ether, ethylene glycol monoethyl ether, ethylene glycol monomethyl ether, dimethoxyethane, propylene glycol, diethoxymethane, triethylene glycol monoethyl ether, glycerin, and 1,2-hexanediol, and derivatives thereof, alcohol solvents such as methanol, ethanol, propanol, isopropanol, and cyclohexanol, sulfoxide solvents such as dimethyl sulfoxide, and amide solvents such as N-methyl-2-pyrrolidone and N,N-dimethylformamide. The organic solvent may be used alone, or two or more organic solvents may be used in combination.

An excitation source such as visible light, ultraviolet ray, vacuum ultraviolet ray, light having a wavelength shorter than that of vacuum ultraviolet ray, electron injection, electric field, heat, stress, ultrasound, and radiation other than electromagnetic wave, can be used to cause the metal complex of the present invention to emit light. Accordingly, the metal complex of the present invention can be used for an ultraviolet light-emitting device such as a three-wavelength fluorescent lamp, an ultraviolet-visible excitation light-emitting device such as a white LED, an electron excitation light-emitting device such as CRT, an electron injection light-emitting device such as an organic EL, and excitation light-emitting device by radiation other than electromagnetic wave such as film patch. For example, when the metal complex of the present invention is used for an organic EL, the metal complex of the present invention can be used by dissolving or dispersing it in an organic solvent, and making a thin film by application.

The metal complex of the present invention is useful for a conducting material, and is also useful for a charge transport material and a charge injection material. In addition, the metal complex of the present invention is useful for materials for an organic transistor device and an organic photoelectric conversion device, raw materials for synthesis of compounds, materials for an additive, a modifier, a cell, a catalyst and a sensor, and the like.

When the device of the present invention is a light-emitting device, examples thereof include a device having an electron transport layer or a hole block layer between a cathode and a light-emitting layer, a device having a hole transport layer or an electron block layer between an anode and a light-emitting layer, a device having an electron transport layer or a hole block layer between a cathode and a light-emitting layer and a hole transport layer or an electron block layer between an anode and a light-emitting layer, and the like.

A specific structure of the device of the present invention is shown as follows. Herein, the mark "/" shows that the respective layers are adjacent to each other and laminated. Hereinbelow, the same meaning is applied to the mark as above.
a) anode / (charge injection layer) / light-emitting layer / (charge injection layer) / cathode
b) anode / (charge injection layer) / hole transport layer / light-emitting layer / (charge injection layer) / cathode
c) anode / (charge injection layer) / light-emitting layer / electron transport layer / (charge injection layer) /cathode
d) anode / (charge injection layer) / hole transport layer / light-emitting layer / electron transport layer / (charge injection layer) / cathode

Further, two or more layers of the light-emitting layer, hole transport layer, and electron transport layer may be each independently provided in the device of the present invention.

In general, among charge transport layers (hole transport layers and electron transport layers) provided adjacent to electrodes, a layer having a function of improving charge injection efficiency from the electrode and having an effect of decreasing a drive voltage of the device is sometimes referred to as a charge injection layer (hole injection layer and electron injection layer). The devices having a charge injection layer include a device which includes a charge injection layer adjacent to a cathode, and a device which includes a charge injection layer adjacent to an anode.

In order to enhance adhesion properties to the electrode and improve injection of charge from the electrode, the device of the present invention may be provided with an insulation layer which is adjacent to the electrode and has a film thickness of 2 nm or less. Materials used in the insulation layer include metal fluorides, metal oxides, organic insulation material, and the like. The devices including an insulation layer having a film thickness of 2 nm or less include a device including the insulation layer adjacent to a cathode, a device including the insulation layer adjacent to an anode, and the like.

In order to enhancing adhesion properties of an interface and prevent the layers from mixing, in the device of the present invention, buffer layers having an average film thickness of 2 nm or less may be provided between the electrode and light-emitting layer so as to be adjacent to the electrode, and on the charge transport layer, the light-emitting layer, and the interface.

The device of the present invention is not limited to the structures shown above, and includes a device, in which the order and number of layers, and the thickness of each layer are appropriately determined in consideration of light-emitting efficiency, photoelectric efficiency, or device lifetime.

The respective layers of device of the present invention will be described.

The light-emitting layer of device of the present invention has a function capable of injecting holes from an adjacent layer on an anode side and injecting electrons from an adjacent on a cathode side under application of an electric field, a function of transporting the injected charges (electrons and holes) by the force of the electric field, and a function of providing a field of recombination of the electrons and holes to cause light-emission. The light-emitting layer of an organic electroluminescent device of the present invention preferably contains the above metal complexes, and may contain a host material which can handle the metal complex as a guest material.

The host materials include a compound having a fluorene skeleton, a compound having a carbazole skeleton, a compound having a diarylamine skeleton, a compound having a pyridine skeleton, a compound having a pyrazine skeleton, a compound having a triazine skeleton, a compound having an arylsilane skeleton, and the like. T1 (energy level of the lowest triplet excitation state) of the host material is preferably higher than T1 of the guest material, and it is further preferable that a difference between T1 of the host and guest materials be larger than 0.2 eV. The host material may be a low molecular compound or a macromolecular compound. Further, the host material and a light-emitting material such as a metal complex are mixed and the mixture is applied, or is subjected to codeposition, so as to form a light-emitting layer in which the light-emitting material is doped to the host material.

The content of the metal complex in a layer containing the metal complex is typically from 0.01 to 100% by weight, preferably from 0.1 to 50% by weight, and more preferably from 1 to 30% by weight with respect to the entire layer.

The materials for the hole transport layer include compounds described in Japanese Patent Application Laid-Open Nos. Sho. 63-70257, Sho. 63-175860, Hei. 2-135359, Hei. 2-135361, Hei. 2-209988, Hei. 3-37992, and Hei. 3-152184. Specifically, the materials include polyvinylcarbazole and derivatives thereof, polysilane and derivatives thereof, a polysiloxane derivative having an aromatic amine compound group in a side chain or a main chain, a pyrazoline derivative, an arylamine derivative, a stilbene derivative, a triphenyldiamine derivative, polyanion and derivatives thereof, polyaminophene and derivatives thereof, polypyrrole and derivatives thereof, poly(p-phenylenevinylene) and derivatives thereof, and poly(2,5-thienylenevinylene) and derivatives thereof.

The film thickness of the hole transport layer is appropriately determined so that light-emitting efficiency or photoelectric efficiency and drive voltage are proper values. The optimum values are varied depending on materials to be used, and the film thickness should be so thick that a pinhole cannot be generated. When the film thickness is too thick, the drive voltage of the device tends to increase in the hole transport layer. Therefore, the film thickness of the hole transport layer is preferably 1 nm to 1 µm, more preferably from 2 to 500 nm, and particularly preferably from 5 to 200 nm.

The materials for the electron transport layer include compounds described in Japanese Patent Application Laid-Open Nos. Sho. 63-70257, Sho. 63-175860, Hei. 2-135359, Hei. 2-135361, Hei. 2-209988, Hei. 3-37992, and Hei. 3-152184. Specifically, the materials include an oxadiazole derivative, anthraquinodimethane and derivatives thereof, benzoquinone and derivatives thereof, naphthoquinone and derivatives thereof, anthraquinone and derivatives thereof, tetracyanoanthraquinodimethane and derivatives thereof, a fluorenone derivative, diphenyldicyanoethylene and derivatives thereof, a diphenoquinone derivative, 8-hydroxyquinoline and metal complexes of derivatives thereof, polyquinoline and derivatives thereof, polyquinoxaline and derivatives thereof, polyfluorene and derivatives thereof, and the like.

The film thickness of the electron transport layer is appropriately determined so that light-emitting efficiency or photoelectric efficiency and drive voltage are proper values. The optimum values are varied depending on materials to be used, and the film thickness should be so thick that a pinhole cannot be generated. When the film thickness is too thick, the drive voltage of the device tends to increase in the electron transport layer. Therefore, the film thickness of the electron transport layer is preferably from 1 nm to 1 µm, more preferably from 2 to 500 nm, and particularly preferably from 5 to 200 nm.

The device of the present invention is usually formed using a substrate. An electrode is formed on one surface of the substrate, and the respective layers of the device are formed on the other surface. The substrate used in the present invention may be any substrates that are not chemically changed when the electrode and the respective layers of the device are formed. Examples of the substrate include substrates made of glass, plastics, polymer film, silicon, and the like. When the substrate is opaque, it is preferable that a counter electrode to be formed be transparent or translucent.

In general, at least one of an anode and a cathode is transparent or translucent, and it is preferable that the anode be transparent or translucent. Further, when the device of the present invention is a photoelectric conversion device, at least one electrode of an anode and a cathode may be formed as an interdigitated array. In this case, the electrode may be opaque, but it is preferable that the electrode be transparent or translucent.

The materials for the anode include a conductive metal oxide film, a translucent metal thin film, and the like. Specifically, the materials include indium oxide, zinc oxide, and tin oxide, and a composite thereof (indium/tin/oxide (ITO) and indium/zinc/oxide, etc.), antimony/tin/oxide (NESA), gold, platinum, silver, copper, and the like. Among them, ITO, indium/zinc/oxide, and tin oxide are preferable. Further, as the anode, an organic transparent conductive film made of polyanion and derivatives thereof, and polyaminophene and derivatives thereof may be used.

The methods for forming an anode include a vacuum deposition method, a sputtering method, an ion-plating method, a plating method, and the like.

The film thickness of an anode can be appropriately determined in consideration of light permeability and electrical conductivity. For example, the film thickness is preferably from 10 nm to 10 µm, more preferably from 20 nm to 1 µm, and particularly preferably from 50 to 500 nm.

The material used in the cathode is preferably a material having a small work function. The materials include metal such as lithium, sodium, potassium, rubidium, cesium, beryllium, magnesium, calcium, strontium, barium, aluminum, scandium, vanadium, zinc, yttrium, indium, cerium, samarium, europium, terbium, and ytterbium; alloy of two or more kinds of metal among them; alloys of one or more kinds of metal among them, and one or more kinds of metal of gold, silver, platinum, copper, manganese, titanium, cobalt, nickel, tungsten, and tin; graphite; graphite intercalation compound; and the like. The alloys include magnesium-silver alloy, magnesium-indium alloy, magnesium-aluminum alloy, indium-silver alloy, lithium-aluminum alloy, lithium-magnesium alloy, lithium-indium alloy, calcium-aluminum alloy, and the like.

The methods for forming an anode and a cathode include a vacuum deposition method, a sputtering method, and a laminating method for thermocompression-bonding a metal thin film, and the like. Further, a cathode having a two or more-layered structure may be formed.

The film thickness of a cathode can be appropriately determined in consideration of electrical conductivity and durability. For example, the film thickness is preferably from 10 nm to 10 µm, more preferably from 20 nm to 1 µm, and particularly preferably from 50 to 500 nm.

Further, a layer consisting of conductive polymer, or a layer having an average film thickness of 2 nm or less, which is formed from a metal oxide, a metal fluoride, an organic insulating material or the like may be provided between the cathode and organic layer.

In the device of the present invention, a protective layer and/or a protective cover for protecting the device may be formed after formation of a cathode in order to protect a device against outside and use the device stably for a long time.

The materials used in such a protective layer include a polymer compound, metal oxide, metal fluoride, metal boride, and the like. Further, the protective covers include a glass board, a plastic board applying a treatment for lowering water permeability to a surface, and the like. Among them, it is preferable that the protective cover be bonded with the device using a thermosetting resin or a photosetting resin, to seal the device.

The charge injection layers include a layer containing a conductive polymer, a layer containing a material having an ionization potential which is intermediate between those of an anode material and a hole transport material contained in the hole transport layer (when the layer is provided between the anode and hole transport layer), a layer containing a material having an electron affinity which is intermediate between those of a cathode material and an electron transport material contained in the electron transport layer (when the layer is provided between the cathode and electron transport layer), and the like.

The materials used in the charge injection layer may be selected depending on relation of materials for electrodes and adjacent layers. Specifically, the materials used in the charge injection layer include polyaniline and derivatives thereof, polyaminophene and derivatives thereof, polypyrrole and derivatives thereof, polyphenylenevinylene and derivatives thereof, polythienylenevinylene and derivatives thereof, polyquinoline and derivatives thereof, polyquinoxaline and derivatives thereof, conductive polymers such as a polymer having an aromatic amine structure in a main chain or a side chain, metal phthalocyanine (copper phthalocyanine, etc.), carbon, and the like.

The film thickness of the charge injection layer is preferably from 1 to 100 nm, more preferably from 1 to 50 nm, and further more preferably from 1 to 10 nm.

The present invention also relates to compounds represented by formulae (14) and (15): 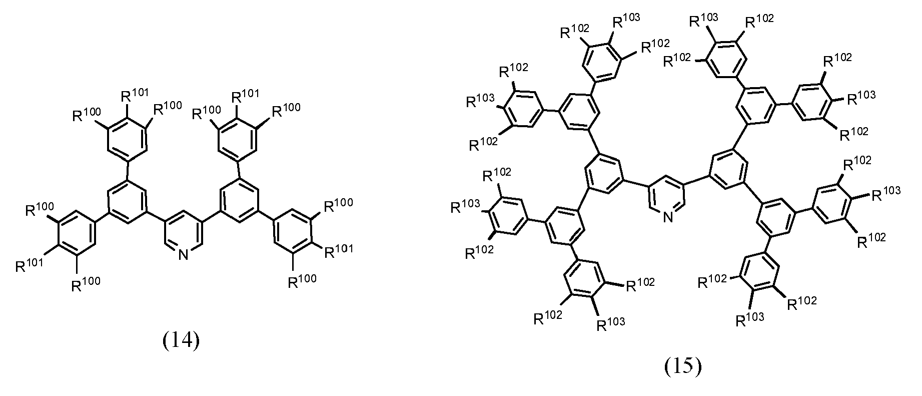 wherein R¹⁰⁰, R¹⁰¹, R¹⁰², and R¹⁰³ each independently represent a hydrogen atom, a halogen atom, a cyano group, a nitro group, a carboxyl group, an amino group, an acylamino group, an imido group, a silyl group, a hydroxyl group, an acyl group, a hydrocarbyl group, a hydrocarbyloxy group, or a hydrocarbylthio group.

Specific examples of the respective groups for R¹⁰⁰ and R¹⁰² are as described above. The groups are preferably a hydrogen atom, a hydrocarbyl group, and a hydrocarbyloxy group, more preferably a hydrogen atom and a hydrocarbyl group, and further preferably a hydrogen atom.

Specific examples of the respective groups for R¹⁰¹ and R¹⁰³ are as described above. The groups are preferably a hydrogen atom, a hydrocarbyl group, and a hydrocarbyloxy group, more preferably a hydrogen atom and a hydrocarbyl group, and further preferably a hydrocarbyl group.

The present invention is also formula (16): 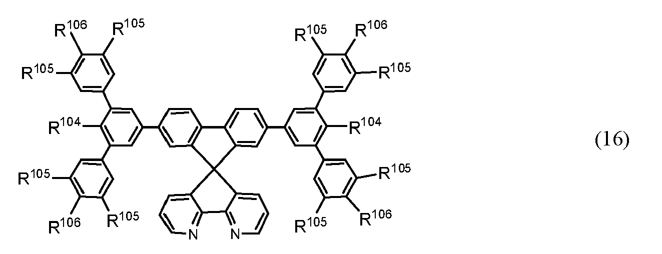 wherein R¹⁰⁴, R¹⁰⁵, and R¹⁰⁶ each independently represent a hydrogen atom, a halogen atom, a cyano group, a nitro group, a carboxyl group, an amino group, an acylamino group, an imido group, a silyl group, a hydroxyl group, an acyl group, a hydrocarbyl group, a hydrocarbyloxy group, or a hydrocarbylthio group.

Specific examples of the respective groups for R¹⁰⁴ and R¹⁰⁵ are as described above. The groups are preferably a hydrogen atom, a hydrocarbyl group, and a hydrocarbyloxy group, more preferably a hydrogen atom and a hydrocarbyl group, and further preferably a hydrogen atom.

Specific examples of the respective groups for R¹⁰⁶ are as described above. The groups are preferably a hydrogen atom, a hydrocarbyl group, and a hydrocarbyloxy group, more preferably a hydrogen atom and a hydrocarbyl group, and further preferably a hydrocarbyl group.

### EXAMPLES

Hereinafter, the present invention will be described in detail using Examples.

An emission spectrum was measured at an excitation wavelength of 365 nm with a fluorescence spectrometer (manufactured by JOBINYVON-SPEX, trade name: Fluorolog-Tau3). An excitation lifetime in an emission peak wavelength of the emission spectrum was determined.

### EXAMPLE 1

### <Synthesis of Compound B1>

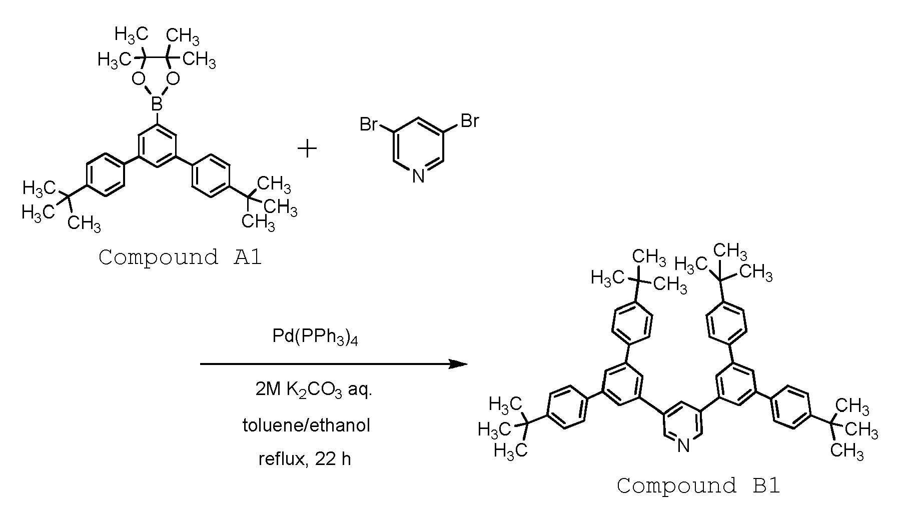

The compound A1 was synthesized in accordance with the method described in Chinese Journal of Chemistry 24, 1631-1638 (2006).

Then, toluene (3 mL), ethanol (1 mL), and a 2M potassium carbonate aqueous solution (1.5 mL) were added to the compound A1 (100 mg, 0.213 mmol), 3,5-dibromopyridine (21 mg, 0.0889 mmol), and tetrakis(triphenylphosphine)palladium(0) (10 mg, 0.00889 mmol) under an argon atmosphere, and the mixture was stirred for 22 hours while heating at 85°C. After cooling to room temperature, the resulting reaction solution was separated into an organic layer and an aqueous layer. The organic layer was concentrated and dried to obtain a crude product. The crude product was purified by a silica gel column chromatography to obtain the compound B1 (68 mg).
¹H NMR (300 MHz, CDCl₃): δ 8.96 (s, 2H), 8.22 (s, 1H), 7.85 (s, 2H), 7.81 (s, 4H), 7.64 (d, J = 6.6Hz, 8H), 7.51 (d, J = 6.6 Hz, 8H), 1.38 (s, 36H).
¹³C NMR (75 MHz, CDCl₃): δ 150.79, 147.33, 142.60, 138.68, 137.90, 136.77, 133.16, 127.00, 125.87, 124.79, 34.58, 31.35.
DART-MS(M/Z): found 760.29, calcd for M⁺ 759.48.

### <Synthesis of Compound C1>

Copper iodide(I) (2 mg, 0.0105 mmol) was added to a solution (2 mL) of the compound B1 (16 mg, 0.0210 mmol) in chloroform under an argon atmosphere, and the mixture was stirred at room temperature for 12 hours, resulting in precipitation. The precipitate was collected to obtain the metal complex C1 (15.7 mg) represented by compositional formula: 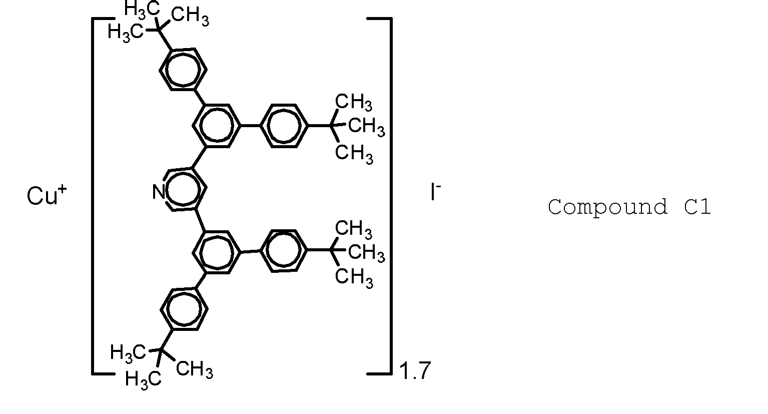 Elemental Analysis Found(%) C: 77.95, H: 6.86, N: 1.61, Cu: 3.76, Calcd for C1(%) C: 78.30, H: 7.03, N: 1.60, Cu: 4.36

The metal complex C1 emitted yellow light under ultraviolet excitation (365 nm), the emission peak wavelength was 583 nm, and the excitation lifetime was 1.0 µS.

The metal complex C1 was easily dissolved in chloroform, toluene, xylene, and hexane at room temperature.

When the metal complex C1 was left to stand in air at 100°C for 7 hours, change in emission intensity was hardly observed. Further, when the metal complex C1 was heated with a thermogravimetric and differential thermal analyzer (manufactured by Seiko Instruments Inc., trade name: EXSTAR-6300), decrease in mass in a temperature range of 30 to 400°C was not observed.

### EXAMPLE 2

### <Synthesis of Compound B2>

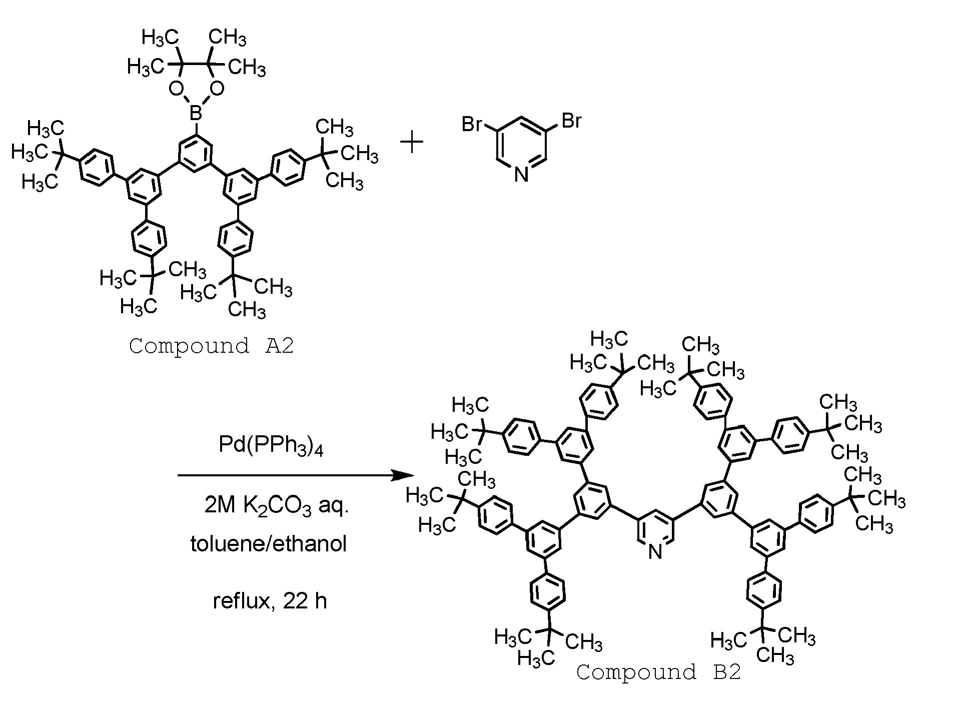

The compound A2 was synthesized in accordance with the method described in Chinese Journal of Chemistry 24, 1631-1638 (2006).

Then, toluene (2 mL), ethanol (0.7 mL), and a 2M potassium carbonate aqueous solution (1 mL) were added to the compound A2 (100 mg, 0.113 mmol), 3,5-dibromopyridine (12 mg, 0.0507 mmol), and tetrakis(triphenylphosphine)palladium(0) (6 mg, 0.00507 mmol) under an argon atmosphere, and the mixture was stirred for 22 hours while heating at 85°C. After cooling to room temperature, the resulting reaction solution was separated into an organic layer and an aqueous layer. The organic layer was concentrated and dried to obtain a crude product. The crude product was purified by a silica gel column chromatography to obtain the compound B2 (69 mg, yield: 85%).
¹H NMR (300 MHz, CDCl₃): δ 9.01 (s, 2H), 8.30 (s, 1H), 8.04 (s, 2H), 7.95 (s, 4H), 7.87 (s, 8H), 7.82 (s, 4H), 7.64 (d, J = 7.3 Hz, 16H), 7.49 (d, J = 7.3 Hz, 16H), 1.36 (s, 72H).
¹³C NMR(75MHz, CDCl₃): δ 150.61, 147.64, 143.02, 142.41, 141.63, 139.17, 138.15, 136.86, 133.55, 127.04, 125.81, 125.68, 125.48, 125.00, 34.56, 31.36.

### <Synthesis of Metal complex C2>

Copper(I) iodide (1 mg, 0.00525 mmol) was added to a solution (2 mL) of the compound B2 (17 mg, 0.0105 mmol) in chloroform under an argon atmosphere, and the mixture was stirred at room temperature for 12 hours. The resulting solution was concentrated and dried to obtain a metal complex C2 (18 mg) represented by compositional formula:

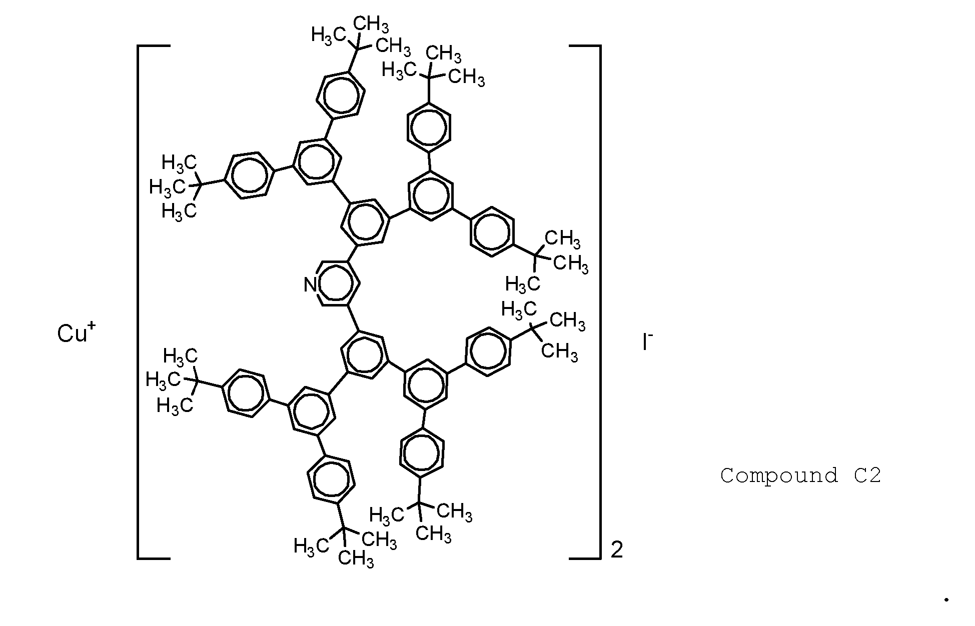

The metal complex C2 emitted yellow-green light under ultraviolet excitation (365 nm), the emission peak wavelength was 546 nm, and the excitation lifetime was 0.9 µs.

The metal complex C2 was easily dissolved in chloroform, toluene, xylene, and hexane at room temperature.

When the metal complex C2 was left to stand in air at 100°C for 7 hours, change in emission intensity was hardly observed. Further, when the metal complex C2 was heated with a thermogravimetric and differential thermal analyzer (manufactured by Seiko Instruments Inc., trade name: EXSTAR-6300), decrease in mass in a temperature range of 30 to 400°C was not observed.

### EXAMPLE 3

### <Synthesis of Metal complex C3>

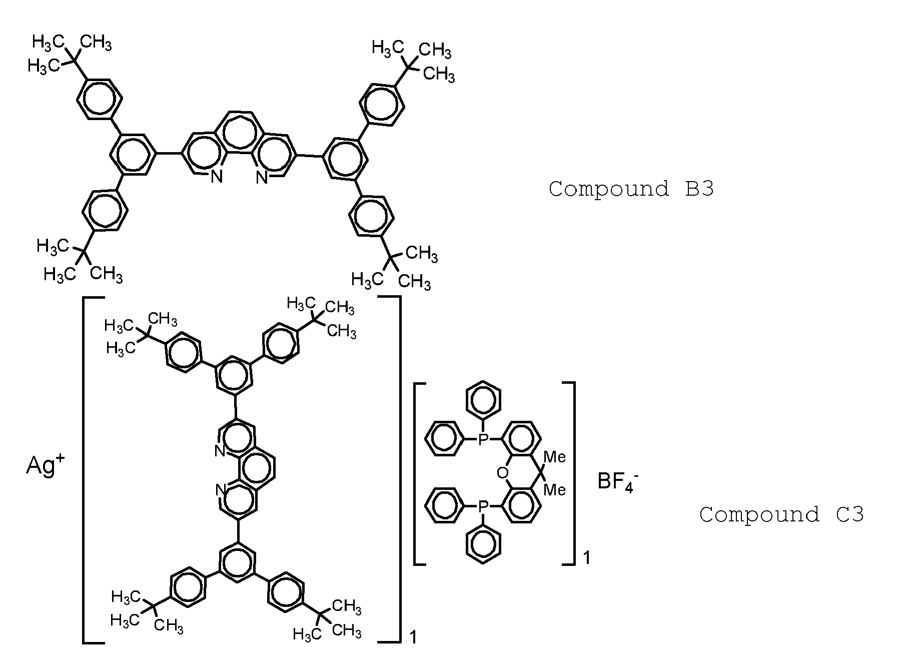

The compound B3 was synthesized in accordance with the method described in Tetrahedron Letters 41, 6809-6813 (2000) .

Silver(I) tetrafluoroborate (6.10 mg, 0.0313 mmol) and 4,5-bis(diphenylphosphino)-9,9-dimethylxanthene (18.1 mg, 0.0313 mmol) were stirred in dehydrated methylene chloride (2 mL) under an argon atmosphere at room temperature for 5 minutes. The compound B3 (31.0 mg, 0.0360 mmol) was added to the reaction solution, and the homogeneous reaction solution was stirred under heating at 40°C for 5 minutes. Vapor of diethyl ether was diffused gradually in the reaction solution to perform recrystallization. The obtained crystal was dried under vacuum at 50°C for 5 hours to obtain the metal complex C3 (35.0 mg, yield: 68%).
¹H NMR (300 MHz, CDCl₃): δ 8.78 (s, 2H), 8.67 (s, 2H), 8.27 (s, 2H), 7.89 (s, 2H), 7.69 (d, J = 8.4 Hz, 8H), 7.58-7.56 (m, 12H), 7.26-7.19 (m, 8H), 7.12-7.08 (m, 16H), 6.99-6.94 (m, 2H), 6.61 (br, 2H), 1.42 (s, 36H).
³¹P NMR (122 MHz, CDCl₃): δ-4.8 (d, J(³¹P-¹⁰⁷Ag, ¹⁰⁹Ag) = 375, 433 Hz).
Elemental Analysis Found(%) C: 75.54, H: 5.77, N: 1.61, Calcd for C3(%) C: 75.69, H: 5.92, N: 1.71

The metal complex C3 emitted green light under ultraviolet excitation (365 nm), the emission peak wavelength was 553 nm, and the excitation lifetime was 1.8 µs.

The metal complex C3 was easily dissolved in chloroform at room temperature.

When the metal complex C3 was left to stand in air at 100°C for 7 hours, change in emission intensity was hardly observed.

### <Manufacture of Organic EL device of Metal complex C3>

A poly(ethylene dioxythiophene) / poly(styrene sulfonate) solution (Bayer AG, trade name: Baytron P) was subjected to a spin coating method, to make a film having a thickness of 65 nm on a glass substrate on which an ITO film having a thickness of 150 nm had been formed by a sputtering method. The film was dried on a hot plate at 200°C for 10 minutes. The metal complex C3 was spin coated to make a film. The film thickness was about 70 nm. The formed film was dried under an atmosphere of nitrogen gas at 130°C for 10 minutes, and then about 4 nm of barium and about 100 nm of aluminum were sequentially deposited to make a cathode to manufacture an organic EL device.

When a voltage is applied to the obtained organic EL device, electroluminescence having a peak near 560 nm was observed.

### EXAMPLE 4

### <Synthesis of Metal complex C4>

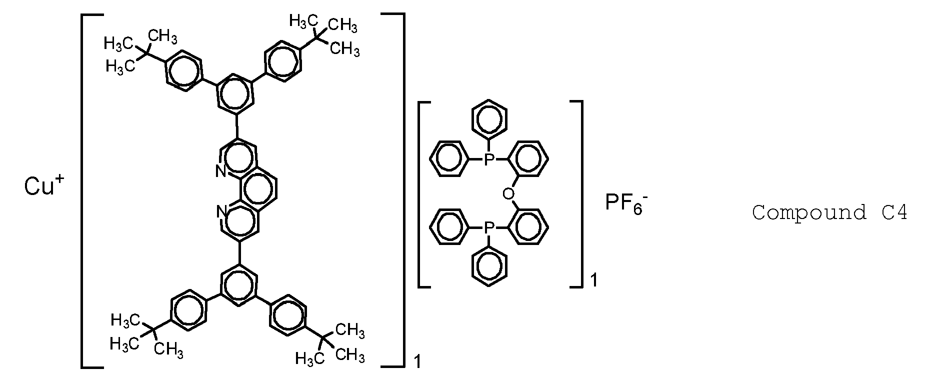

Copper(I) hexafluorophosphate (1.6 mg, 0.0043 mmol) and bis[2-(diphenylphosphino)phenyl]ether (2.3 mg, 0.0043 mmol) were stirred in dehydrated methylene chloride (2 mL) under an argon atmosphere at room temperature for 2 hours. A solution (1 mL) of the above compound B3 (3.7 mg, 0.0043 mmol) in dehydrated methylene chloride was added to the reaction solution, the homogeneous reaction solution was stirred at room temperature for 1 hour, and the reaction solution was concentrated and dried to obtain the metal complex C4.
¹H NMR (300 MHz, CDCl₃): δ 8.92 (s, 2H), 8.76 (s, 2H), 8.25 (s, 2H), 7.90 (s, 2H), 7.64 (d, J = 8.4 Hz, 8H), 7.58 (d, J = 8.4 Hz, 8H), 7.54 (s, 4H), 7.19 (t, J = 7.3 Hz, 2H), 7.04 (t, J = 7.5 Hz, 8H), 6.10-6.92 (m, 14H), 6.81-6.70 (br, 4H), 1.43 (s, 36H).
³¹P NMR (122 MHz, CDCl₃): δ-9.06.

The metal complex C4 emitted yellow light under ultraviolet excitation (365 nm), the emission peak wavelength was 581 nm, and the excitation lifetime was 2.4 µs.

The metal complex C4 was easily dissolved in chloroform, toluene, and xylene at room temperature.

When the metal complex C4 was left to stand in air at 100°C for 7 hours, change in emission intensity was hardly observed.

### EXAMPLE 5

### <Synthesis of Metal complex C5>

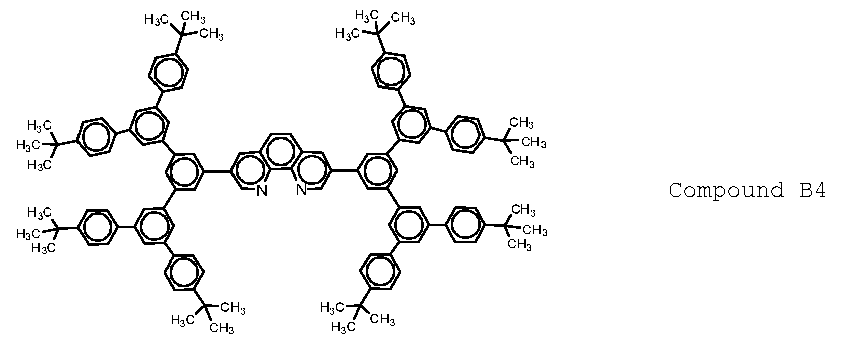

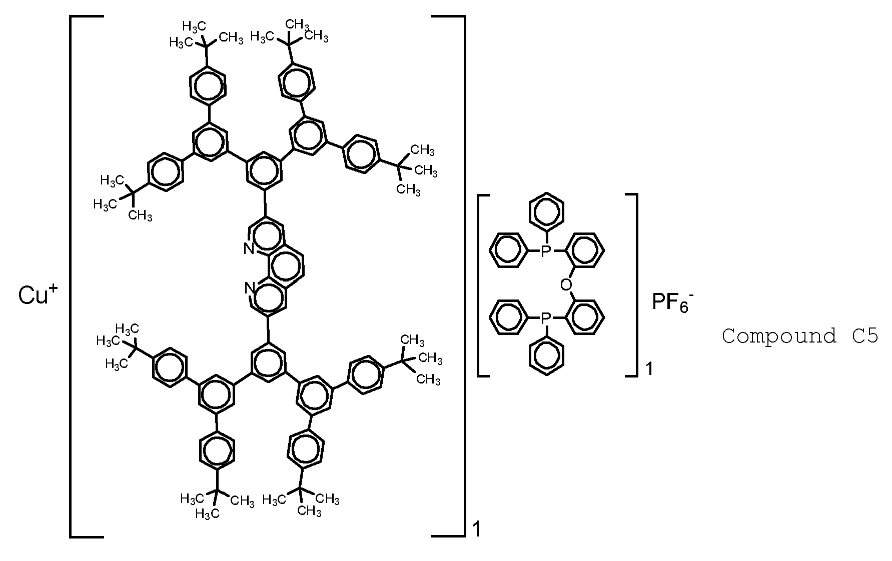

The compound B4 was synthesized in accordance with the method described in Tetrahedron Letters 41, 6809-6813 (2000).

Copper(I) hexafluorophosphate (2.4 mg, 0.0064 mmol) and bis[2-(diphenylphosphino)phenyl]ether (3.4 mg, 0.0064 mmol) were stirred in dehydrated methylene chloride (2 mL) under an argon atmosphere at room temperature for 2 hours. A solution (1 mL) of the compound B4 (10.8 mg, 0.0064 mmol) in dehydrated methylene chloride was added to the reaction solution, the homogeneous reaction solution was stirred at room temperature for 1 hour, and the reaction solution was concentrated and dried to obtain the metal complex C5.
¹H NMR (300MHz, CDCl₃): δ 9.00 (s, 2H), 8.79 (s, 2H), 8.28 (s, 2H), 8.11 (s, 2H), 7.91 (s, 4H), 7.86 (s, 8H), 7.70 (d, J = 7.2 Hz, 16H), 7.52 (d, J = 7.2Hz, 16H), 6.95 (br, 20H), 6.89-6.80 (m, 4H), 6.74 (br, 2H), 6.69 (br, 2H), 1.38 (s, 36H).
³¹P NMR (122 MHz, CDCl₃): δ-8.82.

The metal complex C5 emitted yellow light under ultraviolet excitation (365 nm), the emission peak wavelength was 572.5 nm, and the excitation lifetime was 1.4 µs.

The metal complex C5 was easily dissolved in chloroform, toluene, and xylene at room temperature.

When the metal complex C5 was left to stand in air at 100°C for 7 hours, change in emission intensity was hardly observed.

### EXAMPLE 6

### <Synthesis of Compound B5>

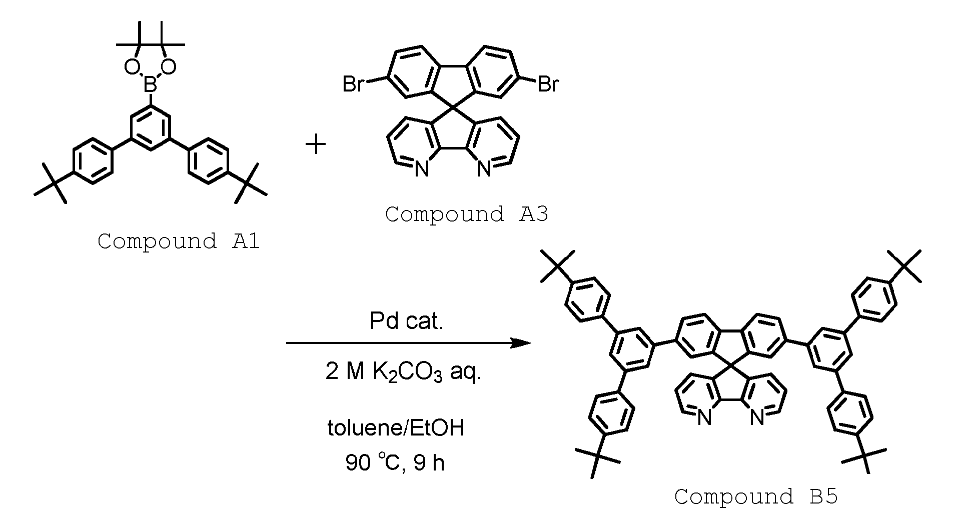

Toluene (4 mL), ethanol (1 mL), and a 2M potassium carbonate aqueous solution (1 mL) were added to the compound A1 (120 mg, 0.256 mmol), the compound A3 (55.4 mg, 0.116 mmol), and (1,3-diisopropylimidazol-2-ylidene)(3-chloropyridyl)palladium(II) dichloride (7.91 mg, 0.0116 mmol) under an argon atmosphere, and the solution was stirred at 90°C for 9 hours. Water and chloroform were added to the reaction solution, and an organic layer was collected, and dried over sodium sulfate. The solvent was concentrated and dried to obtain a crude product. The crude product was purified by a silica gel column chromatography to obtain a compound B5 (11.2 mg, 96.7%) as a pale yellow solid.
¹H NMR (300MHz, CDCl₃): δ 8.78-8.76 (m, 2H), 8.00 (d, J = 8.0 Hz, 2H), 7.79 (d, J = 8.0 Hz, 2H), 7.69 (s, 2H), 7.57-7.53 (m, 12H), 7.46 (d, J = 7.2 Hz, 8H), 7.25 (br, 2H), 7.18-7.16 (m, 2H), 7.07 (s, 2H), 1.36 (s, 36H).
Elemental Analysis Found(%) C: 87.16, H: 7.00, N: 2.54,
Calcd for B5+2H₂O(%) C: 87.00, H: 7.20, N: 2.71.

### <Synthesis of Metal complex C6>

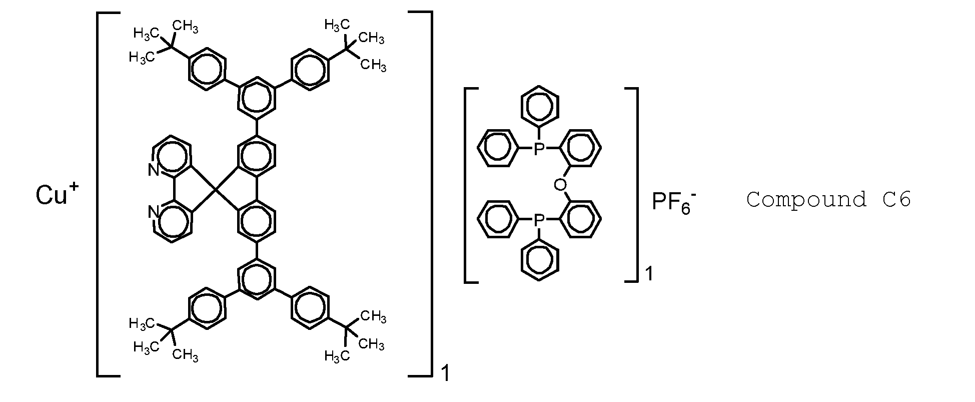

Copper(I) hexafluorophosphate (2.4 mg, 0.0064 mmol) and bis[2-(diphenylphosphino)phenyl]ether (3.4 mg, 0.0064 mmol) were stirred in dehydrated methylene chloride (2 mL) under an argon atmosphere at room temperature for 2 hours. The solution (1 mL) of the compound B5 (6.6 mg, 0.0064 mmol) in dehydrated methylene chloride was added to the reaction solution, and the homogeneous reaction solution was stirred at room temperature for 1 hour, and was concentrated and dried to obtain the metal complex C6.
¹H NMR (300 MHz, CDCl₃): δ 8.26 (br, 2H), 8.06 (d, J = 8.0 Hz, 2H), 7.80 (d, J = 8.0 Hz, 2H), 7.72 (s, 2H), 7.60-7.50 (m, 12H), 7.44 (m, 8H), 7.35-7.22 (m, 4H), 7.15-6.80 (m, 26H), 6.75 (br, 2H), 6.63 (br, 2H), 1.34 (s, 36H).
³¹P NMR (122 MHz, CDCl₃): δ-12.0.

The metal complex C6 emitted yellow light under ultraviolet excitation (365 nm), the emission peak wavelength was 566.0 nm, and the excitation lifetime was 1.4 µs.

The metal complex C6 was easily dissolved in chloroform, toluene, and xylene at room temperature.

When the metal complex C6 was left to stand in air at 100°C for 7 hours, change in emission intensity was hardly observed.

### COMPARATIVE EXAMPLE 1

### <Synthesis of Metal complex D1>

Pyridine (5 mL) and dry acetone (10 mL) were added to copper(I) iodide (400 mg, 2.1 mmol) under an argon atmosphere, and the mixture was stirred at room temperature to prepare a solution. When this solution was cooled to - 18°C, a crystal was formed. The crystal was collected to obtain the metal complex D1 (312 mg) represented by the following compositional formula:

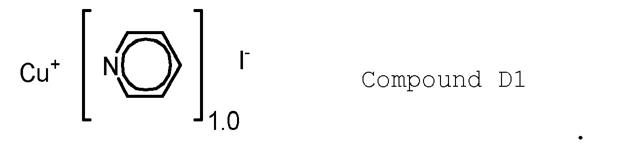

The metal complex D1 emitted yellow light under ultraviolet excitation (365 nm), the emission peak wavelength was 560 nm, and the excitation lifetime was 6.3 µs.

The metal complex D1 was poorly dissolved in chloroform, toluene, and xylene, and was not dissolved in hexane at room temperature.

When the metal complex D1 was left to stand in air at 100°C for 7 hours, emission was not observed. Further, when the metal complex D1 was heated with the thermogravimetric and differential thermal analyzer (manufactured by Seiko Instruments Inc., trade name: EXSTAR-6300), decrease in mass was observed at 123°C.

### INDUSTRIAL APPLICABILITY

The metal complex of the present invention is excellent in durability. In a preferred embodiment, the metal complex has high solubility to an organic solvent, and is useful also for manufacture of a device by an application method. Further, the metal complex of the present invention is useful for synthesis of compounds, and materials of an additive, a modifier, a cell, a catalyst, a sensor and the like, and materials of an organic EL device, an organic transistor device, an organic photoelectric conversion device and the like.

## Claims

1. A metal complex comprising a nitrogen-containing aromatic ring ligand which has a dendritic molecular chain, and a copper(I) ion or a silver(I) ion.

2. The metal complex according to claim 1, wherein the dendritic molecular chain has a conjugated system.

3. The metal complex according to claim 2, wherein a branched portion within the dendritic molecular chain is a trivalent group having an aromatic ring and a terminal portion having an aromatic ring is bonded to the branched portion.

4. The metal complex according to claim 3, wherein the branched portion is a trivalent group represented by formula (1): 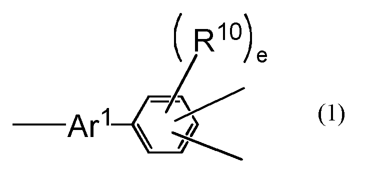 wherein Ar¹ represents a direct bond, an oxygen atom, an imino group, a -Q¹-CONH- group, a diimido group, a hydrocarbondiyl group, a -Q¹-O- group, a -Q¹-S- group, or a divalent heterocyclic group, and Q¹ indicates a hydrocarbondiyl group, wherein the imino group and the hydrocarbondiyl group optionally have a substituent; R¹⁰ represents a halogen atom, a cyano group, a nitro group, a carboxyl group, an amino group, an acylamino group, an imido group, a silyl group, a hydroxyl group, an acyl group, a hydrocarbyl group, a hydrocarbyloxy group, a hydrocarbylthio group, or a heterocyclic group, wherein the amino group, the hydrocarbyl group, the hydrocarbyloxy group and the hydrocarbylthio group optionally have a substituent; e is an integer of from 0 to 3; and when e is 2 or more, R¹⁰ which is plurally present may be the same as or different from each other, and
the terminal portion is bonded to the branched portion to form the nitrogen-containing aromatic ring ligand, wherein the terminal portion is represented by formula (2): 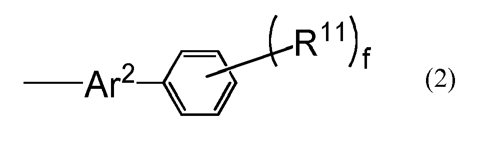 wherein Ar² represents a direct bond, an oxygen atom, an imino group, a -Q²-CONH- group, a diimido group, a hydrocarbondiyl group, a -Q²-O- group, a -Q²-S- group, or a divalent heterocyclic group, and Q² indicates a hydrocarbondiyl group, wherein the imino group and the hydrocarbondiyl group optionally have a substituent; R¹¹ represents a halogen atom, a cyano group, a nitro group, a carboxyl group, a -COO⁻ group, an amino group, a phosphono group, a sulfo group, an acylamino group, an imido group, a silyl group, a hydroxyl group, an acyl group, an ammonio group , a hydrocarbyl group, a hydrocarbyloxy group, a hydrocarbylthio group, or a heterocyclic group, wherein the amino group, the phosphono group, the sulfo group, the silyl group, the ammonio group, the hydrocarbyl group, the hydrocarbyloxy group and the hydrocarbylthio group optionally have a substituent; f is an integer of from 0 to 5; and when f is 2 or more, R¹¹ which is plurally present may be the same as or different from each other.

5. The metal complex according to claim 4, wherein Ar¹ and Ar² are direct bonds.

6. The metal complex according to claim 1, wherein the nitrogen-containing aromatic ring ligand which has the dendritic molecular chain is a ligand represented by formula (3): 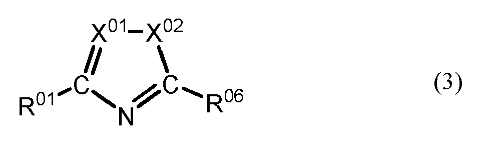 wherein X⁰¹ is a nitrogen atom or a -C(R⁰²)= group, and X⁰² is a -X⁰³=X⁰⁴- group, a -N(R⁰³)- group, N⁻, an oxygen atom, or a sulfur atom; X⁰³ is a nitrogen atom or a -C(R0⁴)= group, and X⁰⁴ is a nitrogen atom or a -C(R⁰⁵)= group; provided that, when X⁰² is the -X⁰³-X⁰⁴- group, at least one of X⁰¹, x⁰³, and X⁰⁴ is not the nitrogen atom; when X⁰¹ is the nitrogen atom, X⁰² is the -X⁰³-X⁰⁴- group or the -N(R⁰³)-group; and when X⁰² is N⁻, the oxygen atom, or the sulfur atom, X⁰¹ is the -C(R°2)= group; R⁰¹, R⁰²,R⁰⁴,R⁰⁵, and R⁰⁶ each independently represent a hydrogen atom, a halogen atom, a cyano group, a nitro group, a carboxyl group, a - COO⁻ group, an acylamino group, an imido group, a silyl group, a hydroxyl group, an acyl group, a hydrocarbyl group, a hydrocarbyloxy group, a hydrocarbylthio group, or a heterocyclic group, wherein the silyl group, the hydrocarbyl group, the hydrocarbyloxy group and the hydrocarbylthio group optionally have a substituent; R⁰³ represents a hydrogen atom, a hydrocarbyl group, or a heterocyclic group, wherein the hydrocarbyl group optionally has a substituent; at least one of R⁰¹, R⁰², R⁰³, R⁰⁴, R⁰⁵, and R⁰⁶ has a dendritic molecular chain; and R⁰¹ and R⁰², R⁰² and R⁰³, R⁰² and R⁰⁴_{,} R⁰³ and R⁰⁶, R⁰⁴ and R⁰⁵, or R⁰⁵ and R⁰⁶ may be each taken together to form a ring.

7. The metal complex according to claim 1, wherein the nitrogen-containing aromatic ring ligand which has the dendritic molecular chain is a ligand represented by formula (100): 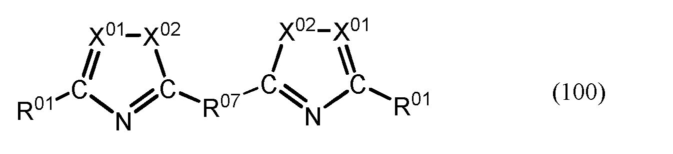 wherein X⁰¹ is a nitrogen atom or a -C(R⁰²)= group, X⁰² is a -X⁰³-X⁰⁴- group, a -N(R⁰³)- group, N⁻, an oxygen atom, or a sulfur atom; X⁰³ is a nitrogen atom or a -C(R⁰⁴)= group, and X⁰⁴ is a nitrogen atom or a -C(R⁰⁵)= group; provided that, when X⁰² is the -X⁰³=X⁰⁴- group, at least one of X⁰¹, x⁰³, and X⁰⁴ is not the nitrogen atom; when X⁰¹ is the nitrogen atom, X⁰² is the -X⁰³=X⁰⁴- group or the -N(R⁰³) group, and when X⁰² is N⁻, the oxygen atom, or the sulfur atom, X⁰¹ is the -C(R⁰²)= group; R⁰¹, R⁰², R⁰⁴, R⁰⁵, and R⁰⁶ each independently represent a hydrogen atom, a halogen atom, a cyano group, a nitro group, a carboxyl group, a - COO⁻ group, an acylamino group, an imido group, a silyl group, a hydroxyl group, an acyl group, a hydrocarbyl group, a hydrocarbyloxy group, a hydrocarbylthio group, or a heterocyclic group, wherein the silyl group, the hydrocarbyl group, the hydrocarbyloxy group and the hydrocarbylthio group optionally have a substituent; R⁰³ represents a hydrogen atom, a hydrocarbyl group, or a heterocyclic group, wherein the hydrocarbyl group optionally has a substituent; at least one of R⁰¹, R⁰², R⁰³, R⁰⁴, R⁰⁵, and R⁰⁶ has a dendritic molecular chain; R⁰¹ and R⁰², R⁰² and R⁰³, R⁰² and R⁰⁴, R⁰³ and R⁰⁶, R⁰⁴ and R⁰⁵, or R⁰⁵ and R⁰⁶ may be each taken together to form a ring; R⁰⁷ represents a direct bond, an oxygen atom, an imino group, a -Q³-CONH- group, a diimido group, a hydrocarbondiyl group, a -Q³-O- group, a -Q³-S- group, or a divalent heterocyclic group; Q³ indicates a hydrocarbondiyl group which optionally has a substituent; R⁰¹, X⁰¹, and X⁰² which are plurally present may be the same as or different from each other; and when each X⁰² is the -N(R⁰³)- group, both R⁰³ may be taken together to form a ring, when each X⁰² is the - X⁰³-X⁰⁴- group, both X⁰⁴ may be taken together to form a ring, and when one X⁰² is the N(R⁰³)- group and the other X⁰² is the -X⁰³=X⁰⁴- group, R⁰³ and X⁰⁴ may be taken together to form a ring.

8. The metal complex according to claim 1, wherein the nitrogen-containing aromatic ring ligand which has the dendritic molecular chain is represented by formula (4): 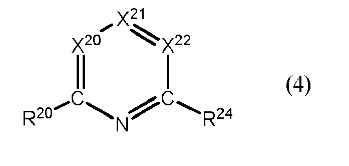 wherein X²⁰ is a nitrogen atom or a -C(R²¹)= group, X²¹ is a nitrogen atom or a -C(R²²)= group, and X²² is a nitrogen atom or a -C(R²³)= group; provided that at least one of X²⁰, X²¹, and X²² is not a nitrogen atom; at least one of R²⁰, R²¹, R²², R²³, and R²⁴ represents a dendritic molecular chain, and R²⁰, R²¹, R²², R²³, and R²⁴ each independently represent a hydrogen atom, a halogen atom, a cyano group, a nitro group, a carboxyl group, a -COO⁻ group, an acylamino group, an imido group, a silyl group, a hydroxyl group, an acyl group, a hydrocarbyl group, a hydrocarbyloxy group, a hydrocarbylthio group, or a heterocyclic group, wherein the silyl group, the hydrocarbyl group, the hydrocarbyloxy group and the hydrocarbylthio group optionally have a substituent; and R²⁰ and R²¹, R²¹ and R²², R²² and R²³, and R²³ and R²⁴ may be each taken together to form a ring, or formula (5): 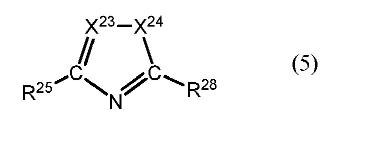 wherein X²³ is a nitrogen atom or a -C(R²⁶)= group, and X²⁴ is a -N(R²⁷)= group, N⁻, an oxygen atom, or a sulfur atom; provided that, when X²³ is the nitrogen atom, X²⁴ is the -N(R²⁷)= group, when X²⁴ is N⁻, the oxygen atom, or the sulfur atom, X²³ is the -C(R²⁶)= group; at least one of R²⁵, R²⁶, R²⁷, and R²⁸ represents a dendritic molecular chain, and R²⁵, R²⁶, and R²⁸ each independently represent a hydrogen atom, a halogen atom, a cyano group, a nitro group, a carboxyl group, a -COO⁻ group, an acylamino group, an imido group, a silyl group, a hydroxyl group, an acyl group, a hydrocarbyl group, a hydrocarbyloxy group, a hydrocarbylthio group, or a heterocyclic group, wherein the silyl group, the hydrocarbyl group, the hydrocarbyloxy group and the hydrocarbylthio group optionally have a substituent; R²⁷ represents a hydrogen atom, a hydrocarbyl group, or a heterocyclic group, wherein the hydrocarbyl group optionally has a substituent; and R²⁵ and R²⁶, R²⁶ and R²⁷, and R²⁷ and R²⁸ may be each taken together to form a ring.

9. The metal complex according to claim 1, which is represented by the following compositional formula (6): 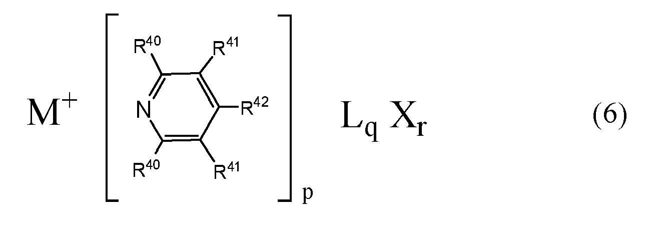 wherein M⁺ is a copper(I) ion or a silver(I) ion; L is a ligand; and X is a counter ion; p is a positive number; and q and r are each independently numerical numbers of 0 or more; R⁴⁰, R⁴¹, and R⁴² each independently represents a hydrogen atom, a halogen atom, a cyano group, a nitro group, a carboxyl group, an amino group, an acylamino group, a silyl group, a hydroxyl group, an acyl group, a hydrocarbyl group, a hydrocarbyloxy group, a hydrocarbylthio group, or a heterocyclic group, wherein the amino group, the silyl group, the hydrocarbyl group, the hydrocarbyloxy group and the hydrocarbylthio group optionally have a substituent; at least one hydrogen atom in R⁴⁰, R⁴¹, and R⁴¹ is substituted with a group indicated by formula (7): 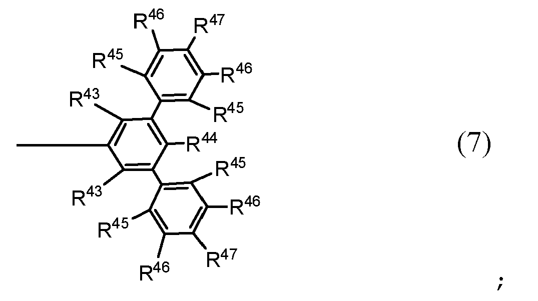 the respective R⁴⁰ or the respective R⁴¹ may be the same as or different from each other, and R⁴⁰ and R⁴¹, and R⁴¹ and R⁴² may be each taken together to form a ring; in formula (7), R⁴³, R⁴⁴, R⁴⁵, R⁴⁶, and R⁴⁷ represents a hydrogen atom, a halogen atom, a cyano group, a nitro group, a carboxyl group, an amino group, an acylamino group, a silyl group, a hydroxyl group, an acyl group, a hydrocarbyl group, a hydrocarbyloxy group, or a hydrocarbylthio group, wherein the amino group, the silyl group, the hydrocarbyl group, the hydrocarbyloxy group and the hydrocarbylthio group optionally have a substituent; R⁴³, R⁴⁵, R⁴⁶, or R⁴⁷ which is plurally present may be the same as or different from each other; R⁴³ and R⁴⁵, R⁴⁴ and R⁴⁵, R⁴⁵ and R⁴⁶, or R⁴⁶ and R⁴⁷ may be each taken together to form a ring; and when L, X, and a structure represented by the following formula: 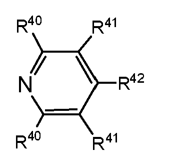 are plurally present, they may be the same as or different from each other.

10. The metal complex according to claim 1, which is represented by compositional formula (8): 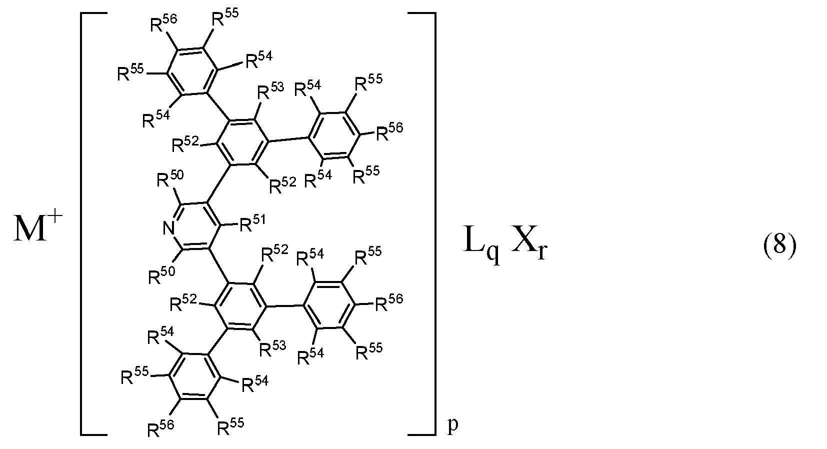 wherein M⁺ is a copper(I) ion or a silver(I) ion; L is a ligand; and X is a counter ion; p is a positive number; and q and r are each independently numerical numbers of 0 or more; R⁵⁰, R⁵¹, R⁵², R⁵³, R⁵⁴, R⁵⁵, and R⁵⁶ represent a hydrogen atom, a halogen atom, a cyano group, a nitro group, a carboxyl group, an amino group, an acylamino group, a silyl group, a hydroxyl group, an acyl group, a hydrocarbyl group, a hydrocarbyloxy group, or a hydrocarbylthio group, wherein the amino group, the silyl group, the hydrocarbyl group, the hydrocarbyloxy group and the hydrocarbylthio group optionally have a substituent; R⁵⁰, R⁵², R⁵³, R⁵⁴, R⁵⁵, or R⁵⁶ which is plurally present may be the same as or different from each other; R⁵² and R⁵⁴, R⁵³ and R⁵⁴, R⁵⁴ and R⁵⁵, and R⁵⁵ and R⁵⁶ may be each taken together to form a ring; and when L, X, and a structure represented by formula: 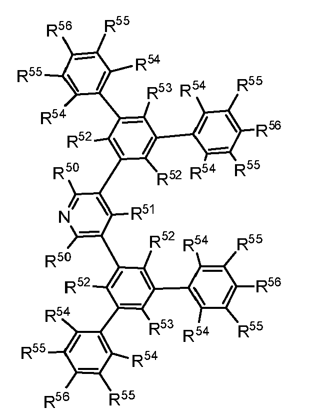 are plurally present, they may be the same as or different from each other.

11. The metal complex according to claim 1, which is represented by the following compositional formula (9): 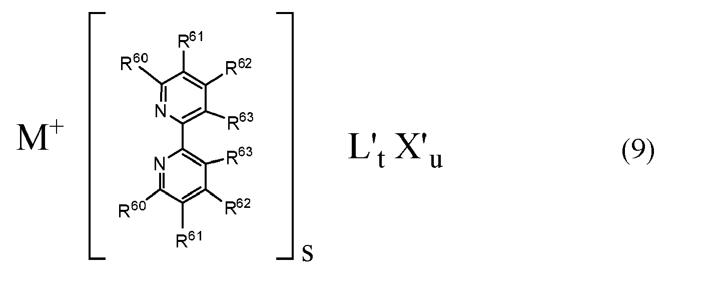 wherein M⁺ is a copper(I) ion or a silver(I) ion; L' is a ligand; and X' is a counter ion; s is a positive number; and t and u are each independently numerical numbers of 0 or more; R⁶⁰, R⁶¹, R⁶², and R⁶³ each independently represent a hydrogen atom, a halogen atom, a cyano group, a nitro group, a carboxyl group, an amino group, an acylamino group, a silyl group, a hydroxyl group, an acyl group, a hydrocarbyl group, a hydrocarbyloxy group, a hydrocarbylthio group, or a heterocyclic group, wherein the amino group, the silyl group, the hydrocarbyl group, the hydrocarbyloxy group and the hydrocarbylthio group optionally have a substituent; at least one hydrogen atom in R⁶⁰, R⁶¹, R⁶², and R⁶³ is substituted with a group represented by the formula (7); the respective R⁶⁰, the respective R⁶¹, the respective R⁶², or the respective R⁶³ may be the same as or different from each other, and R⁶⁰ and R⁶¹, R⁶¹ and R⁶², R⁶² and R⁶³, and both R⁶³ may be each taken together to form a ring; and when L', X', and a structure represented by formula: 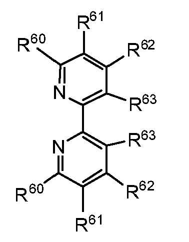 are plurally present, they may be the same as or different from each other,
or the following formula (10): 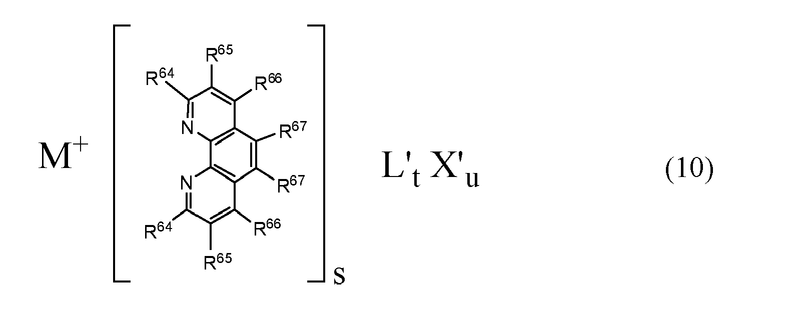 wherein M⁺ is a copper(I) ion or a silver(I) ion; L' is a ligand; and X' is a counter ion; s is a positive number; and t and u are each independently numerical numbers of 0 or more; R⁶⁴, R⁶⁵, R⁶⁶, and R⁶⁷ each independently represent a hydrogen atom, a halogen atom, a cyano group, a nitro group, a carboxyl group, an amino group, an acylamino group, a silyl group, a hydroxyl group, an acyl group, a hydrocarbyl group, a hydrocarbyloxy group, a hydrocarbylthio group, or a heterocyclic group, wherein the respective groups optionally have a substituent; at least one hydrogen atom in R⁶⁴, R⁶⁵, R⁶⁶, and R⁶⁷ is substituted with the formula (7); the respective R⁶⁴, the respective R⁶⁵, the respective R⁶⁶, or the respective R⁶⁷ may be the same as or different from each other, and each of R⁶⁴ and R⁶⁵, R⁶⁵ and R⁶⁶, R⁶⁶ and R⁶⁷, and both R⁶⁷ may be each taken together to form a ring; when L', X', and a structure represented by formula: 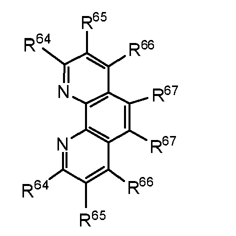 are plurally present, they may be the same as or different from each other,
or formula (11): 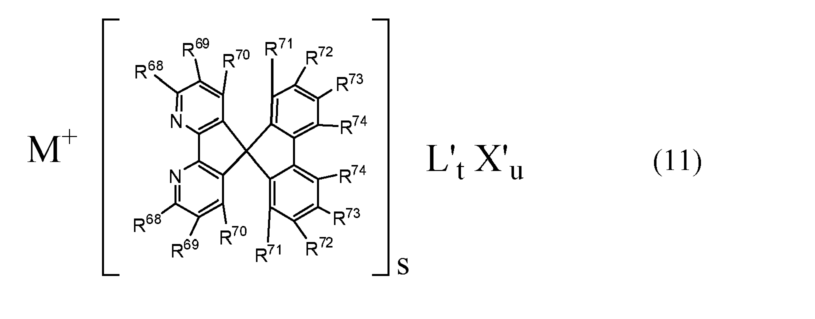 wherein M⁺ is a copper(I) ion or a silver(I) ion; L' is a ligand; and X' is a counter ion; s is a positive number; and t and u are each independently numerical numbers of 0 or more; R⁶⁸, R⁶⁹, R⁷⁰, R⁷¹, R⁷², R⁷³, and R⁷⁴ each independently represent a hydrogen atom, a halogen atom, a cyano group, a nitro group, a carboxyl group, an amino group, an acylamino group, a silyl group, a hydroxyl group, an acyl group, a hydrocarbyl group, a hydrocarbyloxy group, a hydrocarbylthio group, or a heterocyclic group, wherein the amino group, the silyl group, the hydrocarbyl group, the hydrocarbyloxy group and the hydrocarbylthio group optionally have a substituent; at least one hydrogen atom in R⁷¹, R⁷², R⁷³, and R⁷⁴ is substituted with the formula (7); the respective R⁶⁸, the respective R⁶⁹, the respective R⁷⁰, the respective R⁷¹, the respective R⁷², the respective R⁷³, or the respective R⁷⁴ may be the same as or different from each other, and R⁶⁸ and R⁶⁹, R⁶⁹ and R⁷⁰, R⁷¹ and R⁷², R⁷² and R⁷³, R⁷³ and R⁷⁴, and both R⁷⁴ may be each taken together to form a ring; and when L', X', and a structure represented by the following formula: 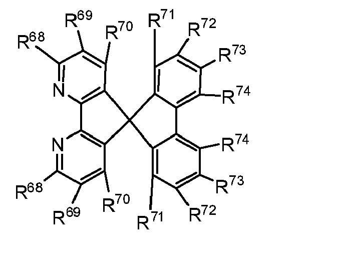 are plurally present, they may be the same as or different from each other.

12. The metal complex according to claim 1, which is represented by compositional formula (12): 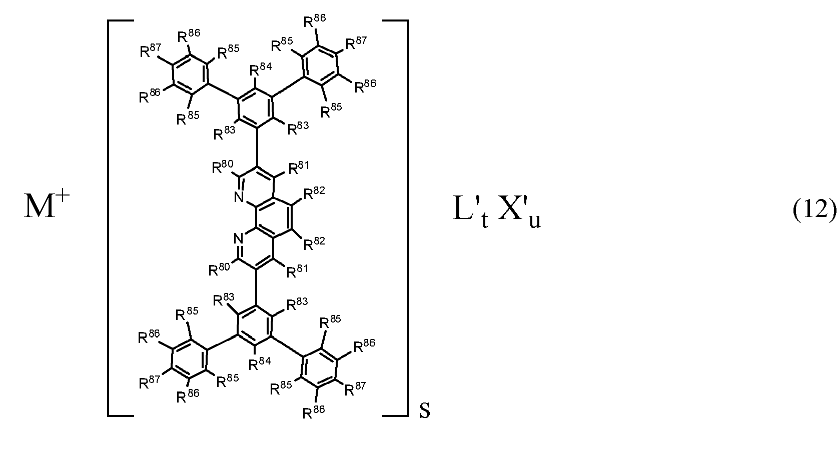 wherein M⁺ is a copper(I) ion or a silver(I) ion; L' is a ligand; and X' is a counter ion; s is a positive number; and t and u are each independently numerical numbers of 0 or more; R⁸⁰, R⁸¹, R⁸², R⁸³, R⁸⁴, R⁸⁵, R⁸⁶, or R⁸⁷ each independently represents a hydrogen atom, a halogen atom, a cyano group, a nitro group, a carboxyl group, an amino group, an acylamino group, an imido group, a silyl group, a hydroxyl group, an acyl group, a hydrocarbyl group, a hydrocarbyloxy group, or a hydrocarbylthio group, wherein the amino group, the silyl group, the hydrocarbyl group, the hydrocarbyloxy group and the hydrocarbylthio group optionally have a substituent; R⁸⁰, R⁸¹, R⁸², R⁸³, R⁸⁴, R⁸⁵, R⁸⁶, or R⁸⁷ which is plurally present may be the same as or different from each other; R⁸³ and R⁸⁵, R⁸⁴ and R⁸⁵, R⁸⁵ and R⁸⁶, and R⁸⁶ and R⁸⁷ may be each taken together to form a ring; and when L', X', and a structure represented by formula: 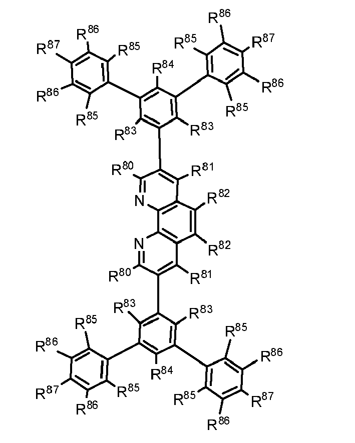 are plurally present, they may be the same as or different from each other.

13. The metal complex according to claim 1, which is represented by compositional formula (13): 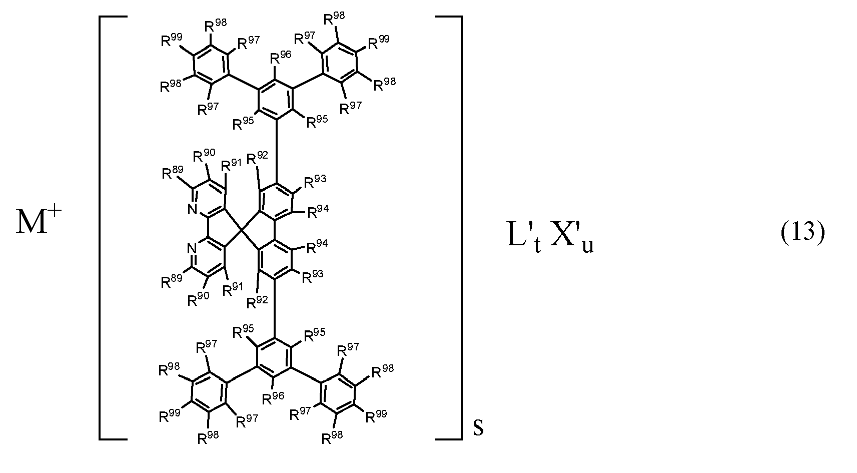 wherein M⁺ is a copper(I) ion or a silver(I) ion;, L' is a ligand; and X' is a counter ion; s is a positive number; and t and u are each independently numerical numbers of 0 or more; R⁸⁹, R⁹⁰, R⁹¹, R⁹², R⁹³, R⁹⁴, R⁹⁵, R⁹⁶, R⁹⁷, R⁹⁸, or R⁹⁹ each independently represents a hydrogen atom, a halogen atom, a cyano group, a nitro group, a carboxyl group, an amino group, an acylamino group, an imido group,
a silyl group, a hydroxyl group, an acyl group, a hydrocarbyl group, a hydrocarbyloxy group, or a hydrocarbylthio group, wherein the amino group, the silyl group, the hydrocarbyl group, the hydrocarbyloxy group and the hydrocarbylthio group optionally have a substituent; R⁸⁹, R⁹⁰, R⁹¹, R⁹², R⁹³, R⁹⁴, R⁹⁵, R⁹⁶, R⁹⁷, R⁹⁸, or R⁹⁹ which is plurally present may be the same as or different from each other; each of R⁸⁹ and R⁹⁰, R⁹⁰ and R⁹¹, R⁹² and R⁹⁵, R⁹³ and R⁹⁴, both R⁹⁴, R⁹³ and R⁹⁵, R⁹⁵ and R⁹⁷, R⁹⁶ and R⁹⁷, R⁹⁷ and R⁹⁸, and R⁹⁸ and R⁹⁹ may be each taken together to form a ring; and when L', X', and a structure represented by formula: 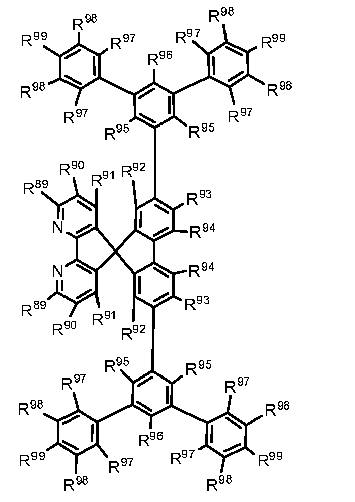 are plurally present, they may be the same as or different from each other.

14. The metal complex according to claim 3, wherein the branched portion is a group represented by formulae (a-1) to (a-16): 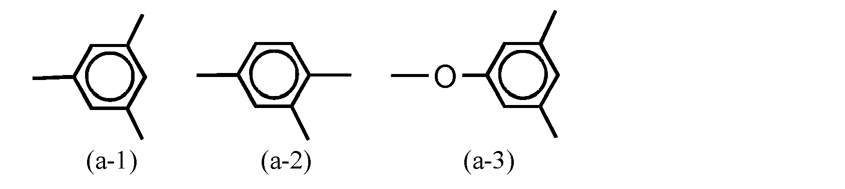 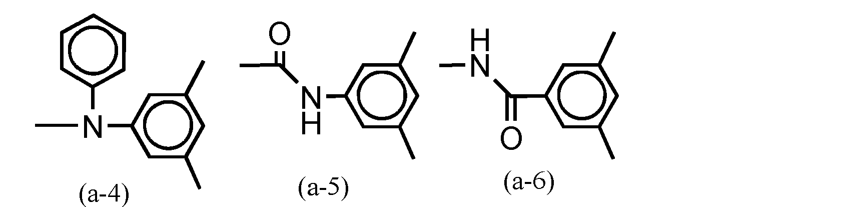 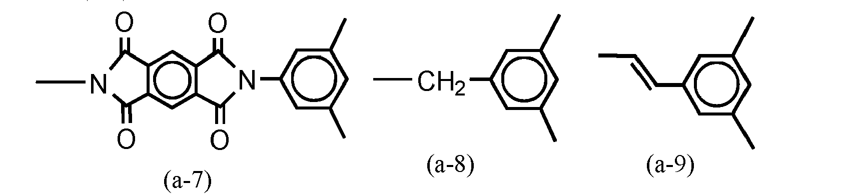 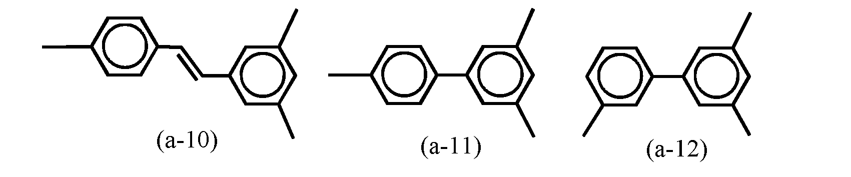 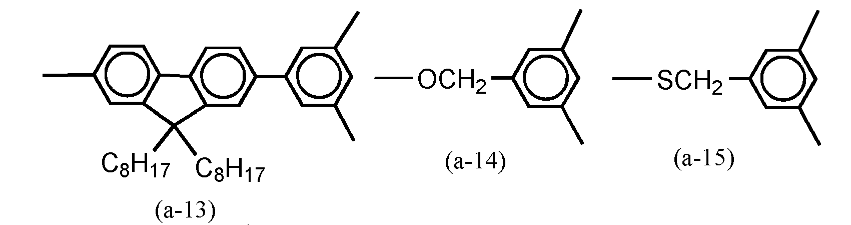 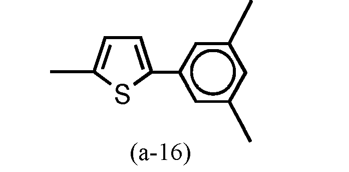 , and the terminal portion is a group represented by formulae (b-1) to (b-32):

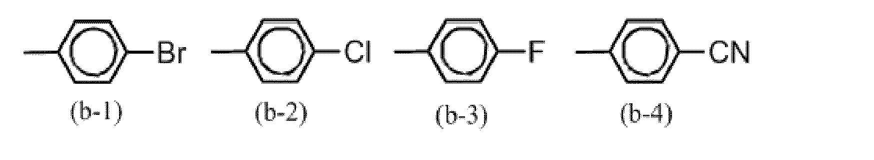

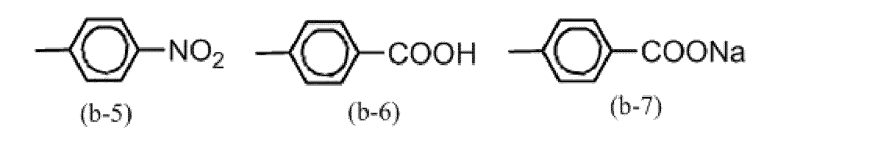

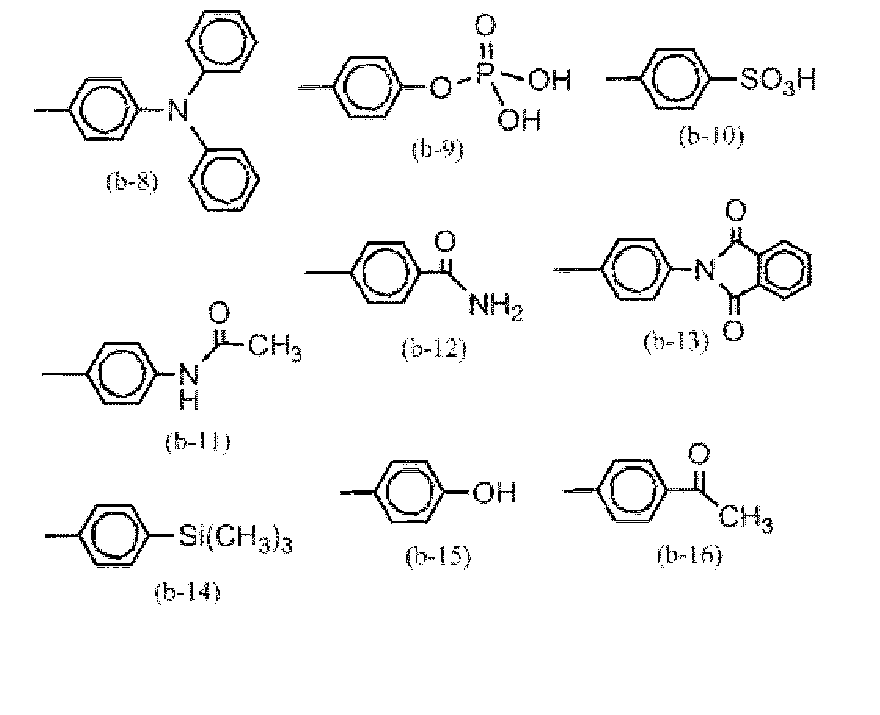

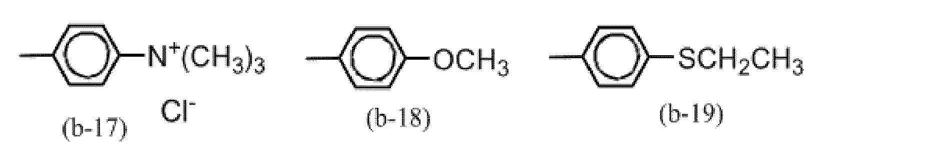

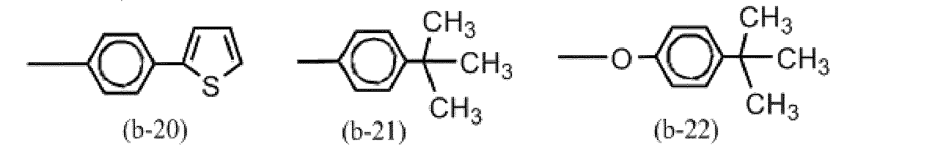

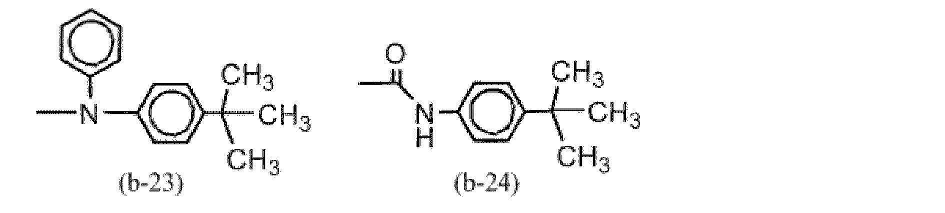

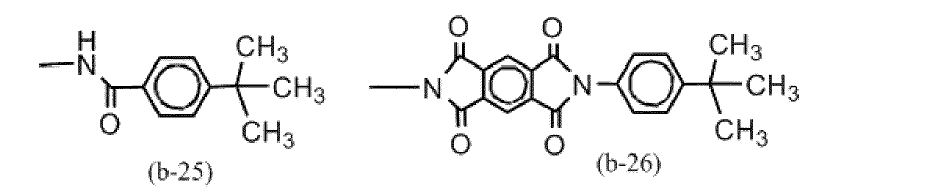

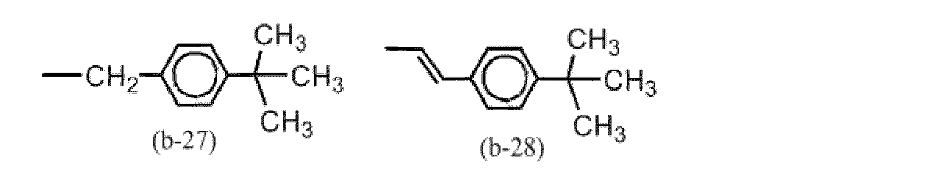

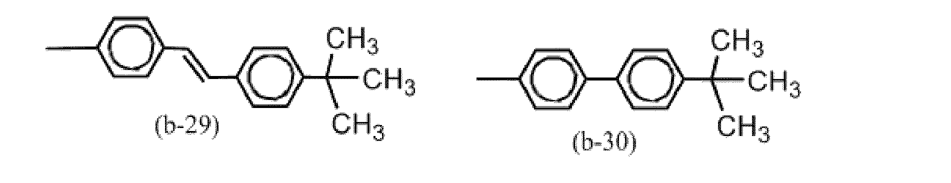

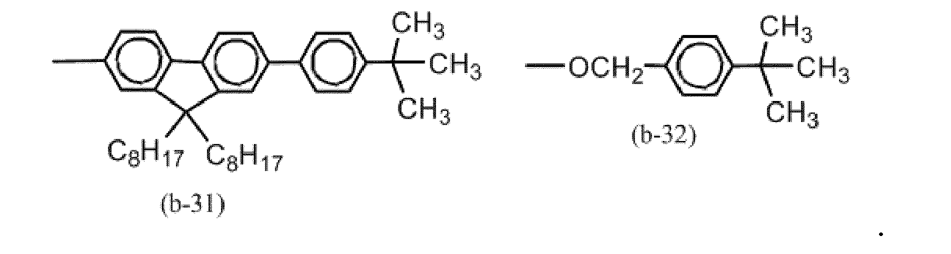

15. Use of the metal complex according to claim 1 for a light-emitting device.

16. A compound represented by the following formulae (14) or (15): 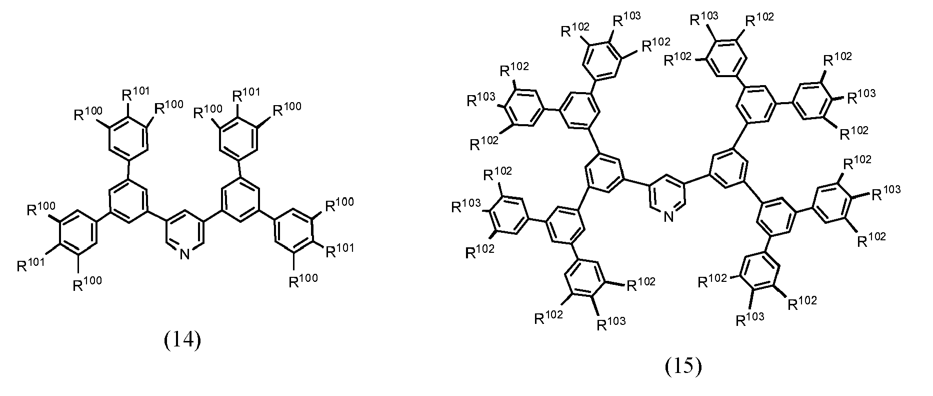 wherein R¹⁰⁰, R¹⁰¹, R¹⁰², and R¹⁰³ each independently represent a hydrogen atom, a halogen atom, a cyano group, a nitro group, a carboxyl group, an amino group, an acylamino group, an imido group, a silyl group, a hydroxyl group, an acyl group, a hydrocarbyl group, a hydrocarbyloxy group, or a hydrocarbylthio group.

17. A compound represented by formula (16): 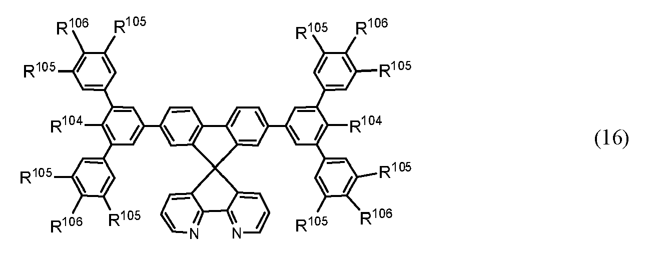 wherein R¹⁰⁴, R¹⁰⁵, and R¹⁰⁶ each independently represent a hydrogen atom, a halogen atom, a cyano group, a nitro group, a carboxyl group, an amino group, an acylamino group, an imido group, a silyl group, a hydroxyl group, an acyl group, a hydrocarbyl group, a hydrocarbyloxy group, or a hydrocarbylthio group.
